# EUROPEAN PATENT SPECIFICATION

(11) **EP 4 504 201 B1**
(45) Date of publication and mention of the grant of the patent: **03.06.2026**
(21) Application number: 23717086.5
(22) Date of filing: 31.03.2023
(51) Int. Cl.: A61K 31/513, A61K 45/06, A61P 7/02, A61P 9/10, A61K 31/381, A61K 31/422, A61K 31/427

(54) **INHIBITORS OF CHYMASE FOR USE IN THE SELECTIVE RESOLUTION OF THROMBI IN THROMBOTIC OR THROMBOEMBOLIC DISORDERS**
CHYMASEINHIBITOREN ZUR VERWENDUNG BEI DER SELEKTIVEN AUFLÖSUNG VON THROMBEN BEI THROMBOTISCHEN ODER THROMBOEMBOLISCHEN ERKRANKUNGEN
INHIBITEURS DE LA CHYMASE DESTINÉS À ÊTRE UTILISÉS DANS LA RÉSOLUTION SÉLECTIVE DES THROMBUS DANS LES TROUBLES THROMBOTIQUES OU THROMBOEMBOLIQUES

(30) Priority: 05.04.2022 US 202263327684 P; 04.05.2022 EP 22171543
(43) Date of publication of application: 12.02.2025
(73) Proprietor: Socpra Sciences Santé Et Humaines S.E.C., Sherbrooke, Québec J1L 1E2 (CA); Bayer Aktiengesellschaft, 51373 Leverkusen (DE)
(72) Inventor: D'ORLÉANS-JUSTE, Pedro, Sherbrooke, Québec J1L 1E2 (CA); LAPOINTE, Catherine, Sherbrooke, Québec J1L 1E2 (CA); HEITMEIER, Stefan, 42489 Wülfrath (DE); TINEL, Hanna, 42113 Wuppertal (DE); DAY, Robert, Sherbrooke, Québec J1L 1E2 (CA); VINCENT, Laurence, Sherbrooke, Québec J1L 1E2 (CA); SCHWERTANI, Adel, Sherbrooke, Québec J1L 1E2 (CA); ZIMMERMANN, Katja, 40489 Düsseldorf (DE); TERSTEEGEN, Adrian, 42111 Wuppertal (DE)
(74) Representative: BIP Patents
(86) International application number: PCT/EP2023/058464
(87) International publication number: WO 2023/194222

(56) References cited:
- EP-A1- 1 312 379
- EP-A1- 1 325 920
- EP-A1- 1 666 067
- WO-A1-2004/071531
- US-A1- 2008 167 348
- US-A1- 2015 148 340
- US-A1- 2016 289 220
- US-B2- 7 071 220
- DUENGEN HANS-DIRK ET AL: "Effects of the chymase inhibitor fulacimstat on adverse cardiac remodeling after acute myocardial infarction-Results of the Chymase Inhibitor in Adverse Remodeling after Myocardial Infarction (CHIARA MIA) 2 trial", AMERICAN HEART JOURNAL, ELSEVIER, AMSTERDAM, NL, vol. 224, 25 January 2020 (2020-01-25), pages 129 - 137, XP086175102, ISSN: 0002-8703, [retrieved on 20200125], DOI: 10.1016/J.AHJ.2020.01.012
- AROOJ MAHREEN ET AL: "Finding off-targets, biological pathways, and target diseases for chymase inhibitors via structure-based systems biology approach : Finding Off-Targets for Chymase Inhibitors", PROTEINS: STRUCTURE, FUNCTION, AND BIOINFORMATICS, vol. 83, no. 7, 4 May 2015 (2015-05-04), US, pages 1209 - 1224, XP055962089, ISSN: 0887-3585, DOI: 10.1002/prot.24677
- VALENT P ET AL: "NEW ASPECTS IN THROMBOSIS RESEARCH: POSSIBLE ROLE OF MAST CELLS AS PROFIBRINOLYTIC AND ANTITHROMBOTIC CELLS", THROMBOSIS AND HAEMOSTASIS, SCHATTAUER GMBH, DE, vol. 87, no. 5, 1 January 2002 (2002-01-01), pages 786 - 790, XP009075804, ISSN: 0340-6245
- LAPOINTE CATHERINE ET AL: "Chymase Inhibition Resolves and Prevents Deep Vein Thrombosis Without Increasing Bleeding Time in the Mouse Model", JOURNAL OF THE AMERICAN HEART ASSOCIATION, vol. 12, no. 4, 21 February 2023 (2023-02-21), XP093078841, DOI: 10.1161/JAHA.122.028056

## Description

The present invention covers chymase inhibitors **,** in particular substituted bicyclically substituted uracils of general formula (I) as described and defined for use in the treatment or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

Hemostasis is a protective physiological mechanism, which covers leaking damages in the blood vessel wall quickly and reliably, with the aim to avoid excessive loss of blood or to keep it to a minimum. Following such an injury of the blood vessel wall, activation, adhesion and aggregation of platelets and formation of insoluble fibrin after activation of the coagulation system result in the rapid formation of blood clots closing the leak in the vessel wall. However, the clot formation has to be in balance - to protect from (severe) bleedings, but do not lead to excessive clot formation, which may fill a large portion of the vessel lumen, thereby reducing the blood flow partially or fully and finally causing a lack of oxygen- and nutrient supply in the surrounding tissue. While physiological mechanisms to a) keep the activity of the coagulation system under control with endogenous inhibitors and to b) resolve already generated fibrin clot material (by the fibrinolytic system) do exist, they are not always sufficient. Uncontrolled, excessive activation of the coagulation system or defects in the hemostatic balance may lead to thrombosis by either formation of local thrombi or emboli, blood clots that became dislodged from another site in the circulatory system and occlude a distant vessel. The occlusion of arteries in the heart, brain and lung, known as myocardial infarction, stroke or pulmonary embolism respectively, are among the most common causes of death and morbidity worldwide. The occurrence of large numbers of microthrombi blocking smaller organ vessels has recently gained increasing attention during the COVID-19 pandemic.

The prognosis of patients with a thrombotic event is strongly dependent on a timely re-supply of the respective organ with fresh blood and thus, identifying safe ways for fast re-opening of occluded vessels is an important goal in clinical research.

While the current anticoagulant drugs, vitamin k antagonists, direct factor Xa or thrombin inhibitors, or heparins as indirect dual FXa/thrombin inhibitors, reduce the coagulability of blood and have shown to be efficacious in preventing thrombotic events, their main effect on the resolution of already existing thrombi is believed to be only indirect: reducing the activity of the coagulation system shifts the hemostatic balance towards the physiological fibrinolytic system and thereby supports the degradation of fibrin material. However, higher doses of anticoagulants may increase the risk of bleedings and due to the indirect impact, the thrombus resolution is rather slow, thus, restricting their use to indications, which do not require fast vessel re-opening.

Current approaches for rapid vessel re-opening being necessary in many indications, are based on the mechanical removal of the occluding clots or on the degradation of the fibrin clot by forced fibrinolysis.

Recently, mechanical approaches like thrombectomies, have been increasingly applied, often in large artery stroke patients. However, despite all technical improvements the thrombus accessibility and the clinical availability are frequent limitations, so that the procedures can only be applied to a minority of patients.

The main enzyme for fibrin degradation, plasmin, is activated from the plasma protein plasminogen in the presence of fibrin by plasminogen activators (tissue plasminogen activator (tPA), urokinase-type plasminogen activator (uPA)), which are secreted from endothelial cells. In blood, active plasmin is inactivated fast by a large excess of α2-antiplasmin, but it is known to remain active while being bound to fibrin. Current fibrinolytic strategies use systemically or locally administered plasminogen-activating enzymes to generate plasmin. Recombinant tissue-type plasminogen activator (rtPA, alteplase) is the most widely used fibrinolytic drug, FDA-approved in myocardial infarction, ischemic stroke, pulmonary embolism or re-establishment of patency in occluded intravenous catheters (Hughes RH et al., StatPearls2020) and in many areas standard of care for the treatment of acute ischemic stroke in patients, who can be treated within a time window of 4.5 h after the event (Powes WJ et al., Stroke50(2019)e344-e418). However, administration of tPA is associated with a dose-dependent risk of bleedings, including life-threatening intracranial bleedings (Emberson J et al., Lancet 384(2014)1929), which limit the dosing and thereby fibrinolytic efficacy of tPA. In addition to this risk/benefit profile of the drug, the short half-life in human plasma (dominant half-life < 5 min; Tanswell P et al., Arzneimittelforschung41(1991)1310-9)*,* and additional adverse effects, including re-thrombosis and the more uncommon, yet morbid tPA-associated angioedema (Rathburn KM, Oxf.Med.CaseRep.2019; Fröhlich K et al., Stroke50(2019)1682), represent an overall challenging drug profile, which is responsible for often sub-optimal efficacy and restricted use.

Therefore, identifying fast, reliable, and safe strategies for medical revascularization remain a challenging task associated with a very high medical need.

So far, the role of mast cells and their content in the pathology of thrombotic events may have been vastly under-evaluated and under-estimated and therapeutic approaches targeting mast cell granular components for thrombus resolution do not exist.

Mast cells are long-lived perivascular resident cells of hematopoietic origin distributed in most tissues, often located at the boundaries between tissues and the external environment. For example, they can be found at mucosal or epithelial surfaces of the gut and lungs, in the skin, and tend to cluster around blood and lymphatic vessels and nerves. (Prussin C, Metcalfe DD, J.AllergyClin.Immunol. 111(2003)S486-94). Only small numbers of progenitor cells can be found in the blood before re-localizing in tissues where their maturation is completed. They are multifunctional immune cells implicated in several health and disease states and are best known for their roles in allergy and anaphylaxis in lungs, gut or skin. However, they are involved in additional processes, including protective strategies, like host defense against pathogens, immune tolerance, wound healing, angiogenesis, as well as in several pathophysiological processes (Miyazaki et al., Pharmacol. Ther.112 (2006), 668-676; Shiota et al., J. Hypertens.21(2003)1823-1825)*.* An increase in their number has been observed in patients with heart failure, myocardial infarction and ischemia, in human atherosclerotic plaques and in abdominal aortic aneurysms (Kovanen PT et al., Circ.92(1995)1084-1088; Libby P, Shi GP, Circ.115(2007)2555-2558; Bankl HC et al., Circ.91(1995)275-83). Recently, the role of mast cell activation in the cause of SARS-CoV-2 infection has been discussed (Wu ML et al., SignalTransduct. Targ. Ther.6(2021)428; Kempuraj D. et al., Neuroscientist 26(2020)402-414).

Mast cells contain many large cytoplasmic granules, which release upon stimulation in addition to large amounts of histamine and heparin many other active ingredients, including several cytokines, proteoglycans and serine proteases, such as tryptase, chymase, chymotrypsin, cathepsin G and carboxypeptidase A (Lindstedt KA et al., J.Cell.Mol.Med.11(2007)739-758). Some of the stored compounds, like heparin, polyphosphates or histamine, are known to interact with the coagulation system (Guilarte M et al. Front. Immunol2017), but it is still a matter of debate, whether the overall effect of mast cells might be anti- or procoagulant and whether it has a relevant impact on clot formation. An anti-fibrinolytic impact on thrombi in the vascular system, has so far not been described for mast cells or the granule ingredients.

Recently, in an experimental venous stenosis model in mice, in contrast to untreated mice, two strains of mast cell-deficient mice were completely protected against venous thrombosis (Ponomaryov et al., CircRes.121(2017)941-950*).* It was concluded that the potential procoagulant effect of mast cells might be attributed to the release of histamine, which could not be verified with histamine receptor blockers. A potential effect on the fibrinolytic system and thereby an impact on the resolution of thrombi was not described. In a mouse model of arterial thrombosis (Ponomaryov et al. (CircRes.121(2017)941-950), in which thrombus generation was triggered by ferric chloride-induced injury of a mesenteric arteriole, no statistically significant difference in the occlusion time of wild type- and mast cell-deficient mice was observed. The authors concluded that mast cells might only be relevant in venous thrombosis and potentially not in arterial thrombosis.

In a review on mast cells' interactions with the fibrinolytic system, Bankl and Valent (Thromb.Res.105(2002)359*)* stated that mast cells themselves might be profibrinolytic due to the selective production of tPA and not of its endogenous inhibitor PAI-1. Their conclusion that mast cells are profibrinolytic is in contrast to the results related to the surprising findings of this invention, showing that chymase has antifibrinolytic properties and an inhibitor could be used for vessel re-opening.

Chymase is a serine protease, which is stored as a complex with heparin in mast cell granules. Upon activation of the mast cells, it is released into the extracellular space, where the chymase is activated and - like other mast cell proteases - is under strict control to prevent damage to the host tissue (Pejler G. et al., Adv.Immunol.95(2007)167-255*).* However, these control mechanisms have not been totally elucidated yet: in human whole blood chymase is subject to fast inhibition by endogenous circulating inhibitors, including α-1 antitrypsin, α-1 antichymotrypsin, α-2 macroglobulin (Metcalfe D.D. et al., World Allergy Organ. J. 11(2016)7*),* so that chymase activity can usually not be measured in blood, serum or plasma. Therefore, it was not expected that chymase activity could be measured in thrombi in the vessel.

Several substrates of chymase have been described: It is involved in the production of angiotensin II in the heart, in the artery wall and in the lung (Fleming I., Circ. Res. 98 (2006), 887-896) and of endothelin-1 (Nakano et al., J. Immunology, 1997; 159:1987-1992; D'Orléans-Juste P et al., Vascular Pharm.49(2008)51-62*).* In addition, chymase leads to degradation of extracellular matrix proteins, such as fibronectin, procollagen and vitronectin, and to the breakoff of focal adhesions (Pejler, J Innate Immun 2020;12:357-372). It brings about activation and release of TGFβ from its latent form, which plays an important role in the genesis of cardiac hypertrophy and cardiac fibrosis (Cho et al. Am J Respir Cell Mol Biol. 2015 Jan; 52(1): 88-95; Oyamada S, et al. J Pharmacol Exp Ther. 2011 Oct; 339(1): 143-51). The enzyme has atherogenic action, by degrading apolipoproteins and preventing the absorption of cholesterol by HDL (Lee et al., ATVB 2002;22:1475-1481). The action of chymase also leads to release and activation of the cytokine interleukin 1 with its pro-inflammatory properties (Mizutani et al., J Exp Med. 1991;174(4):821-825).

The link of chymase to the fibrinolytic system, its potential role and relevance has been unclear so far:
Tchougounova and Pejler investigated the effects of peritoneal mast cells derived from heparin-deficient mice or wildtype mice on the activation and inactivation of thrombin and plasmin in *in vitro* experiments (Tchougounova E, Pejler G., FASEB J. 15(2002)2763-5*).* In their studies, the effects on the regulation of thrombin and plasmin were heparin-dependent and could be inhibited by the unspecific endogenous protease inhibitor α1-antichymotrypsin. Therefore, they attributed these proteolytic findings to chymase or elastase. However, the validity or translatability of these findings is unclear, since several chymase-like enzymes with different relevance for the translation to human are present in murine peritoneal mast cells and no further validation with a specific chymase inhibitors was conducted. In addition, it is suggested that this regulation of thrombin and plasmin may play a role outside of the blood vessel, but not intravascular. This is in contrast to the results of this invention, where the activity of chymase in intravascular fibrin clots and its inhibition play a central role.

Recently, Lipitsä et al. reported histological experiments revealing a colocalization of chymase and fibrin in vasculitis specimens. Subsequent *in vitro* experiments with chymase added to fibrinogen and fibrin revealed a potential slow degradation of fibrinogen and fibrin by chymase, which could be found only in the absence of plasma (Lipitsä T. et al., Brit.J.Dermatol181(2019)296-303*).* It was concluded that chymase itself might have fibrinolytic properties under these circumstances. The hypothesis that chymase might be profibrinolytic and therefore could represent a treatment opportunity in vasculitis is in clear contrast to the findings described in the present invention.

In conclusion, according to the current common knowledge it is very unclear, what is causing the effects of the mast cells in the venous stenosis experiments in mice, whether it might be translatable to arterial models and to clinical situations. What kind of role chymase could play in the circumstances of revascularization or prevention of a thrombotic event has not been investigated at all.

Inhibitors of chymase have been disclosed in WO2013167495, WO2015067650, WO2015067651, and WO0222595 and have been evaluated in several pharmaceutical companies (Ahmad S, Ferrario CM, Expert.Opin.Ther.Pat28(2018)755-764). Fulacimstat (1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid) and TY-51469 ((2-(4-((5-fluoro-3-methylbenzo-[b]thiophene)-2-sulfonamido)-3-(methylsulfonyl)phenyl)thiazole-4-carboxylic acid) are selective chymase inhibitors of different structural classes with IC₅₀ values of 4 nM and 20 nM, respectively. Their selectivity has been tested against various proteases. IC₅₀ values above 2 µM were obtained for plasmin, plasma kallikrein, thrombin, trypsin, tissue plasminogen activator, factor Xa and factor XIa. The closest protease cathepsin G is inhibited by TY-51469 with 60-fold and by fulacimstat with 35-fold less potency than chymase, therefore viewed not relevant in vivo. Both compounds have been evaluated in many preclinical models, covering mostly inflammatory and fibrotic diseases in various organs, including heart, kidney, lung, gut and skin and autoimmune diseases (Pejler G, J.Innate Immun.12(2020)357-372)*.* Fulacimstat is bioavailable after oral intake. The compound was well tolerated in clinical studies without any hints for an impact on bleeding risk and has a favorable pharmacokinetic profile with once-daily dosing regimens (Kanefendt F et al., Clin.Pharmacol.DrugDev.8(2019)467-479)*,* Düngen HD et al., Clin.Pharmacol.DrugDev. 8(2019) 942-951), Düngen HD et al., Am.HeartJ.224(2020)129-137)*,* Rossing P et al., Nephrol. Dial. Transplant26(2021)2263-2273).

For medicaments to be applied in the setting of an acute thrombotic event, rapid lysis of the clot at a low risk for bleeding is of utmost importance.

It is therefore an object of the present invention to provide a novel strategy for the resolution of thrombi to treat thrombotic events in veins, capillaries, arteries and lymph vessels in humans and animals, without increasing the bleeding risk.

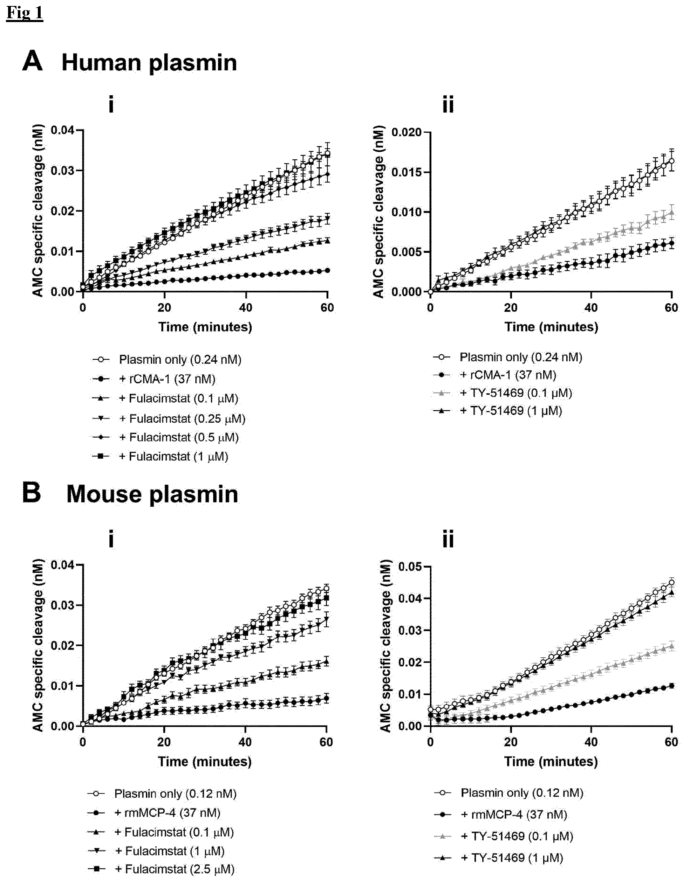

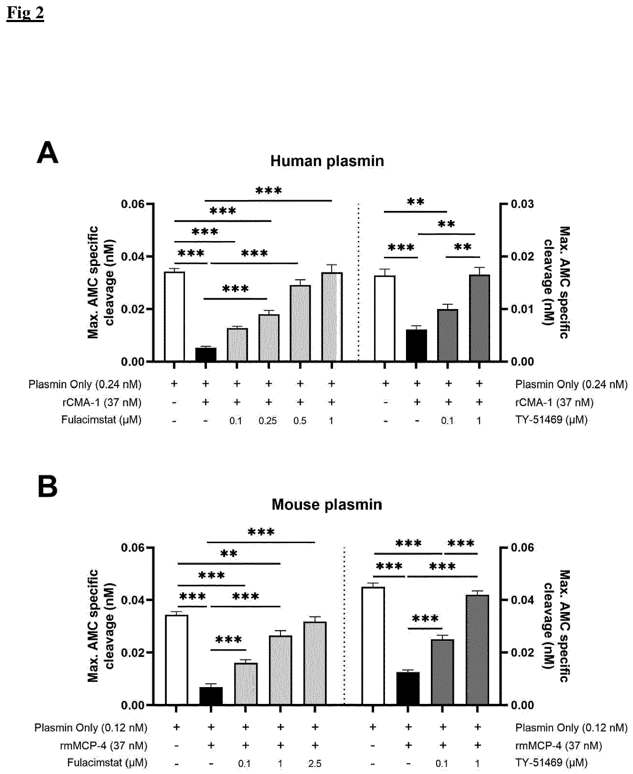

Surprisingly, it has now been found as part of this invention that plasmin is a chymase substrate and this degradation and inactivation of plasmin can be prevented by the chymase inhibitors of the present invention in animals and human (experimental part, section 3; Fig.1 and 2), rendering these chymase inhibitors suitable for thrombus resolution, especially in the cause of acute thrombotic and thromboembolic events.

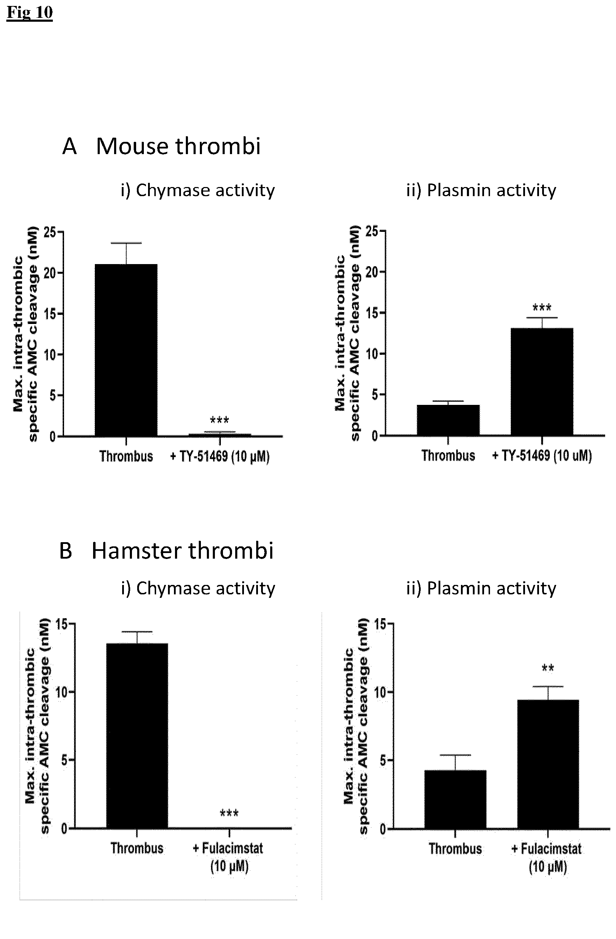

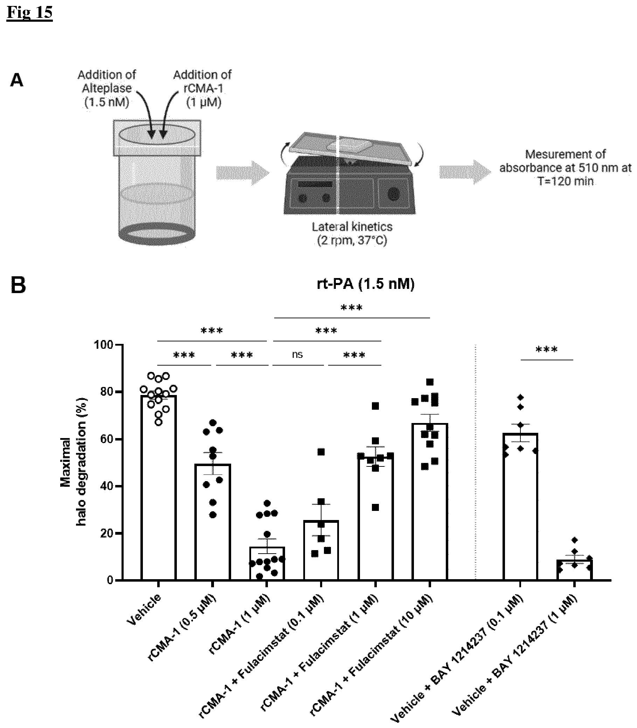

In addition, due to its rapid inactivation in plasma it was not expected that chymase remains active in the local microenvironment of a thrombus, and that the addition of chymase inhibitors of the present invention results in a decrease of chymase activity and an increase of its substrate plasmin in this environment (experimental part, section 9; Fig. 10, 15).

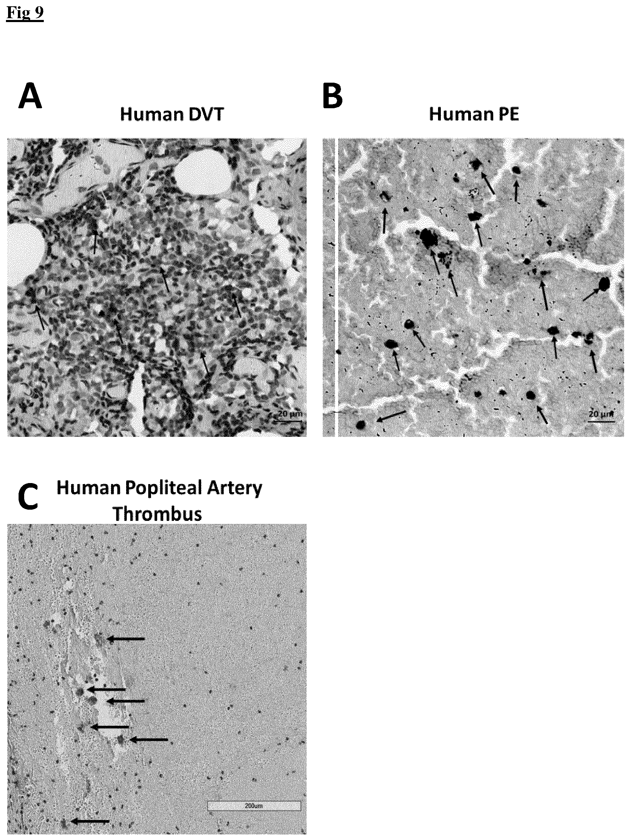

Surprisingly, it could be shown in specimens from human venous, arterial, and pulmonary embolism clots, that chymase is not only present in thrombi from animal experiments, but could be found in human clots, as well, and thereby plays an important role in human thrombotic events (experimental part, section 8; Fig. 9).

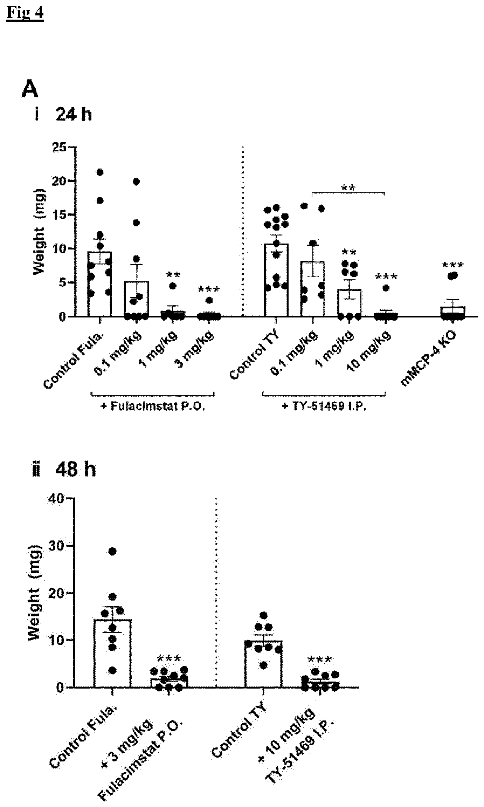

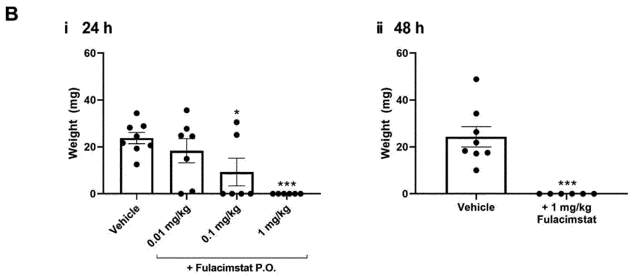

Surprisingly, under conditions of stenosis in the inferior vena cava, administration of a chymase inhibitor of the present invention results in a reduction of thrombus weight and length - even in a clear intervention setting, for instance when given 24 h after the start of thrombus formation. These results were obtained by using two structurally different chymase inhibitors, 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) and TY-51469 in the well-established mouse DVT model as well as with fulacimstat in a newly developed hamster DVT model (both animal models with inferior vena cava stenosis) in order to confirm the general character of these findings (experimental part, section 4; Fig. 4)

While it has been common belief that the principal mechanism behind the murine venous stenosis experiment of Ponomaryov et al. was related to coagulation, activity measurements of chymase and plasmin activity in the removed thrombi from animals in our studies reveal a strong association between chymase and the fibrinolytic system: while chymase activity was reduced by the inhibitors, plasmin activity was increased in the thrombi of these animals (experimental part, section 9.2; Fig. 10).

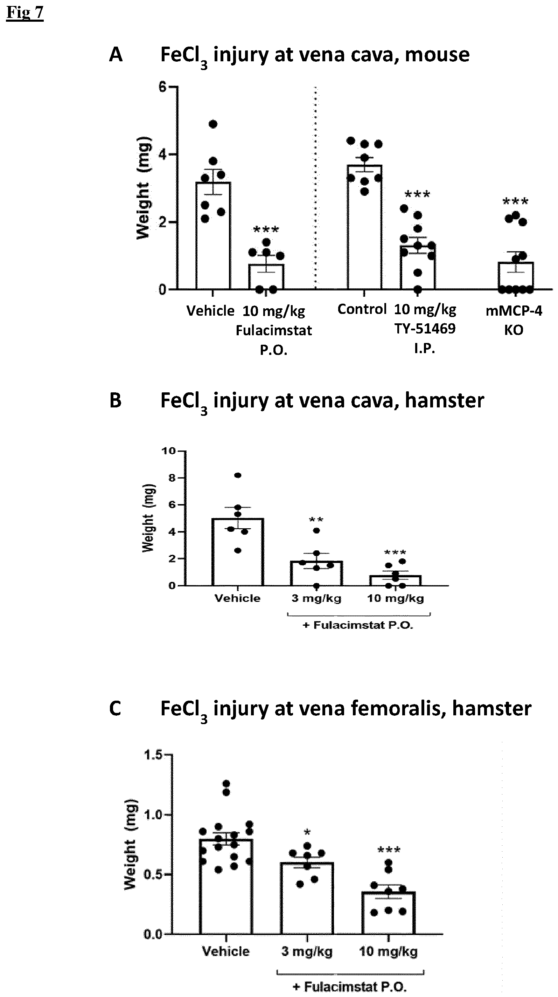

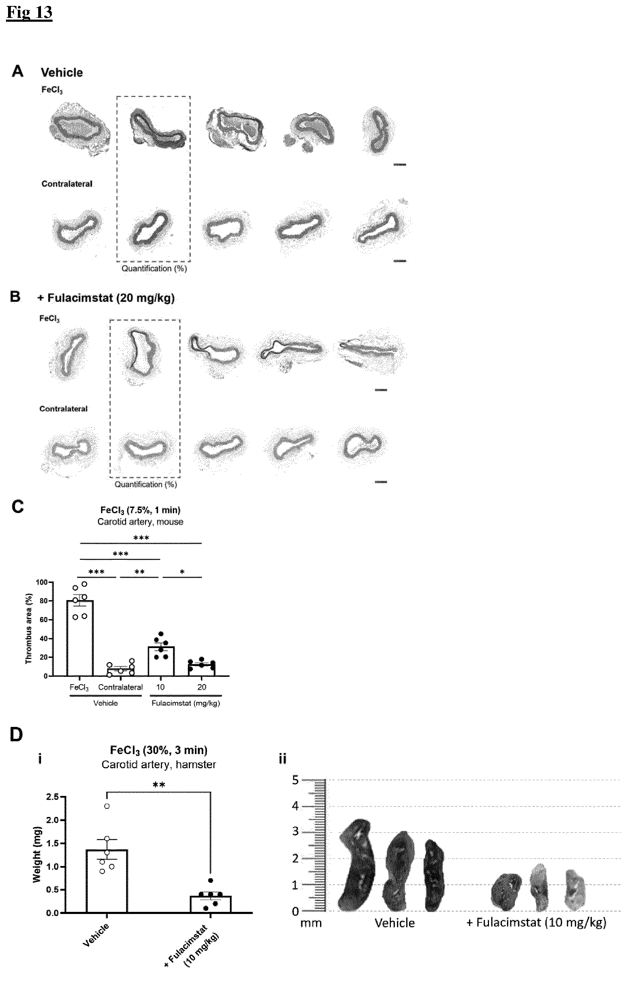

In contrast to the ferric chloride-induced injury experiments in mesenteric arterioles known in the state of the art of Ponomaryov et al., which showed no significant impact of mast cells on thrombus growth, ferric chloride-induced injury experiments at different vessels described in this invention demonstrate significant differences between the groups treated with chymase inhibitors of the present invention vs. the vehicle groups. In these acute thrombosis experiments, the compounds were given before the initiation of the thrombus formation by ferric chloride, implying that even in the early phase of ongoing clot formation chymase inhibitors have an impact on the thrombus weight and thereby on the risk for vessel occlusion (experimental part, section 7; Fig. 7, 13). This was not only observed in venous vessels, but in arteries, e.g. carotid arteries, as well.

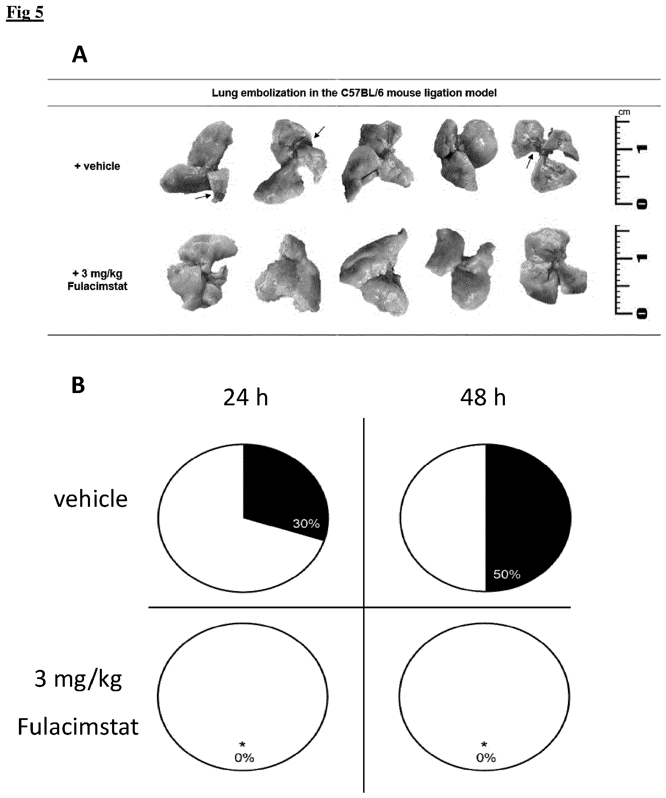

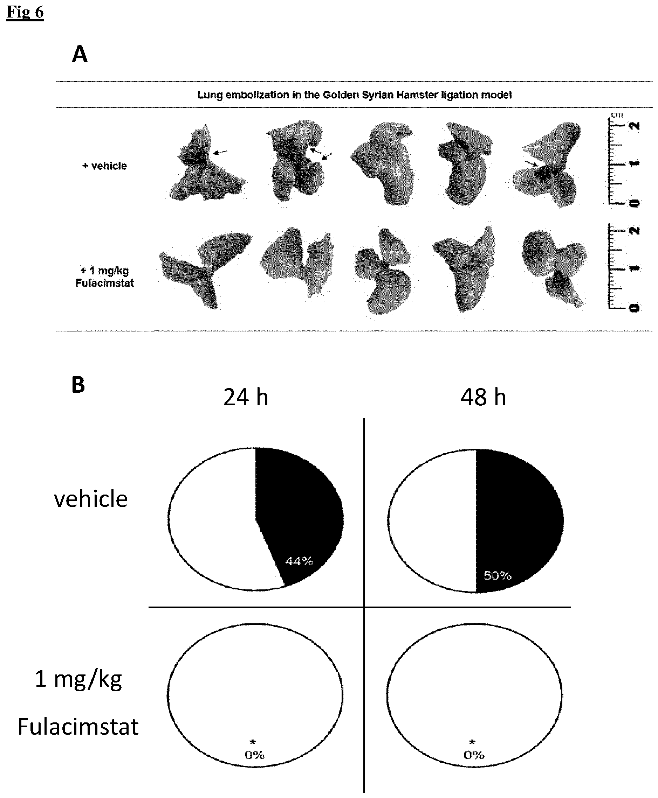

Surprisingly, when examining the lungs in the inferior vena cava (IVC) stenosis experiments in mice and hamsters, a reduced number of pulmonary emboli in the lungs of the animals with chymase inhibition compared to control animals was observed (experimental part, section 6; Fig. 5 and 6). This very important and relevant case of thromboembolism implies that chymase has not only a role at the local site of thrombus formation, but has antifibrinolytic effects in emboli, as well. Therefore, chymase inhibitors are effective agents in thrombo-embolus resolution, including for example the resolution of emboli from deep veins causing pulmonary embolism or from the left atrial appendix in atrial fibrillation patients causing stroke or systemic embolism.

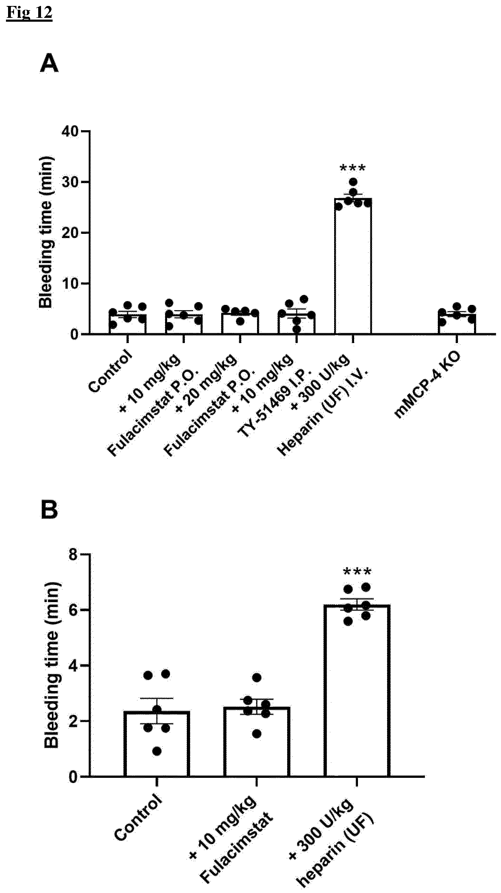

In a tail bleeding model in mice and a femoral vein puncture model in hamster no impact on bleeding times were observed, which is remarkable, because the experiments were conducted with doses, which lead to strong effect in thrombus resolution - making the chymase inhibition approach not only efficacious, but unexpectedly safe, as well (experimental part, section 10; Fig. 12).

The particular importance and surprising finding of this invention is therefore that - by discovering the unexpected impact of chymase on plasmin and its relevance - a mechanism was identified that leads to a reduction in thrombus resolution in specific situations, including inflamed vessel walls with mast cell degranulation and pathologically reduced blood flow, but may not interfere with hemostasis in bleeding events. Therefore, chymase inhibitors of the present invention are not unspecific fibrinolytic agents, but rather novel selective fibrinolysis modulators within thrombi.

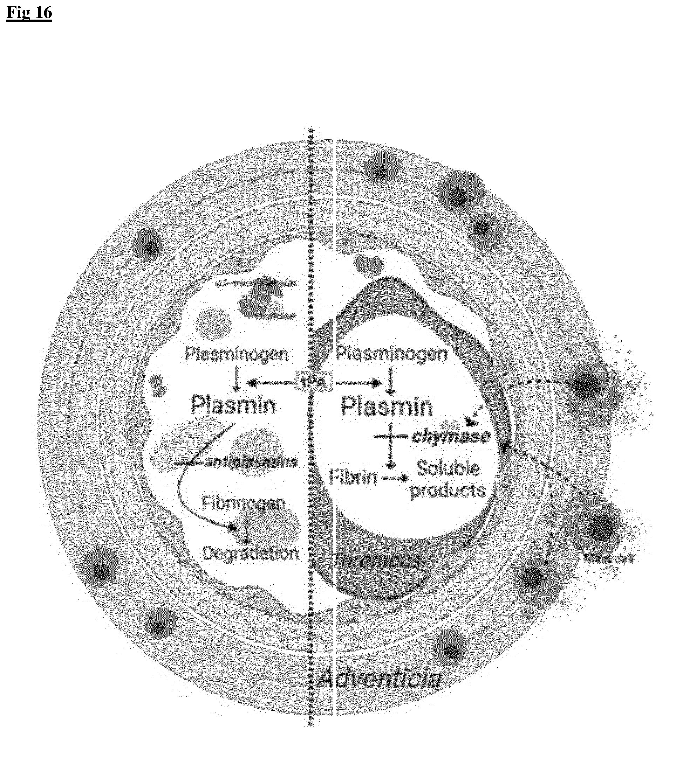

In summary, in various parts of this invention it has been shown for the first time that chymase inhibitors inhibit the degradation of plasmin by chymase in thrombi, which has been identified for the first time as pathophysiologically relevant process in animal and human thrombi with an impact on thrombus size in *in vivo* experiments. Inhibiting the degradation of plasmin via chymase inhibitors of the present invention accelerates the lysis of fibrin clots in vessels without impacting bleeding time or hemostasis and thereby represents a novel approach for safe revascularization of vessels occluded by thrombi or emboli (experimental part, Fig. 16) with the potential to save lives of patients experiencing thrombotic events like stroke, pulmonary embolism or myocardial infarction.

The present invention relates to compounds of the general formula (I) 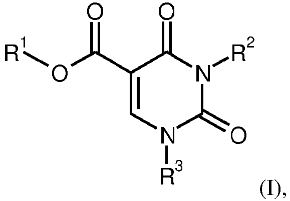 wherein
- R¹: is hydrogen, methyl or ethyl,
- R²: is a group of the formula 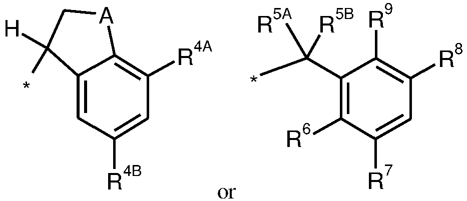 where
* is the attachment site to the uracil nitrogen atom,
A is -CH₂-, -CH₂-CH₂-, -O-CH₂-## or oxygen, in which
## is the attachment site to the phenyl ring,
R^{4A} is hydrogen, fluorine, chlorine, trifluoromethyl or methyl,
R^{4B} is hydrogen, fluorine, chlorine, trifluoromethyl or methyl, with the proviso that at least one of the R^{4A} and R^{4B} radicals is not hydrogen,
R^{5A} is hydrogen,
R^{5B} is hydrogen,
R⁶ is hydrogen,
R⁷ is hydrogen,
R⁸ is fluorine, chlorine, difluoromethyl, trifluoromethyl or methyl,
R⁹ is fluorine, chlorine, difluoromethyl, trifluoromethyl or methyl,
- R³: is a group of the formula 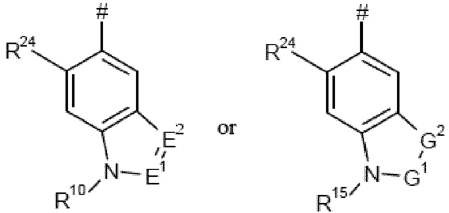 where
# is the attachment site to the uracil nitrogen atom,
E¹ is CR¹¹ or N,
in which
R¹¹ is hydrogen, (C₁-C₄)-alkyl, (C₃-C₇)-cycloalkyl or aminocarbonyl,
- E²: is CR¹² or N,
in which
R¹² is hydrogen, (C₁-C₄)-alkyl or (C₃-C₇)-cycloalkyl,
- G¹: is C=O or SO₂,
- G²: is CR^{16A}R^{16B}, NR¹⁷, O or S,
in which
R^{16A} is hydrogen, fluorine, (C₁-C₄)-alkyl or hydroxyl,
R^{16B} is hydrogen, fluorine, chlorine, (C₁-C₄)-alkyl or trifluoromethyl,
or
R^{16A} and R^{16B} together with the carbon atom to which they are bonded form a 3- to 6-membered carbocycle,
R¹⁷ is hydrogen, (C₁-C₆)-alkyl, (C₃-C₇)-cycloalkyl or (C₁-C₄)-alkoxycarbonyl,
in which (C₁-C₆)-alkyl may be substituted by 1 to 3 substituents independently selected from the group of fluorine, trifluoromethyl, cyano, (C₃-C₇)-cycloalkyl, hydroxyl, trifluoromethoxy, (C₁-C₄)-alkoxy, azetidinyl, oxetanyl, tetrahydrofuranyl and pyrrolidinyl,
- R²⁴: is fluorine or methyl,
- n: is a number 0 or 1,
- R¹⁰: is (C₁-C₄)-alkyl or (C₃-C₇)-cycloalkyl,
in which (C₁-C₄)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
- R¹⁵: is hydrogen, (C₁-C₆)-alkyl or (C₃-C₇)-cycloalkyl,
in which (C₁-C₆)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
and the salts, solvates and solvates of the salts thereof.

The present invention relates to compounds of the general formula (I) 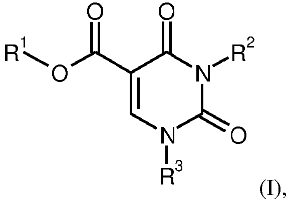 wherein
- R¹: is hydrogen, methyl or ethyl,
- R²: is a group of the formula 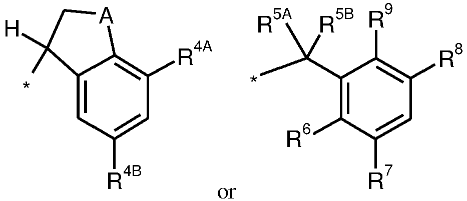 where
* is the attachment site to the uracil nitrogen atom,
A is -CH₂-, -CH₂-CH₂-, -O-CH₂-## or oxygen,
in which
## is the attachment site to the phenyl ring,
R^{4A} is hydrogen, fluorine, chlorine, trifluoromethyl or methyl,
R^{4B} is hydrogen, fluorine, chlorine, trifluoromethyl or methyl, with the proviso that at least one of the R^{4A} and R^{4B} radicals is not hydrogen,
R^{5A} is hydrogen,
R^{5B} is hydrogen,
R⁶ is hydrogen,
R⁷ is hydrogen,
R⁸ is fluorine, chlorine, difluoromethyl, trifluoromethyl or methyl,
R⁹ is fluorine, chlorine, difluoromethyl, trifluoromethyl or methyl,
- R³: is a group of the formula 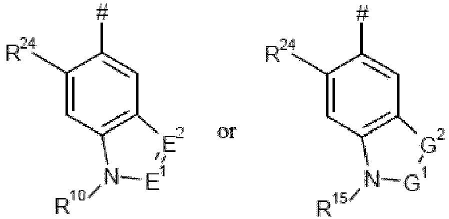 where
# is the attachment site to the uracil nitrogen atom,
E¹ is CR¹¹ or N,
in which
R¹¹ is hydrogen, (C₁-C₄)-alkyl, (C₃-C₇)-cycloalkyl or aminocarbonyl,
E² is CR¹² or N,
in which
R¹² is hydrogen, (C₁-C₄)-alkyl or (C₃-C₇)-cycloalkyl,
G¹ is C=O or SO₂,
G² is CR^{16A}R^{16B}, NR¹⁷, O or S,
in which
R^{16A} is hydrogen, fluorine, (C₁-C₄)-alkyl or hydroxyl,
R^{16B} is hydrogen, fluorine, chlorine, (C₁-C₄)-alkyl or trifluoromethyl,
or
R^{16A} and R^{16B} together with the carbon atom to which they are bonded form a 3- to 6-membered carbocycle,
R¹⁷ is hydrogen, (C₁-C₆)-alkyl, (C₃-C₇)-cycloalkyl or (C₁-C₄)-alkoxycarbonyl,
in which (C₁-C₆)-alkyl may be substituted by 1 to 3 substituents independently selected from the group of fluorine, trifluoromethyl, cyano, (C₃-C₇)-cycloalkyl, hydroxyl, trifluoromethoxy, (C₁-C₄)-alkoxy, azetidinyl, oxetanyl, tetrahydrofuranyl and pyrrolidinyl,
R²⁴ is fluorine or methyl,
n is a number 0 or 1,
R¹⁰ is (C₁-C₄)-alkyl or (C₃-C₇)-cycloalkyl,
in which (C₁-C₄)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
- R¹⁵: is hydrogen, (C₁-C₆)-alkyl or (C₃-C₇)-cycloalkyl,
in which (C₁-C₆)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
and the salts, solvates and solvates of the salts thereof for use in a method for the treatment and/or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention relates to compounds of the general formula (I) 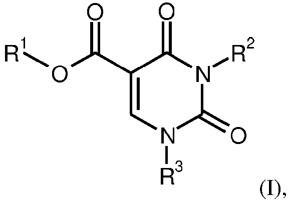 wherein
- R¹: is hydrogen, methyl or ethyl,
- R²: is a group of the formula 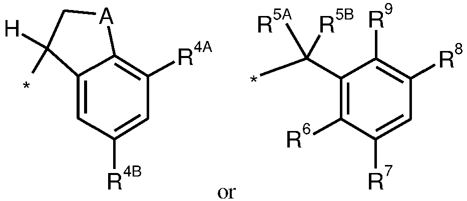 where
* is the attachment site to the uracil nitrogen atom,
A is -CH₂-, -CH₂-CH₂-, -O-CH₂-## or oxygen,
in which
## is the attachment site to the phenyl ring,
R^{4A} is hydrogen, fluorine, chlorine, trifluoromethyl or methyl,
R^{4B} is hydrogen, fluorine, chlorine, trifluoromethyl or methyl, with the proviso that at least one of the R^{4A} and R^{4B} radicals is not hydrogen,
R^{5A} is hydrogen,
R^{5B} is hydrogen,
R⁶ is hydrogen,
R⁷ is hydrogen,
R⁸ is fluorine, chlorine, difluoromethyl, trifluoromethyl or methyl,
R⁹ is fluorine, chlorine, difluoromethyl, trifluoromethyl or methyl,
- R³: is a group of the formula 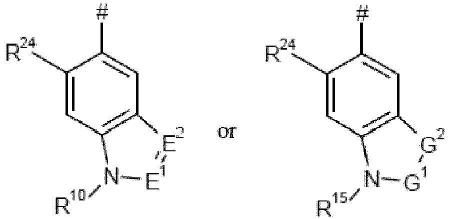 where
# is the attachment site to the uracil nitrogen atom,
E¹ is CR¹¹ or N,
in which
R¹¹ is hydrogen, (C₁-C₄)-alkyl, (C₃-C₇)-cycloalkyl or aminocarbonyl,
E² is CR¹² or N,
in which
R¹² is hydrogen, (C₁-C₄)-alkyl or (C₃-C₇)-cycloalkyl,
G¹ is C=O or SO₂,
G² is CR^{16A}R^{16B}, NR¹⁷, O or S,
in which
R^{16A} is hydrogen, fluorine, (C₁-C₄)-alkyl or hydroxyl,
R^{16B} is hydrogen, fluorine, chlorine, (C₁-C₄)-alkyl or trifluoromethyl,
or
R^{16A} and R^{16B} together with the carbon atom to which they are bonded form a 3- to 6-membered carbocycle,
R¹⁷ is hydrogen, (C₁-C₆)-alkyl, (C₃-C₇)-cycloalkyl or (C₁-C₄)-alkoxycarbonyl,
in which (C₁-C₆)-alkyl may be substituted by 1 to 3 substituents independently selected from the group of fluorine, trifluoromethyl, cyano, (C₃-C₇)-cycloalkyl, hydroxyl, trifluoromethoxy, (C₁-C₄)-alkoxy, azetidinyl, oxetanyl, tetrahydrofuranyl and pyrrolidinyl,
R²⁴ is fluorine or methyl,
n is a number 0 or 1,
R¹⁰ is (C₁-C₄)-alkyl or (C₃-C₇)-cycloalkyl,
in which (C₁-C₄)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
- R¹⁵: is hydrogen, (C₁-C₆)-alkyl or (C₃-C₇)-cycloalkyl,
in which (C₁-C₆)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
and the salts, solvates and solvates of the salts thereof for use in a method for thrombus resolution without bleeding risk and thereby for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention relates to compounds of the general formula (I) 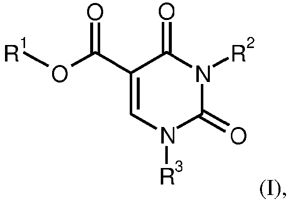 wherein
- R¹: is hydrogen, methyl or ethyl,
- R²: is a group of the formula 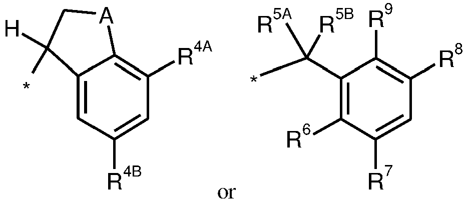 where
* is the attachment site to the uracil nitrogen atom,
A is -CH₂-, -CH₂-CH₂-, -O-CH₂-## or oxygen,
in which
## is the attachment site to the phenyl ring,
R^{4A} is hydrogen, fluorine, chlorine, trifluoromethyl or methyl,
R^{4B} is hydrogen, fluorine, chlorine, trifluoromethyl or methyl, with the proviso that at least one of the R^{4A} and R^{4B} radicals is not hydrogen,
R^{5A} is hydrogen,
R^{5B} is hydrogen,
R⁶ is hydrogen,
R⁷ is hydrogen,
R⁸ is fluorine, chlorine, difluoromethyl, trifluoromethyl or methyl,
R⁹ is fluorine, chlorine, difluoromethyl, trifluoromethyl or methyl,
- R³: is a group of the formula 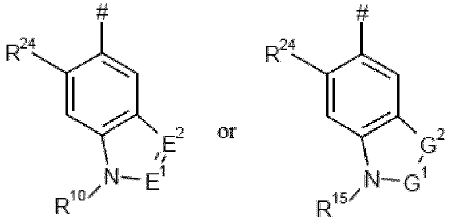 where
# is the attachment site to the uracil nitrogen atom,
E¹ is CR¹¹ or N,
in which
R¹¹ is hydrogen, (C₁-C₄)-alkyl, (C₃-C₇)-cycloalkyl or aminocarbonyl,
E² is CR¹² or N,
in which
R¹² is hydrogen, (C₁-C₄)-alkyl or (C₃-C₇)-cycloalkyl,
G¹ is C=O or SO₂,
G² is CR^{16A}R^{16B}, NR¹⁷, O or S,
in which
R^{16A} is hydrogen, fluorine, (C₁-C₄)-alkyl or hydroxyl,
R^{16B} is hydrogen, fluorine, chlorine, (C₁-C₄)-alkyl or trifluoromethyl,
or
R^{16A} and R^{16B} together with the carbon atom to which they are bonded form a 3- to 6-membered carbocycle,
R¹⁷ is hydrogen, (C₁-C₆)-alkyl, (C₃-C₇)-cycloalkyl or (C₁-C₄)-alkoxycarbonyl,
in which (C₁-C₆)-alkyl may be substituted by 1 to 3 substituents independently selected from the group of fluorine, trifluoromethyl, cyano, (C₃-C₇)-cycloalkyl, hydroxyl, trifluoromethoxy, (C₁-C₄)-alkoxy, azetidinyl, oxetanyl, tetrahydrofuranyl and pyrrolidinyl,
R²⁴ is fluorine or methyl,
n is a number 0 or 1,
R¹⁰ is (C₁-C₄)-alkyl or (C₃-C₇)-cycloalkyl,
in which (C₁-C₄)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
- R¹⁵: is hydrogen, (C₁-C₆)-alkyl or (C₃-C₇)-cycloalkyl,
in which (C₁-C₆)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
and the salts, solvates and solvates of the salts thereof for use in a method for the treatment and/or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention relates to compounds of the general formula (I) 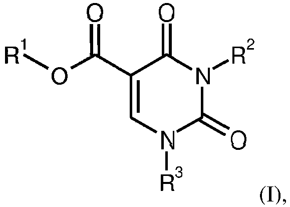 wherein
- R¹: is hydrogen, methyl or ethyl,
- R²: is a group of the formula 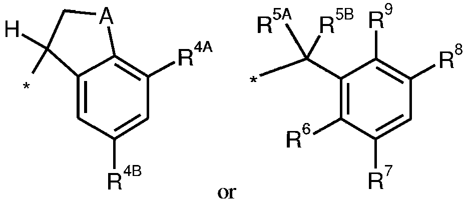 where
* is the attachment site to the uracil nitrogen atom,
A is -CH₂-, -CH₂-CH₂-, -O-CH₂-## or oxygen,
in which
## is the attachment site to the phenyl ring,
R^{4A} is hydrogen, fluorine, chlorine, trifluoromethyl or methyl,
R^{4B} is hydrogen, fluorine, chlorine, trifluoromethyl or methyl, with the proviso that at least one of the R^{4A} and R^{4B} radicals is not hydrogen,
R^{5A} is hydrogen,
R^{5B} is hydrogen,
R⁶ is hydrogen,
R⁷ is hydrogen,
R⁸ is fluorine, chlorine, difluoromethyl, trifluoromethyl or methyl,
R⁹ is fluorine, chlorine, difluoromethyl, trifluoromethyl or methyl,
- R³: is a group of the formula 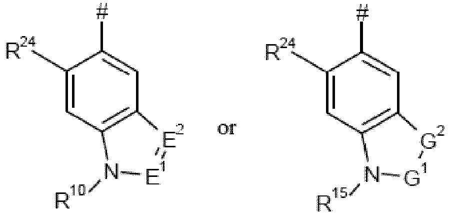 where
# is the attachment site to the uracil nitrogen atom,
E¹ is CR¹¹ or N,
in which
R¹¹ is hydrogen, (C₁-C₄)-alkyl, (C₃-C₇)-cycloalkyl or aminocarbonyl,
E² is CR¹² or N,
in which
R¹² is hydrogen, (C₁-C₄)-alkyl or (C₃-C₇)-cycloalkyl,
G¹ is C=O or SO₂,
G² is CR^{16A}R^{16B}, NR¹⁷, O or S,
in which
R^{16A} is hydrogen, fluorine, (C₁-C₄)-alkyl or hydroxyl,
R^{16B} is hydrogen, fluorine, chlorine, (C₁-C₄)-alkyl or trifluoromethyl,
or
R^{16A} and R^{16B} together with the carbon atom to which they are bonded form a 3- to 6-membered carbocycle,
R¹⁷ is hydrogen, (C₁-C₆)-alkyl, (C₃-C₇)-cycloalkyl or (C₁-C₄)-alkoxycarbonyl,
in which (C₁-C₆)-alkyl may be substituted by 1 to 3 substituents independently selected from the group of fluorine, trifluoromethyl, cyano, (C₃-C₇)-cycloalkyl, hydroxyl, trifluoromethoxy, (C₁-C₄)-alkoxy, azetidinyl, oxetanyl, tetrahydrofuranyl and pyrrolidinyl,
R²⁴ is fluorine or methyl,
n is a number 0 or 1,
R¹⁰ is (C₁-C₄)-alkyl or (C₃-C₇)-cycloalkyl,
in which (C₁-C₄)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
R¹⁵ is hydrogen, (C₁-C₆)-alkyl or (C₃-C₇)-cycloalkyl,
in which (C₁-C₆)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
and the salts, solvates and solvates of the salts thereof for use in a method for thrombus resolution without bleeding risk and thereby for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention relates to compounds of the general formula (I) 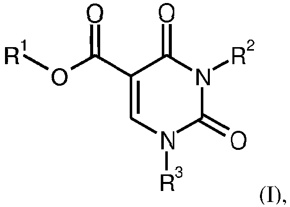 wherein
- R¹: is hydrogen, methyl or ethyl,
- R²: is a group of the formula 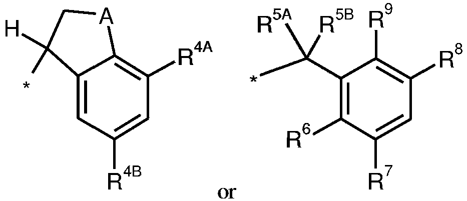 where
* is the attachment site to the uracil nitrogen atom,
A is -CH₂-, -CH₂-CH₂-, -O-CH₂-## or oxygen,
in which
## is the attachment site to the phenyl ring,
R^{4A} is hydrogen, fluorine, chlorine, trifluoromethyl or methyl,
R^{4B} is hydrogen, fluorine, chlorine, trifluoromethyl or methyl, with the proviso that at least one of the R^{4A} and R^{4B} radicals is not hydrogen,
R^{5A} is hydrogen,
R^{5B} is hydrogen,
R⁶ is hydrogen,
R⁷ is hydrogen,
R⁸ is fluorine, chlorine, difluoromethyl, trifluoromethyl or methyl,
R⁹ is fluorine, chlorine, difluoromethyl, trifluoromethyl or methyl,
- R³: is a group of the formula 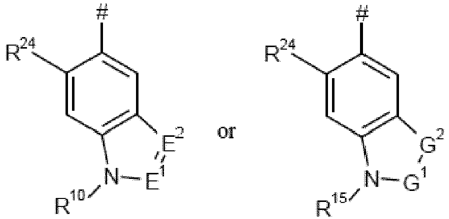 where
# is the attachment site to the uracil nitrogen atom,
E¹ is CR¹¹ or N,
in which
R¹¹ is hydrogen, (C₁-C₄)-alkyl, (C₃-C₇)-cycloalkyl or aminocarbonyl,
E² is CR¹² or N,
in which
R¹² is hydrogen, (C₁-C₄)-alkyl or (C₃-C₇)-cycloalkyl,
G¹ is C=O or SO₂,
G² is CR^{16A}R^{16B}, NR¹⁷, O or S,
in which
R^{16A} is hydrogen, fluorine, (C₁-C₄)-alkyl or hydroxyl,
R^{16B} is hydrogen, fluorine, chlorine, (C₁-C₄)-alkyl or trifluoromethyl,
or
R^{16A} and R^{16B} together with the carbon atom to which they are bonded form a 3- to 6-membered carbocycle,
R¹⁷ is hydrogen, (C₁-C₆)-alkyl, (C₃-C₇)-cycloalkyl or (C₁-C₄)-alkoxycarbonyl,
in which (C₁-C₆)-alkyl may be substituted by 1 to 3 substituents independently selected from the group of fluorine, trifluoromethyl, cyano, (C₃-C₇)-cycloalkyl, hydroxyl, trifluoromethoxy, (C₁-C₄)-alkoxy, azetidinyl, oxetanyl, tetrahydrofuranyl and pyrrolidinyl,
R²⁴ is fluorine or methyl,
n is a number 0 or 1,
R¹⁰ is (C₁-C₄)-alkyl or (C₃-C₇)-cycloalkyl,
in which (C₁-C₄)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
- R¹⁵: is hydrogen, (C₁-C₆)-alkyl or (C₃-C₇)-cycloalkyl,
in which (C₁-C₆)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
and the salts, solvates and solvates of the salts thereof for use in a method for the treatment and/or prophylaxis of stroke, or pulmonary embolism.

The present invention relates to compounds of the general formula (I) 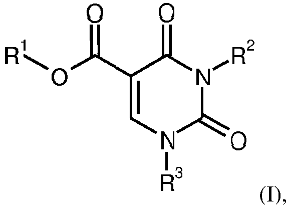 wherein
- R¹: is hydrogen, methyl or ethyl,
- R²: is a group of the formula 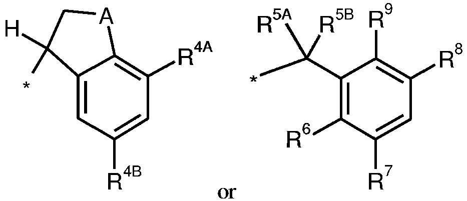 where
* is the attachment site to the uracil nitrogen atom,
A is -CH₂-, -CH₂-CH₂-, -O-CH₂-## or oxygen,
in which
## is the attachment site to the phenyl ring,
R^{4A} is hydrogen, fluorine, chlorine, trifluoromethyl or methyl,
R^{4B} is hydrogen, fluorine, chlorine, trifluoromethyl or methyl, with the proviso that at least one of the R^{4A} and R^{4B} radicals is not hydrogen,
R^{5A} is hydrogen,
R^{5B} is hydrogen,
R⁶ is hydrogen,
R⁷ is hydrogen,
R⁸ is fluorine, chlorine, difluoromethyl, trifluoromethyl or methyl,
R⁹ is fluorine, chlorine, difluoromethyl, trifluoromethyl or methyl,
- R³: is a group of the formula 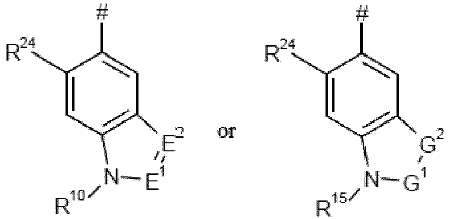 where
# is the attachment site to the uracil nitrogen atom,
E¹ is CR¹¹ or N,
in which
R¹¹ is hydrogen, (C₁-C₄)-alkyl, (C₃-C₇)-cycloalkyl or aminocarbonyl,
E² is CR¹² or N,
in which
R¹² is hydrogen, (C₁-C₄)-alkyl or (C₃-C₇)-cycloalkyl,
G¹ is C=O or SO₂,
G² is CR^{16A}R^{16B}, NR¹⁷, O or S,
in which
R^{16A} is hydrogen, fluorine, (C₁-C₄)-alkyl or hydroxyl,
R^{16B} is hydrogen, fluorine, chlorine, (C₁-C₄)-alkyl or trifluoromethyl, or
R^{16A} and R^{16B} together with the carbon atom to which they are bonded form a 3- to 6-membered carbocycle,
R¹⁷ is hydrogen, (C₁-C₆)-alkyl, (C₃-C₇)-cycloalkyl or (C₁-C₄)-alkoxycarbonyl,
in which (C₁-C₆)-alkyl may be substituted by 1 to 3 substituents independently selected from the group of fluorine, trifluoromethyl, cyano, (C₃-C₇)-cycloalkyl, hydroxyl, trifluoromethoxy, (C₁-C₄)-alkoxy, azetidinyl, oxetanyl, tetrahydrofuranyl and pyrrolidinyl,
R²⁴ is fluorine or methyl,
n is a number 0 or 1,
R¹⁰ is (C₁-C₄)-alkyl or (C₃-C₇)-cycloalkyl,
in which (C₁-C₄)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
- R¹⁵: is hydrogen, (C₁-C₆)-alkyl or (C₃-C₇)-cycloalkyl,
in which (C₁-C₆)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
and the salts, solvates and solvates of the salts thereof for use in a method for thrombus resolution without bleeding risk and thereby for the thrombus resolution in stroke, or pulmonary embolism.

In a further embodiment the present invention covers compounds of general formula (I), supra, in which
- R¹: is hydrogen, methyl or ethyl,
- R²: is a group of the formula 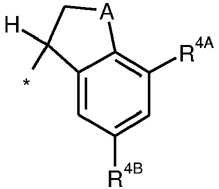 where
* is the attachment site to the uracil nitrogen atom,
A **is** -CH₂-,
R^{4A} is chlorine or trifluoromethyl,
R^{4B} is hydrogen,
- R³: is a group of the formula 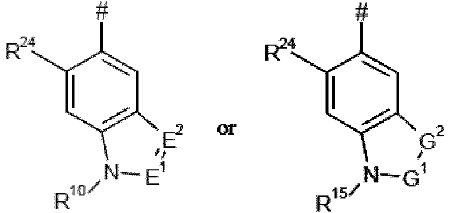 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl, in which methyl and ethyl may be substituted by 1 substituent selected from the group of fluorine, trifluoromethyl and cyclopropyl,
and the salts, solvates and solvates of the salts thereof for use in a method for the treatment and/or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

In a further embodiment the present invention covers compounds of general formula (I), supra, in which
- R¹: is hydrogen, methyl or ethyl,
- R²: is a group of the formula 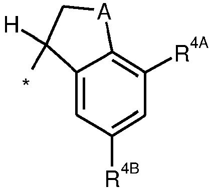 where
* is the attachment site to the uracil nitrogen atom,
A **is** -CH₂-,
R^{4A} is chlorine or trifluoromethyl,
R^{4B} is hydrogen,
- R³: is a group of the formula 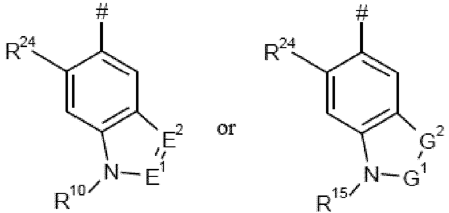 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for thrombus resolution without bleeding risk and thereby for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

In a further embodiment the present invention covers compounds of general formula (I), supra, in which
- R¹: is hydrogen, methyl or ethyl,
- R²: is a group of the formula 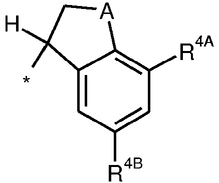 where
* is the attachment site to the uracil nitrogen atom,
A **is** -CH₂-,
R^{4A} is chlorine or trifluoromethyl,
R^{4B} is hydrogen,
- R³: is a group of the formula 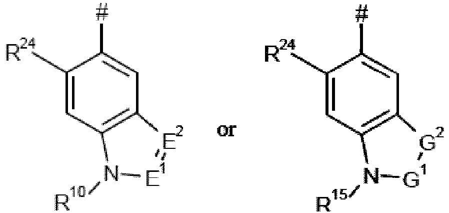 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for the treatment and/or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

In a further embodiment the present invention covers compounds of general formula (I), supra, in which
- R¹: is hydrogen, methyl or ethyl,
- R²: is a group of the formula 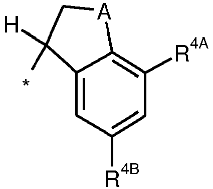 where
* is the attachment site to the uracil nitrogen atom,
A **is** -CH₂-,
R^{4A} is chlorine or trifluoromethyl,
R^{4B} is hydrogen,
- R³: is a group of the formula 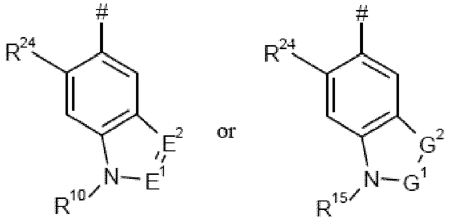 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for thrombus resolution without bleeding risk and thereby for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

In a further embodiment the present invention covers compounds of general formula (I), supra, in which
- R¹: is hydrogen, methyl or ethyl,
- R²: is a group of the formula 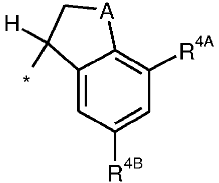 where
* is the attachment site to the uracil nitrogen atom,
A **is** -CH₂-,
R^{4A} is chlorine or trifluoromethyl,
R^{4B} is hydrogen,
- R³: is a group of the formula 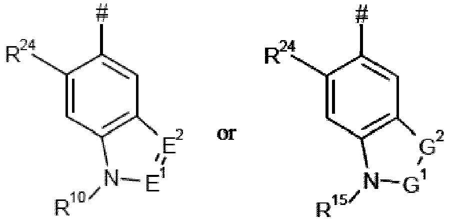 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for the treatment and/or prophylaxis of stroke, or pulmonary embolism

In a further embodiment the present invention covers compounds of general formula (I), supra, in which
- R¹: is hydrogen, methyl or ethyl,
- R²: is a group of the formula 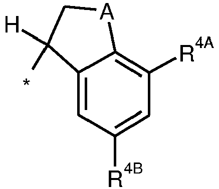 where
* is the attachment site to the uracil nitrogen atom,
A **is** -CH₂-,
R^{4A} is chlorine or trifluoromethyl,
R^{4B} is hydrogen,
- R³: is a group of the formula 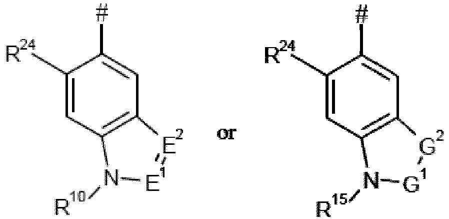 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for thrombus resolution without bleeding risk and thereby for the thrombus resolution in stroke, or pulmonary embolism.

Inventive compounds are the compounds of the formula (I) and the salts, solvates and solvates of the salts thereof, the compounds encompassed by formula (I) of the formulae specified hereinafter and the salts, solvates and solvates of the salts thereof, and the compounds encompassed by formula (I) and specified hereinafter as working examples and the salts, solvates and solvates of the salts thereof, to the extent that the compounds encompassed by formula (I) and specified hereinafter are not already salts, solvates and solvates of the salts.

In the context of the present invention, preferred salts are physiologically acceptable salts of the inventive compounds. Also encompassed are salts which are not themselves suitable for pharmaceutical applications but can be used, for example, for the isolation, purification or storage of the inventive compounds.

Physiologically acceptable salts of the inventive compounds include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphon-ic acid, naphthalenedisulphonic acid, acetic acid, trifluoroacetic acid, propionic acid, lactic acid, tartaric ac-id, malic acid, citric acid, fumaric acid, maleic acid and benzoic acid.

Physiologically acceptable salts of the inventive compounds also include salts of conventional bases, by way of example and with preference alkali metal salts (e.g. sodium and potassium salts), alkaline earth metal salts (e.g. calcium and magnesium salts) and ammonium salts derived from ammonia or organic amines having 1 to 16 carbon atoms, by way of example and with preference ethylamine, diethylamine, triethylamine, N,N-ethyldiisopropylamine, monoethanolamine, diethanolamine, triethanolamine, dimethylaminoethanol, diethylaminoethanol, procaine, dicyclohexylamine, dibenzylamine, N-methylpiperidine, N-methylmorpholine, arginine, lysine, choline and 1,2-ethylenediamine.

In the context of the invention, solvates refer to those forms of the inventive compounds which, in solid or liquid state, form a complex by coordination with solvent molecules. Hydrates are a specific form of the solvates in which the coordination is with water. Preferred solvates in the context of the present invention are hydrates.

Depending on their structure, the inventive compounds may exist in different stereoisomeric forms, i.e. in the form of configurational isomers or if appropriate also as conformational isomers (enantiomers and/or diastereomers, including those in the case of atropisomers). The present invention therefore encompasses the enantiomers or diastereomers and the respective mixtures thereof. The stereoisomerically homogeneous contituents can be isolated from such mixtures of enantiomers and/or diastereomers in a known manner; chromatography processes are preferably used for this purpose, especially HPLC chromatography on an achiral or chiral phase.

Where the inventive compounds can occur in tautomeric forms, the present invention encompasses all the tautomeric forms.

The present invention also encompasses all suitable isotopic variants of the inventive compounds. An isotopic variant of an inventive compound is understood here to mean a compound in which at least one atom within the inventive compound has been exchanged for another atom of the same atomic number, but with a different atomic mass than the atomic mass which usually or predominantly occurs in nature. Examples of isotopes which can be incorporated into an inventive compound are those of hydrogen, carbon, nitrogen, oxygen, phosphorus, sulphur, fluorine, chlorine, bromine and iodine, such as 2H (deuterium), 3H (tritium), 13C, 14C, 15N, 170, 180, 32P, 33P, 33S, 34S, 35S, 36S, 18F, 36Cl, 82Br, 123I, 124I, 129I and 131I. Particular isotop-ic variants of an inventive compound, especially those in which one or more radioactive isotopes have been incorporated, may be beneficial, for example, for the examination of the mechanism of action or of the ac-tive ingredient distribution in the body; due to comparatively easy preparability and detectability, especially compounds labelled with 3H or 14C isotopes are suitable for this purpose. In addition, the incorporation of isotopes, for example of deuterium, can lead to particular therapeutic benefits as a consequence of greater metabolic stability of the compound, for example an extension of the half-life in the body or a reduction in the active dose required; such modifications of the inventive compounds may therefore in some cases also constitute a preferred embodiment of the present invention. Isotopic variants of the inventive compounds can be prepared by processes known to those skilled in the art, for example by the methods described below and the instructions reproduced in the working examples, by using corresponding isotopic modifications of the particular reagents and/or starting compounds therein.

In the context of the present invention, unless specified otherwise, the substituents are each defined as fol-lows:
Alkyl in the context of the invention is a linear or branched alkyl radical having the number of carbon at-oms specified in each case. Preferred examples include: methyl, ethyl, n-propyl, isopropyl, n-butyl, isobu-tyl, 1-methylpropyl, tert-butyl, n-pentyl, isopentyl, 1-ethylpropyl, 1-methylbutyl, 2-methylbutyl, 3-methylbutyl, n-hexyl, 1-methylpentyl, 2-methylpentyl, 3-methylpentyl, 4-methylpentyl, 3,3-dimethylbutyl, 1-ethylbutyl, 2-ethylbutyl, 1,4-dimethylpentyl, 4,4-dimethylpentyl and 1,4,4-trimethylpentyl.

Cycloalkyl in the context of the invention is a monocyclic saturated alkyl radical having 3 to 7 carbon at-oms. Preferred examples include: cyclopropyl, cyclobutyl, cyclopentyl, cyclohexyl and cycloheptyl.

Alkylcarbonyl in the context of the invention is a linear or branched alkyl radical having 1 to 4 carbon at-oms and a carbonyl group attached in the 1 position. Preferred examples include: methylcarbonyl, ethylcar-bonyl, n propylcarbonyl, isopropylcarbonyl, n-butylcarbonyl, isobutylcarbonyl and tert-butylcarbonyl.

Alkoxy in the context of the invention is a linear or branched alkoxy radical having 1 to 4 carbon atoms. Preferred examples include: methoxy, ethoxy, n-propoxy, isopropoxy, n-butoxy and tert-butoxy.

Alkoxycarbonyl in the context of the invention is a linear or branched alkoxy radical having 1 to 4 carbon atoms and a carbonyl group attached to the oxygen. Preferred examples include: methoxycarbonyl, ethox-ycarbonyl, n-propoxycarbonyl, isopropoxycarbonyl and tert-butoxycarbonyl.

Alkylthio in the context of the invention is a linear or branched alkyl radical which has 1 to 4 carbon atoms and is bonded via a sulphur atom. Preferred examples include: methylthio, ethylthio, n-propylthio, iso-propylthio, 1-methylpropylthio, n-butylthio, iso-butylthio and tert-butylthio.

Alkylsulphonyl in the context of the invention is a linear or branched alkyl radical which has 1 to 4 carbon atoms and is bonded via a sulphonyl group. Preferred examples include: methylsulphonyl, ethylsulphonyl, n-propylsulphonyl, isopropylsulphonyl, n-butylsulphonyl and tert-butylsulphonyl.

4- to 7-membered heterocyclyl in the context of the invention is a monocyclic saturated heterocycle which has a total of 4 to 7 ring atoms, contains one or two ring heteroatoms from the group of N, O, S, SO and/or SO2 and is attached via a ring carbon atom or, where appropriate, a ring nitrogen atom. Examples include: azetidinyl, oxetanyl, pyrrolidinyl, pyrazolidinyl, tetrahydrofuranyl, piperidinyl, piperazinyl, tetrahydropy-ranyl, morpholinyl, thiomorpholinyl. Preference is given to: azetidinyl, oxetanyl, pyrrolidinyl, tetrahydro-furanyl, piperidinyl, piperazinyl, tetrahydropyranyl and morpholinyl.

5- to 7 membered heterocyclyl in the context of the invention is a partly unsaturated heterocycle which has a total of 5 to 7 ring atoms, contains 1 to 3 ring heteroatoms from the group of N, O, S and/or SO2 and is fused to the phenyl ring in R3. Examples include: dihydropyrrolyl, dihydroimidazolyl, dihydrothiazole di-oxide, dihydrooxazolyl, dihydropyridyl, tetrahydropyrazinyl and dihydrooxazinyl.

Heteroaryl in the context of the invention is a monocyclic aromatic heterocycle (heteroaromatic) which has a total of 5 or 6 ring atoms, contains up to three identical or different ring heteroatoms from the group of N, O and/or S and is fused to the phenyl ring in R3. Examples include: furyl, pyrrolyl, thienyl, pyrazolyl, imidazolyl, thiazolyl, oxazolyl, isoxazolyl, isothiazolyl, triazolyl, oxadiazolyl, thiadiazolyl, pyridyl, pyrim-idinyl, pyridazinyl, pyrazinyl and triazinyl. Preference is given to pyrazolyl, imidazolyl, thiazolyl and tria-zolyl.

Halogen in the context of the invention includes fluorine, chlorine, bromine and iodine. Preference is given to chlorine or fluorine.

An oxo group in the context of the invention is an oxygen atom bonded via a double bond to a carbon or sulphur atom.

In the formulae of the group that R² and R³ may represent, the end point of the line marked by a symbol * or # or ## does not represent a carbon atom or a CH2 group but is part of the bond to the respective atom to which R² and R³ are bonded.

If radicals in the compounds according to the invention are substituted, the radicals may be mono-or pol-ysubstituted, unless specified otherwise. In the context of the present invention, all radicals which occur more than once are defined independently of one another. Substitution by one or two identical or different substituents is preferred. Very particular preference is given to substitution by one substituent.

In the context of the present invention, the term "treatment" or "treating" includes inhibition, retardation, checking, alleviating, attenuating, restricting, reducing, suppressing, repelling or healing of a disease, a condition, a disorder, an injury or a health problem, or the development, the course or the progress of such states and/or the symptoms of such states. The term "therapy" is understood here to be synonymous with the term "treatment".

The terms "prevention", "prophylaxis" or "preclusion" are used synonymously in the context of the present invention and refer to the avoidance or reduction of the risk of contracting, experiencing, suffering from or having a disease, a condition, a disorder, an injury or a health problem, or a development or advancement of such states and/or the symptoms of such states.

The treatment or prevention of a disease, a condition, a disorder, an injury or a health problem may be partial or complete.

In a further embodiment the present invention covers compounds of general formula (I), supra, in which
- R¹: is hydrogen,
- R²: is a group of the formula 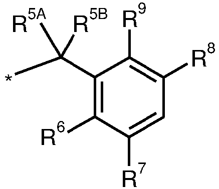 where
* is the attachment site to the uracil nitrogen atom,
R^{5A} is hydrogen,
R^{5B} is hydrogen,
R⁶ is hydrogen,
R⁷ is hydrogen,
R⁸ is fluorine, chlorine or trifluoromethyl,
R⁹ is fluorine, chlorine, trifluoromethyl or methyl,
- R³: is a group of the formula 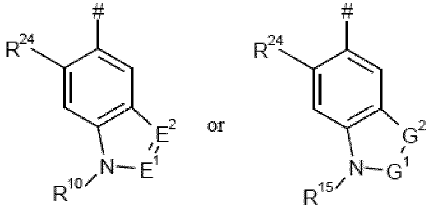 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for the treatment and/or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

In a further embodiment the present invention covers compounds of general formula (I), supra, in which
- R¹: is hydrogen,
- R²: is a group of the formula 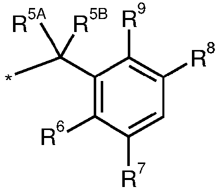 where
* is the attachment site to the uracil nitrogen atom,
R^{5A} is hydrogen,
R^{5B} is hydrogen,
R⁶ is hydrogen,
R⁷ is hydrogen,
R⁸ is fluorine, chlorine or trifluoromethyl,
R⁹ is fluorine, chlorine, trifluoromethyl or methyl,
- R³: is a group of the formula 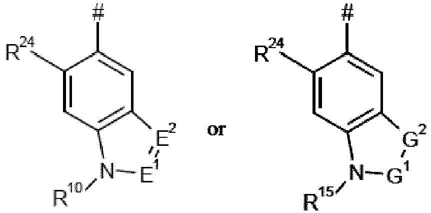 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for thrombus resolution without bleeding risk and thereby for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

In a further embodiment the present invention covers compounds of general formula (I), supra, in which
- R¹: is hydrogen,
- R²: is a group of the formula 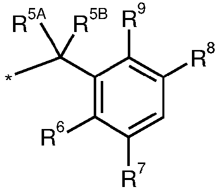 where
* is the attachment site to the uracil nitrogen atom,
R^{5A} is hydrogen,
R^{5B} is hydrogen,
R⁶ is hydrogen,
R⁷ is hydrogen,
R⁸ is fluorine, chlorine or trifluoromethyl,
R⁹ is fluorine, chlorine, trifluoromethyl or methyl,
- R³: is a group of the formula 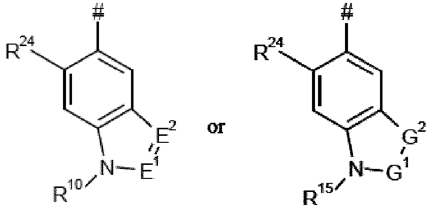 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for the treatment and/or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

In a further embodiment the present invention covers compounds of general formula (I), supra, in which
- R¹: is hydrogen,
- R²: is a group of the formula 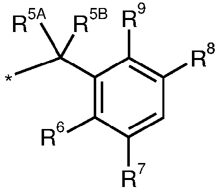 where
* is the attachment site to the uracil nitrogen atom,
R^{5A} is hydrogen,
R^{5B} is hydrogen,
R⁶ is hydrogen,
R⁷ is hydrogen,
R⁸ is fluorine, chlorine or trifluoromethyl,
R⁹ is fluorine, chlorine, trifluoromethyl or methyl,
- R³: is a group of the formula 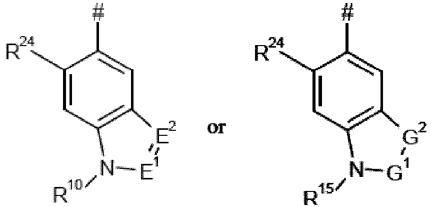 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for thrombus resolution without bleeding risk and thereby for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

In a further embodiment the present invention covers compounds of general formula (I), supra, in which
- R¹: is hydrogen,
- R²: is a group of the formula 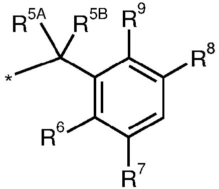 where
* is the attachment site to the uracil nitrogen atom,
R^{5A} is hydrogen,
R^{5B} is hydrogen,
R⁶ is hydrogen,
R⁷ is hydrogen,
R⁸ is fluorine, chlorine or trifluoromethyl,
R⁹ is fluorine, chlorine, trifluoromethyl or methyl,
- R³: is a group of the formula 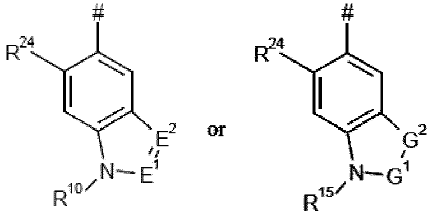 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for the treatment and/or prophylaxis of stroke, pulmonary embolism

In a further embodiment the present invention covers compounds of general formula (I), supra, in which
- R¹: is hydrogen,
- R²: is a group of the formula 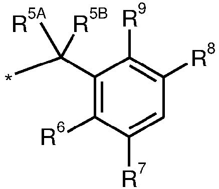 where
* is the attachment site to the uracil nitrogen atom,
R^{5A} is hydrogen,
R^{5B} is hydrogen,
R⁶ is hydrogen,
R⁷ is hydrogen,
R⁸ is fluorine, chlorine or trifluoromethyl,
R⁹ is fluorine, chlorine, trifluoromethyl or methyl,
- R³: is a group of the formula 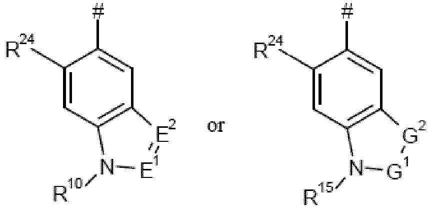 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof for use in a method for thrombus resolution without bleeding risk and thereby for the thrombus resolution in stroke, or pulmonary embolism.

Preference is given in the context of the present invention to a compound selected from the group of 1-(1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(6-fluoro-1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 2,4-dioxo-3-[(1R)-4-(trifluoromethyl)-2,3-dihydro-1H-inden-1-yl]-1-(1,3,3-trimethyl-2-oxo-2,3-dihydro-1H-indol-5-yl)-1,2,3,4-tetrahydropyrimidine-5-carboxylic acid (R enantiomer), 1-(1'-methyl-2'-oxo-1',2'-dihydrospiro[cyclopropane-1,3'-indole]-5'-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzothiazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and ethyl 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylate (R enantiomer) for use in a method for the treatment and/or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

Preference is given in the context of the present invention to a compound selected from the group of 1-(1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(6-fluoro-1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 2,4-dioxo-3-[(1R)-4-(trifluoromethyl)-2,3-dihydro-1H-inden-1-yl]-1-(1,3,3-trimethyl-2-oxo-2,3-dihydro-1H-indol-5-yl)-1,2,3,4-tetrahydropyrimidine-5-carboxylic acid (R enantiomer), 1-(1'-methyl-2'-oxo-1',2'-dihydrospiro[cyclopropane-1,3'-indole]-5'-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzothiazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and ethyl 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylate (R enantiomer) and the salts, solvates and solvates of the salts thereof for use in a method for thrombus resolution without bleeding risk and thereby for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

Preference is given in the context of the present invention to a compound selected from the group of 1-(1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(6-fluoro-1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 2,4-dioxo-3-[(1R)-4-(trifluoromethyl)-2,3-dihydro-1H-inden-1-yl]-1-(1,3,3-trimethyl-2-oxo-2,3-dihydro-1H-indol-5-yl)-1,2,3,4-tetrahydropyrimidine-5-carboxylic acid (R enantiomer), 1-(1'-methyl-2'-oxo-1',2'-dihydrospiro[cyclopropane-1,3'-indole]-5'-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzothiazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and ethyl 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylate (R enantiomer) for use in a method for the treatment and/or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

Preference is given in the context of the present invention to a compound selected from the group of 1-(1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(6-fluoro-1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 2,4-dioxo-3-[(1R)-4-(trifluoromethyl)-2,3-dihydro-1H-inden-1-yl]-1-(1,3,3-trimethyl-2-oxo-2,3-dihydro-1H-indol-5-yl)-1,2,3,4-tetrahydropyrimidine-5-carboxylic acid (R enantiomer), 1-(1'-methyl-2'-oxo-1',2'-dihydrospiro[cyclopropane-1,3'-indole]-5'-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzothiazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and ethyl 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylate (R enantiomer) and the salts, solvates and solvates of the salts thereof for use in a method for thrombus resolution without bleeding risk and thereby for the thrombus resolution in stroke, pulmonary embolism, thrombotic microangiopathy, thrombotic microangiopathy in hypercoagulable states after infection, , , disseminated intravascular coagulation, vaccine-induced immune thrombotic thrombocytopenia, vascular access site thrombosis or occlusion.

Preference is given in the context of the present invention to a compound selected from the group of 1-(1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(6-fluoro-1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 2,4-dioxo-3-[(1R)-4-(trifluoromethyl)-2,3-dihydro-1H-inden-1-yl]-1-(1,3,3-trimethyl-2-oxo-2,3-dihydro-1H-indol-5-yl)-1,2,3,4-tetrahydropyrimidine-5-carboxylic acid (R enantiomer), 1-(1'-methyl-2'-oxo-1',2'-dihydrospiro[cyclopropane-1,3'-indole]-5'-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzothiazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and ethyl 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylate (R enantiomer) for use in a method for the treatment and/or prophylaxis of stroke, or pulmonary embolism.

Preference is given in the context of the present invention to a compound selected from the group of 1-(1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(6-fluoro-1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 2,4-dioxo-3-[(1R)-4-(trifluoromethyl)-2,3-dihydro-1H-inden-1-yl]-1-(1,3,3-trimethyl-2-oxo-2,3-dihydro-1H-indol-5-yl)-1,2,3,4-tetrahydropyrimidine-5-carboxylic acid (R enantiomer), 1-(1'-methyl-2'-oxo-1',2'-dihydrospiro[cyclopropane-1,3'-indole]-5'-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzothiazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and ethyl 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylate (R enantiomer) and the salts, solvates and solvates of the salts thereof for use in a method for thrombus resolution without bleeding risk and thereby for the thrombus resolution in stroke, or pulmonary embolism.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for the treatment and/or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for the treatment and/or prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for thrombus resolution without bleeding risk and thereby for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for thrombus resolution without bleeding risk and thereby for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for the treatment and/or prophylaxis of stroke, or pulmonary embolism.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for thrombus resolution without bleeding risk and thereby for the thrombus resolution in stroke, or pulmonary embolism.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for the treatment and/or prophylaxis of stroke, or pulmonary embolism.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for thrombus resolution without bleeding risk and thereby for the thrombus resolution in stroke, or pulmonary embolism.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for the treatment and/or prophylaxis of stroke, or pulmonary embolism.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for thrombus resolution without bleeding risk and thereby for the thrombus resolution in stroke, or pulmonary embolism.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for the treatment and/or prophylaxis of stroke.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for thrombus resolution without bleeding risk and thereby for the thrombus resolution in stroke.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for the treatment and/or prophylaxis of pulmonary embolism.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for thrombus resolution without bleeding risk and thereby for the thrombus resolution in pulmonary embolism.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for the treatment and/or prophylaxis of thrombotic microangiopathy.

Preference is given in the context of the present invention to 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) for use in a method for thrombus resolution without bleeding risk and thereby for the thrombus resolution in thrombotic microangiopathy.

Preference is also given in the context of the present invention to compounds of the formula (I) in which
- R¹: is hydrogen,
and the salts, solvates and solvates of the salts thereof.

Preference is also given in the context of the present invention to compounds of the formula (I) in which
- R¹: is hydrogen,
- R²: is a group of the formula 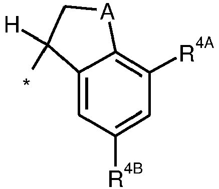
where
- *: is the attachment site to the uracil nitrogen atom,
- A: is -CH₂-,
- R^{4A}: is chlorine or trifluoromethyl,
- R^{4B}: is hydrogen,
and the salts, solvates and solvates of the salts thereof.

Preference is also given in the context of the present invention to compounds of the formula (I) in which
- R²: is a group of the formula 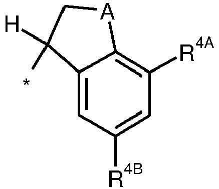
where
- *: is the attachment site to the uracil nitrogen atom,
- A: is -CH₂-,
- R^{4A}: is chlorine or trifluoromethyl,
- R^{4B}: is hydrogen,
and the salts, solvates and solvates of the salts thereof.

In a further non-claimed aspect, the present disclosure covers compounds of general formula (II), 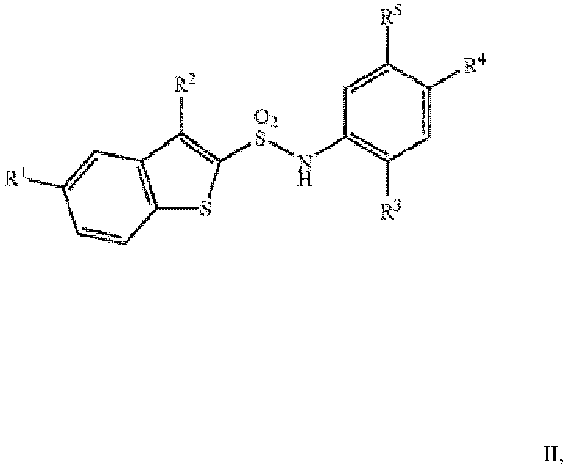 wherein
- R¹: represents a hydrogen atom, a halogen atom or a lower alkyl group;
- R²: represents a lower alkyl group;
- R³ and R⁴: each may be the same or different and represents a hydrogen atom, a lower alkoxycarbonyl group, a lower alkylsulfonyl group, a benzoyl group, an acyl group having 1 to 4 carbon atoms, a lower alkoxy group, a lower alkoxycarbonylmethylthioacetyl group, a nitro group, -CONHR⁶ in which R⁶ represents a hydrogen atom, a lower alkoxycarbonylmethyl group, acarboxymethyl group or -CH(CH₂OH)COOR⁷ in which R⁷ represents a hydrogen atom or a lower alkyl group,
a group represented by fornula 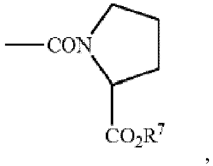
in which R⁷ has the same meaning as above, a group represented by formula 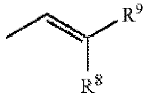
in which R⁸ and R⁹ each may be the same or different and represents a hydrogen atom, a lower alkyl group, a lower alkylsulfanyl group, a lower alkylsulfinyl group, a lower alkylsulfonyl group or a lower alkoxycarbonyl group, a hydroxy lower alkyl group, a cyano group
or a monocyclic heterocyclic group represented by formulae: 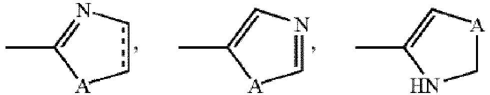 or 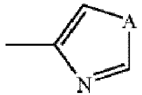
in which A represents an oxygen atom, a sulfur atom or NH and the dotted part represents a single bond or a double bond provided that the hydrogen atom on the ring may be replaced by a lower alkyl group which may be substituted by a halogen atom, a lower alkoxy group, a hydroxy lower alkyl group, a lower alkoxycarbonyl roup or a carboxyl group, provided that at least one of R³ or R⁴ is 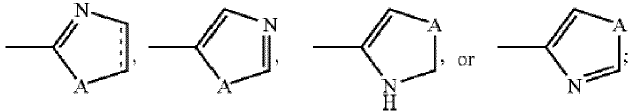 and
- R⁵: represents a hydrogen atom, a lower alkoxy group or a lower alkyl group, except the compounds represented by formulae: 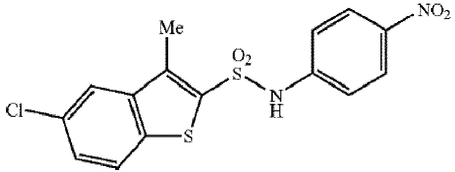 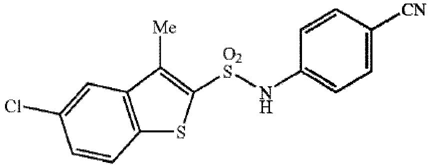 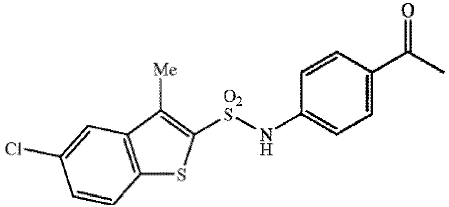 and 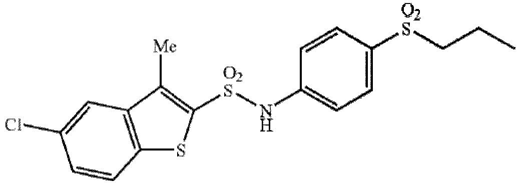
for use in a method for the treatment and/or prophylaxis of stroke, pulmonary embolism, deep or superficial vein thrombosis, thrombotic microangiopathy, thrombotic microangiopathy in hypercoagulable states after infection, inflammation, transplantation, disseminated intravascular coagulation, vaccine-induced immune thrombotic thrombocytopenia, vascular access site thrombosis or occlusion.

In a further non-claimed aspect, the present disclosure covers compounds of general formula (II), 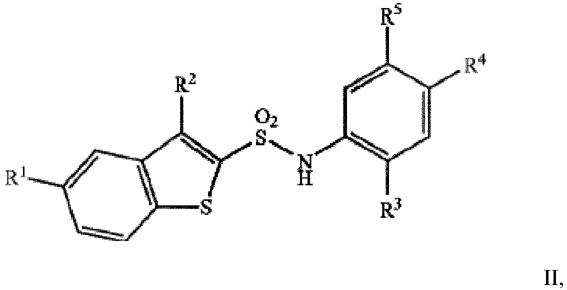 wherein
- R¹: represents a hydrogen atom, a halogen atom or a lower alkyl group;
- R²: represents a lower alkyl group;
- R³ and R⁴: each may be the same or different and represents a hydrogen atom, a lower alkoxycarbonyl group, a lower alkylsulfonyl group, a benzoyl group, an acyl group having 1 to 4 carbon atoms, a lower alkoxy group, a lower alkoxycarbonylmethylthioacetyl group, a nitro group, -CONHR⁶ in which R⁶ represents a hydrogen atom, a lower alkoxycarbonylmethyl group, acarboxymethyl group or -CH(CH₂OH)COOR⁷ in which R⁷ represents a hydrogen atom or a lower alkyl group,
a group represented by fornula 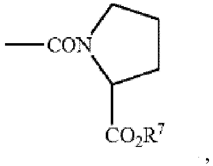
in which R⁷ has the same meaning as above, a group represented by formula 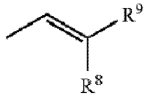
in which R⁸ and R⁹ each may be the same or different and represents a hydrogen atom, a lower alkyl group, a lower alkylsulfanyl group, a lower alkylsulfinyl group, a lower alkylsulfonyl group or a lower alkoxycarbonyl group, a hydroxy lower alkyl group, a cyano group
or a monocyclic heterocyclic group represented by formulae: 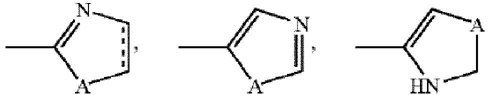 or 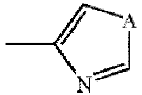
in which A represents an oxygen atom, a sulfur atom or NH and the dotted part represents a single bond or a double bond provided that the hydrogen atom on the ring may be replaced by a lower alkyl group which may be substituted by a halogen atom, a lower alkoxy group, a hydroxy lower alkyl group, a lower alkoxycarbonyl roup or a carboxyl group, provided that at least one of R³ or R⁴ is 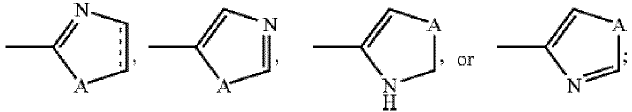 and
- R⁵: represents a hydrogen atom, a lower alkoxy group or a lower alkyl group, except the compounds represented by formulae: 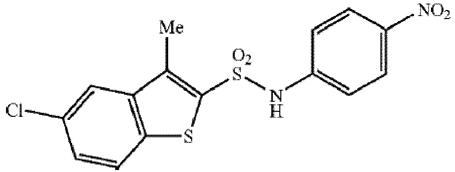 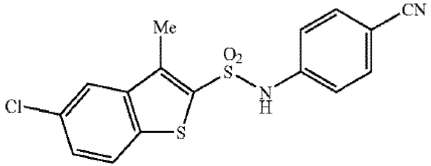 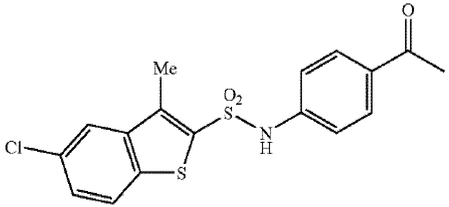 and 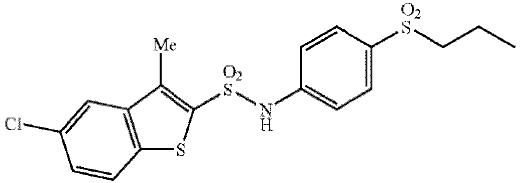 for use in a method for thrombus resolution in stroke, pulmonary embolism, deep or superficial vein thrombosis, thrombotic microangiopathy, thrombotic microangiopathy in hypercoagulable states after infection, inflammation, transplantation, disseminated intravascular coagulation, vaccine-induced immune thrombotic thrombocytopenia, vascular access site thrombosis or occlusion. Examples of the halogen atom for R¹ include a fluorine atom, a chlorine atom, a bromine atom or an iodine atom, and particularly, a fluorine atom or a chlorine atom is preferable.

Examples of the lower alkyl group for R¹, R², R⁵, R⁷, R⁸ and R⁹ include a methyl group, an ethyl group, a propyl group, an isopropyl group, a butyl group, an isobutyl group, a sec-butyl group or a tert-butyl group, and particularly, a methyl group or an ethyl group is preferable.

Examples of the lower alkoxycarbonyl group for R³, R⁴, R⁸ and R⁹ include a methoxycarbonyl group, an ethoxycar- bony! group, a propoxycarbonyl group, an isopropoxycar-bonyl group, a butoxycarbonyl group, an isobutoxycarbonyl group, a sec-butoxycarbonyl group or a tert-butoxycarbonyl group, and particularly, a methoxycarbonyl group, an ethoxycarbonyl group, an isopropoxycarbonyl group or a tert-butoxycarbonyl group is preferable.

Examples of the lower alkylsulfonyl group for R³, R⁴, R⁸ and R⁹ include a methanesulfonyl group, an ethanesulfonyl group, a propanesulfonyl group, an isopropanesulfonyl group, a butanesulfonyl group, an isobutanesulfonyl group, a sec-butanesulfonyl group or a tert-butanesulfonyl group, and particularly, a methanesulfonyl group or an ethanesulfonyl group is preferable.

Examples of the acyl group having 1 to 4 carbon atoms for R³ and R⁴ include a formyl group, an acetyl group, a propionyl group, a butyryl group or an isobutyryl group, and particularly, an acetyl group is preferable.

Examples of the lower alkoxy group for R³, R⁴ and R⁵ include a methoxy group, an ethoxy group, a propoxy group, an isopropoxy group, a butoxy group, an isobutoxy group, a sec-butoxy group or a tert-butoxy group, and particularly, a methoxy group or an ethoxy group is preferable.

Examples of the lower alkoxycarbonylmethylthioacetyl group for R³ and R⁴ include a methoxycarbonylmethylthio-acetyl group, an ethoxycarbonylmethylthioacetyl group, a propoxycarbonylmethylthioacetyl group, an isopropoxycarbonylmethylthioacetyl group, a butoxycarbonylmethylthio-acetyl group, an isobutoxycarbonylmethylthioacetyl group, a sec-butoxycarbonylmethylthioacetyl group or a tert-bu-toxycarbonylmethylthioacetyl group, and particularly, a methoxycarbonylmethylthioacetyl group or an ethoxycarbo- 35 nylmethylthioacetyl group is preferable.

In the case that R³ and R⁴ each is -CONHR⁶, examples of the lower alkoxycarbonylmethyl group for R⁶ include a methoxycarbony lmethy I group, an ethoxycarbony lmethy I group, a propoxycarbonylmethyl group, an isopropoxycar-bonylmethyl group, a butoxycarbonylmethyl group, an isobutoxycarbonylmethyl group, a sec-butoxycarbonylm-ethyl group or a tert-butoxycarbonylmethyl group, and par ticularly, a methoxycarbonylmethyl group or an ethoxycarbonylmethyl group, or an isopropoxycarbonylmethyl group
is preferable.

Examples of the hydroxy lower alkyl group for R³ and R⁴ include a linear or branched hydroxy lower alkyl group having 1 to 4 carbon atoms such as a hydroxymethyl group, a hydroxyethyl group, a hydroxypropyl group or a hydroxy-butyl group, and particularly a hydroxymethyl group or a hydroxyethyl group is preferable.

Examples of the lower alkylsulfanyl group for R⁸ and R⁹ include a linear or branched lower alkylsulfanyl group having 1 to 4 carbon atoms such as a methylsulfanyl group, an ethylsulfanyl group, a propylsulfanyl group or a butyl-sulfanyl group, and particularly, a methylsulfanyl group or an ethylsulfanyl group.

Examples of the lower alkylsulfinyl group for R⁸ and R⁹ include a linear or branched lower alkylsulfinyl group hav-ing 1 to 4 carbon atoms such as a methanesulfinyl group, an ethanesulfinyl group, a propanesulfinyl group or a butanesulfinyl group, and particularly, a methanesulfinyl goup or an ethanesulfinyl group is preferable.

As the group represented by the formula:

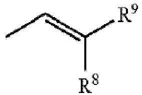

For example, a vinyl group, a methylsulfanylvinyl group, methanesulfinylvinyl group or 2-methanesulfinyl-2-methyl-sulfanylvinyl group is preferable.

The meanings of the lower alkoxy group, the hydroxy lower alkyl group and the lower alkoxycarbonyl group by 15 which the hydrogen atom on the ring represented by the formulae 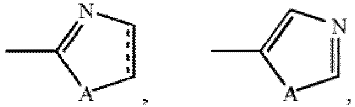 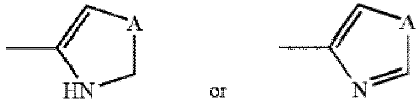 may be replaced are as mentioned in the above. Moreover, the lower alkyl group which may be substituted by a halogen atom means a lower alkyl group substituted by a fluorine atom, a chlorine atom, a bromine atom or an iodine atom in addition to the above-described lower alkyl group. Examples thereof include a chloromethyl group, a bromom-ethyl group, a dichloromethyl group or 1-chloroethyl group.

One or two of the lower alkyl groups which may be substituted by a halogen atom, lower alkoxy groups, hydroxy lower alkyl groups, lower alkoxycarbonyl groups or carboxyl groups may be present as substituent(s) on each heterocycle, and the substituents may be the same or different Examples of the monocyclic heterocylic group repre-sented by the formulae: 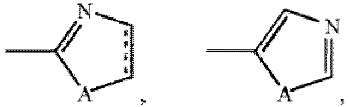 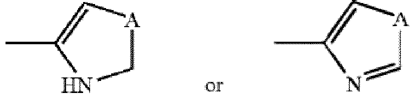 in which A represents an oxygen atom, a sulfur atom or NH and the dotted part represents a single bond or a double bond, include those represented by the following formulae.

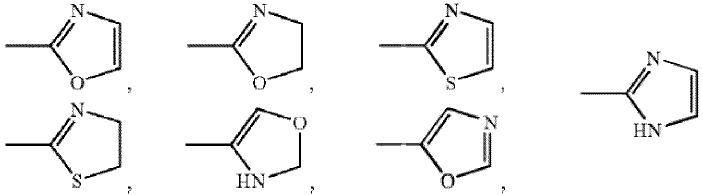

Specific examples of the monocyclic heterocylic group represented by the formulae: 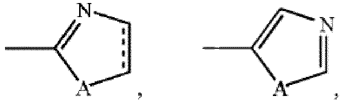 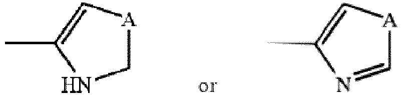 in which A represents an oxygen atom, a sulfur atom or NH and the dotted part represents a single bond or a double bond, as well as the hydrogen atom on the ring may be replaced by a lower alkyl group which may be substituted by a halogen atom, a lower alkoxy group, a hydroxy lower alkyl group, a lower alkoxycarbonyl group or a carboxyl group, preferably include those represented by the formulae: 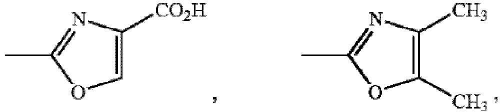 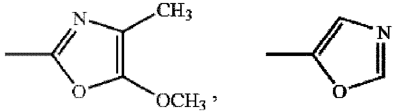 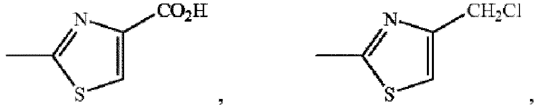 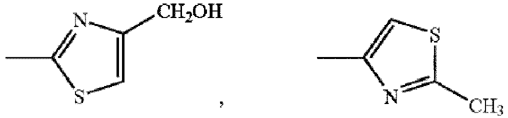 and these substituents are preferably substituted as R⁴, In this case, it is further preferable that R³ is a methanesulfonyl group and R⁵ is a hydrogen atom.

Preference is given in the context of the present non-claimed disclosure to a compound selected from the group of consisting of 2-]4-(5-chloro-3-methylbenzo[b]thiophene-2-sulfonylamino)-3-methanesulfonylphenyl ]ox-azole-4-carboxy lic acid, 2-[ 4-(5-fluoro-3-methylbenzo[b] thiophene-2-sulfonylamino )-3-methanesulfonylphenyl] oxazole-4-carboxylic acid, disodium 2-[4-(5-chloro-3-methylbenzo[b]thiophene-2-sulfonylamino)-3-methanesulfonylphenyl]oxazole-4-carboxylate, disodium 2-[ 4-(5-fluoro-3-methylbenzo[b ]thiophene-2-sulfonylamino)-3-methanesulfonylphenyl] oxazole-4-carboxylate, 2-]4-(5-fluoro-3-methylbenzo[b]thiophene-2-sulfonylamino)-3-methanesulfonylphenyl]thiazole-4-carboxylic acid, 5-fluoro-N-[ 4-( 4-hydroxymethylthiazol-2-yl)-2-methane-sulfonylphenyl]-3-methylbenzo[b]thiophene-2-sulfonamide,5-fluoro-N-[2-methane-sulfony1-4-(5-methoxy-4-methyloxazol-2-yl)phenyl]-3-methylbenzo[b]thiophene-2-sulfonamide, ((2-(4-((5-fluoro-3-methylbenzo-[b]thiophene)-2-sulfonamido)-3-(methylsulfonyl)-phenyl)thiazole-4-carboxylic acid and 5-fluoro-N-[2-methanesulfonyl-4-(5-methyloxazol-2-yl)phenyl]-3-methylbenzo[b]thiophene-2-sulfonamide for use in a method for the treatment and/or prophylaxis of stroke, pulmonary embolism, deep or superficial vein thrombosis, thrombotic microangiopathy, thrombotic microangiopathy in hypercoagulable states after infection, inflammation, transplantation, disseminated intravascular coagulation, vaccine-induced immune thrombotic thrombocytopenia, vascular access site thrombosis or occlusion.

Preference is given in the context of the present non-claimed disclosure to a compound selected from the group of consisting of 2-14-(5-chloro-3-methylbenzo[b]thiophene-2-sulfonylamino)-3-methanesulfonylphenyl ]ox-azole-4-carboxy lic acid, 2-[ 4-(5-fluoro-3-methylbenzo[b] thiophene-2-sulfonylamino )-3-methanesulfonylphenyl] oxazole-4-carboxylic acid, disodium 2-[4-(5-chloro-3-methylbenzo[b]thiophene-2-sulfonylamino)-3-methanesulfonylphenyl]oxazole-4-carboxylate, disodium 2-[ 4-(5-fluoro-3-methylbenzo[b ]thiophene-2-sulfonylamino)-3-methanesulfonylphenyl] oxazole-4-carboxylate, 2-]4-(5-fluoro-3-methylbenzo[b]thiophene-2-sulfonylamino)-3-methane-sulfonylphenyl]thiazole-4-carboxylic acid, 5-fluoro-N-[ 4-( 4-hydroxymethylthiazol-2-yl)-2-methane-sulfonylphenyl]-3-methylbenzo[b]thiophene-2-sulfonamide,5-fluoro-N-[2-methane-sulfonyl-4-(5-methoxy-4-methyloxazol-2-yl)phenyl]-3-methylbenzo[b]thiophene-2-sulfonamide, ((2-(4-((5-fluoro-3-methylbenzo-[b]thiophene)-2-sulfonamido)-3-(methylsulfonyl)-phenyl)thiazole-4-carboxylic acid and 5-fluoro-N-[2-methanesulfonyl-4-(5-methyloxazol-2-yl)phenyl]-3-methylbenzo[b]thiophene-2-sulfonamide for use in a method for thrombus resolution in stroke, pulmonary embolism, deep or superficial vein thrombosis, thrombotic microangiopathy, thrombotic microangiopathy in hypercoagulable states after infection, inflammation, transplantation, disseminated intravascular coagulation, vaccine-induced immune thrombotic thrombocytopenia, vascular access site thrombosis or occlusion.

Preference in the context of the non-claimed disclosure is given to 2-(4-((5-fluoro-3-methylbenzo-[b]thiophene)-2-sulfonamido)-3-(methylsulfonyl)phenyl)thiazole-4-carboxylic acid for use in a method for the treatment and/or prophylaxis of stroke, pulmonary embolism, deep or superficial vein thrombosis, thrombotic microangiopathy, thrombotic microangiopathy in hypercoagulable states after infection, inflammation, transplantation, disseminated intravascular coagulation, vaccine-induced immune thrombotic thrombocytopenia, vascular access site thrombosis or occlusion.

Preference in the context of the non-claimed disclosure is given to 2-(4-((5-fluoro-3-methylbenzo-[b]thiophene)-2-sulfonamido)-3-(methylsulfonyl)phenyl)thiazole-4-carboxylic acid for use in a method for thrombus resolution in stroke, pulmonary embolism, deep or superficial vein thrombosis, thrombotic microangiopathy, thrombotic microangiopathy in hypercoagulable states after infection, inflammation, transplantation, disseminated intravascular coagulation, vaccine-induced immune thrombotic thrombocytopenia, vascular access site thrombosis or occlusion.The compounds according to the invention are chymase inhibitors, especially compounds of the formula (I), and salts, solvates and solvates of the salts thereof, and have an unforeseeable useful pharmacological activity spectrum. These compounds influence the proteolytic activity of the serine protease chymase. The compounds according to the invention inhibit the enzymatic cleavage of chymase substrates.

The compounds of formula (I), their production and their action as selective inhibitors of chymase for the treatment and/or prevention of heart failure, pulmonary hypertension, chronic obstructive pulmonary disease, asthma, kidney failure, nephropathy, fibrotic disorders of the internal organs and dermatological fibroses are disclosed in WO 2013167495 A1.

The compounds of formula (II), their production and their action as inhibitors of chymase for the treatment of hypertension, hypercardia, cardiac failure, cardiac infarction, arteriosclerosis, diabetic or non-diabetic renal disease, diabetic retinopathy, ischemic re-perfusion disorder, restenosis after percutaneous transluminal coronary angioplasty, intimal thickening after bypass grafting, chronic rheumatism, keloid, psoriasis, allergy, inflammation, asthma, atopic dermatitis, solid tumors, and pulmonary hypertension are disclosed in US7,071,220 B2.

In the context of the present invention, the term "treatment" or "treating" includes inhibition, retardation, checking, alleviating, attenuating, restricting, reducing, suppressing, repelling or healing of a disease, a condition, a disorder, an injury or a health problem, or the development, the course or the progression of such states and/or the symptoms of such states. The term "therapy" is understood here to be synonymous with the term "treatment".

The terms "prevention", "prophylaxis" and "preclusion" are used synonymously in the context of the present invention and refer to the avoidance or reduction of the risk of contracting, experiencing, suffering from or having a disease, a condition, a disorder, an injury or a health problem, or a development or advancement of such states and/or the symptoms of such states.

The treatment or prevention of a disease, a condition, a disorder, an injury or a health problem may be partial or complete.

For the purpose of the present invention, "the resolution of thrombi during thrombotic or thromboembolic events" includes disorders and complications, which occur in the arterial, the venous vascular system, the microcirculation and the lymphatic system, which can be treated with the compounds according to the invention.

The compounds according to the invention are suitable for the treatment and prophylaxis of disorders of the venous vascular system leading to the occlusion of venous vessels and thereby resulting in venous thrombosis. These include thrombotic events in venous vessels in the lower and upper extremities, e.g. lower deep vein thrombosis in calf, popliteal, femoral, common femoral and iliac veins with or without the inferior vena cava, upper deep vein thrombosis in the jugular, brachiocephalic, subclavian, and axillary veins, as well as the more distal brachial, ulnar, and radial veins. or superficial vein thrombosis in the small and great saphenous veins and the cephalic and basilic veins, events in venous vessels in organs, including retinal vein occlusion, thrombosis of the cerebral veins and the venous sinuses of the skull, thrombosis of the renal vein, the Budd-Chiari Syndrome and portal vein thrombosis, mesenteric vein thrombosis, ovarian vein thrombosis and partial thrombosis of the corpus cavernosum.

The compounds according to the invention are suitable for the prevention of complications following venous thrombosis, including for example post-thrombotic syndrome, pulmonary hypertension or pulmonary embolism, and cryptogenic stroke linked to paradox thromboembolism with a patent foramen ovale, for deep vein thrombosis in the extremities.

The compounds according to the invention are suitable for the treatment and prophylaxis of disorders of the arterial vascular system leading to the occlusion of arterial vessels and thereby resulting in arterial thrombosis. This includes in particular disorders in the cerebrovascular arteries, such as transitory ischemic attacks (TIA), ischemic strokes including cardioembolic strokes, such as strokes due to atrial fibrillation, non-cardioembolic strokes, such as lacunar stroke, strokes due to large or small artery diseases, or strokes due to undetermined cause, cryptogenic strokes, embolic strokes, embolic strokes of undetermined source, or events of thrombotic and/or thromboembolic origin leading to stroke or TIA, disorders in the coronary arteries of the heart, such as acute coronary syndrome (ACS), myocardial infarction with ST segment elevation (STEMI) and without ST segment elevation (non-STEMI), stable angina pectoris, unstable angina pectoris, reocclusions and restenosis after coronary interventions such as angioplasty, stent implantation or aortocoronary bypass, and disorders of peripheral arteries, leading to peripheral artery disease, including peripheral artery occlusion, acute limb ischemia, amputation, reocclusions and restenoses after interventions such as angioplasty, stent implantation or surgery and bypass, and/or stent thrombosis, renal, hepatic or mesenteric artery thrombosis, retinal artery thrombosis.

The compounds according to the invention are suitable for the treatment and prophylaxis of local or systemic disorders in the microcirculation caused by small vessel thrombosis, including primary thrombotic microangiopathies (TMAs) like thrombotic thrombocytopenic purpura (TTP) or hemolytic uremic syndrome (HUS), and secondary thrombotic microangiopathies. These comprise microthrombosis as a result of hypercoagulable states in the cause of infectious diseases caused by bacteria, viruses or fungi, for example hypercoagulable states in the cause of viral infection with for example corona viruses like SARS-Cov1, MERS, and SARS-Cov2 infections, sepsis, microthrombosis caused by drugs or vaccinations (e.g. vaccination-induced thrombotic thrombocytopenia (VITT), which have occurred as very rare adverse event after COVID vaccination), disseminated intravascular coagulation (DIC), pulmonary thrombosis with and without ARDS, autoimmune diseases (e.g. systemic lupus erythematosus), antiphospholipid syndrome, malignant hypertension, transplantation (e.g. solid organ, bone marrow), pregnancy-associated thrombotic microangiopathy (e.g. preeclampia, HELLP syndrome), microthrombosis of the placenta, venous occlusive diseases of the liver, diabetic microangiopathies (e.g. diabetic retinopathy, glomerulopathy, gangrene), small vessel vasculitis of the capillary system (e.g. cerebral vasculitis).

The compounds according to the invention are suitable for the treatment and prophylaxis of unmet clinical needs in the resolving of venous thromboembolism (VT atherothrombotic occlusion of arterial vessels, in related diseases including acute ischemic stroke, peripheral artery occlusion disease and myocardial infarction, treatment and/or prophylaxis of disorders in the cerebrovascular arteries, such as transitory ischaemic attacks (TIA), ischemic strokes including cardioembolic strokes, such as strokes due to atrial fibrillation, non-cardioembolic strokes, such as lacunar stroke, strokes due to large or small artery diseases, or strokes due to undetermined cause, cryptogenic strokes, embolic strokes, embolic strokes of undetermined source, or events of thrombotic and/or thromboembolic origin leading to stroke or TIA, and/or disorders of peripheral arteries, leading to peripheral artery disease, including peripheral artery occlusion, acute limb ischemia, amputation, reocclusions and restenoses after interventions such as angioplasty, stent implantation or surgery and bypass, and/or stent thrombosis
The compounds according to the invention are suitable for the treatment and prophylaxis of ischemic events caused by occlusion of arteries by thrombotic emboli, in related diseases including pulmonary embolism, cardiogenic stroke and myocardial infarction
The compounds according to the invention are suitable for the treatment and prophylaxis of venous thrombosis, including thrombosis in deep veins and superficial veins of extremities, cerebral veins,
The compounds according to the invention are suitable for the treatment and prophylaxis of thrombotic microangiopathies diseases or diseases with thrombotic complications in the microcirculation, including infectious diseases caused by bacteria, viruses or fungi, sepsis, isolated pulmonary embolism/thrombosis, autoimmune diseases
The compounds according to the invention are suitable for the treatment and prophylaxis of thrombosis in or occlusion of vascular access sites, including arteriovenous fistula, central venous ports
The compounds according to the invention are suitable for the treatment and prophylaxis of extravascular fibrin deposits, e.g. in lung, placenta, brain.

This includes unmet clinical needs in the resolving of venous thromboembolism (VTE) in cancer patients undergoing hormonotherapy, chemotherapy or radiotherapy. These patients, often prone to splanchnic localized- or catheter-induced thrombosis, are anti-coagulated and thus subjected to the bleeding disorders associated with that latter class of medications (Giustozi et al, Trends Cardiovasc Med, 2022; In press). In addition, mast cell activation is increasingly reported to play a role in cancer development (Noto et al., Front Cell Dev Biol. 2021 Oct 12;9:752350; Segura-Villalobos et al., Cells. 2022 Jan 20;11(3):349).

This includes disorders caused by the exposure of biomaterial or artificial surfaces to the blood and/or vascular wall, including procedures for re-opening of vascular access sites, including arteriovenous fistula, central venous ports for central line therapies or dialysis procedures, procedures for revascularization or thrombus degradation in stents, grafts, cardiovascular assist devices, artificial hearts, mechanic or bioprosthetic heart valves, or during transplantations.

This includes disorders related to extravascular fibrin deposits, including fibrin deposits in the interstitial space in the lungs.

This includes use of chymase inhibition in diagnostic approaches for disease and/or therapy control.

Note that only the treatment of the claimed specific disorders is part of the present invention.

The inventive compounds may, depending on their structure, exist in different stereoisomeric forms, i.e. in the form of configurational isomers or else, if appropriate, of conformational isomers (enantiomers and/or diastereomers, including those in the case of rotamers and atropisomers). The present invention therefore encompasses the enantiomers and diastereomers, and the respective mixtures thereof. The stereoisomerically uniform constituents can be isolated from such mixtures of enantiomers and/or diastereomers in a known manner; chromatography processes are preferably used for this, especially HPLC chromatography on an achiral or chiral phase.

Preferred salts in the context of the present invention are physiologically acceptable salts of the compounds according to the invention. However, the invention also encompasses salts which themselves are unsuitable for pharmaceutical applications but which can be used, for example, for the isolation or purification of the compounds according to the invention.

Physiologically acceptable salts of the compounds according to the invention include acid addition salts of mineral acids, carboxylic acids and sulfonic acids, for example salts of hydrochloric acid, hydrobromic acid, sulfuric acid, phosphoric acid, methanesulfonic acid, ethanesulfonic acid, toluenesulfonic acid, benzenesulfonic acid, naphthalenedisulfonic acid, acetic acid, trifluoroacetic acid, propionic acid, lactic acid, tartaric acid, malic acid, citric acid, fumaric acid, maleic acid and benzoic acid.

Physiologically acceptable salts of the compounds according to the invention also include salts of conventional bases, by way of example and with preference alkali metal salts (e.g. sodium and potassium salts), alkaline earth metal salts (e.g. calcium and magnesium salts) and ammonium salts derived from ammonia or organic amines having 1 to 16 carbon atoms, by way of example and with preference ethylamine, diethylamine, triethylamine, ethyldiisopropylamine, monoethanolamine, diethanolamine, triethanolamine, dicyclohexylamine, dimethylaminoethanol, procaine, dibenzylamine, *N-*methylmorpholine, arginine, lysine, ethylenediamine, *N*-methylpiperidine and choline.

Solvates in the context of the invention are described as those forms of the inventive compounds which form a complex in the solid or liquid state by coordination with solvent molecules. Hydrates are a specific form of the solvates in which the coordination is with water.

The treatment or prevention of a disease, a condition, a disorder, an injury or a health problem may be partial or complete.

Chymase inhibitors like the inhibitors covered in the current invention, in particular 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (R enantiomer) fulacimstat and TY-51469 are therefore suitable for use as medicaments for the treatment and/or prophylaxis of diseases in humans and animals.

Chymase inhibitors like the inhibitors covered in the current invention, in particular 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (R enantiomer) fulacimstat are therefore suitable for use as medicaments for the treatment and/or prophylaxis of diseases in humans and animals.

The present invention further provides for the use of the compounds according to the invention for the treatment and/or prophylaxis of disorders, in particular vascular disorders, preferably thrombotic or thromboembolic disorders and/or thrombotic or thromboembolic complications.

Accordingly, the compounds according to the invention are suitable for the treatment and/or prophylaxis of disorders or complications which may arise from the formation of clots.

The present invention further provides for the use of chymase inhibitors of the present invention for the treatment and prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention further provides for the use of chymase inhibitors of the present invention for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention further provides for the use of chymase inhibitors of the present invention for the treatment and prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention further provides for the use of chymase inhibitors of the present invention for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention further provides for the use of chymase inhibitors of the present invention for the treatment and prophylaxis of stroke, pulmonary embolism.

The present invention further provides for the use of chymase inhibitors of the present invention for the thrombus resolution in stroke, pulmonary embolism.

The present invention further provides for the use of chymase inhibitors of general formula I for the treatment and prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention further provides for the use of chymase inhibitors of general formula I for the treatment and prophylaxis of stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention further provides for the use of chymase inhibitors of general formula I for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention further provides for the use of chymase inhibitors of general formula I for the thrombus resolution in stroke, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion.

The present invention further provides for the use of chymase inhibitors of general formula I for the treatment and prophylaxis of stroke, or pulmonary embolism.

The present invention further provides for the use of chymase inhibitors of general formula I for the thrombus resolution in stroke, or pulmonary embolism.

The present invention further provides for the use of chymase inhibitors of general formula I for the treatment and prophylaxis of stroke, or pulmonary embolism.

The present invention further provides for the use of chymase inhibitors of general formula I for the thrombus resolution in stroke, or pulmonary embolism.

The present non-claimed disclosure further provides for the use of chymase inhibitors of general formula II for the treatment and prophylaxis of stroke, pulmonary embolism, deep or superficial vein thrombosis, thrombotic microangiopathy, thrombotic microangiopathy in hypercoagulable states after infection, inflammation, transplantation, disseminated intravascular coagulation, vaccine-induced immune thrombotic thrombocytopenia, vascular access site thrombosis or occlusion.

The present non-claimed disclosure further provides for the use of chymase inhibitors of general formula II for the thrombus resolution in stroke, pulmonary embolism, deep or superficial vein thrombosis, thrombotic microangiopathy, thrombotic microangiopathy in hypercoagulable states after infection, inflammation, transplantation, disseminated intravascular coagulation, vaccine-induced immune thrombotic thrombocytopenia, vascular access site thrombosis or occlusion.

The present non-claimed disclosure further provides for the use of chymase inhibitors of general formula II for the thrombus resolution in stroke, pulmonary embolism, deep or superficial vein thrombosis, thrombotic microangiopathy, thrombotic microangiopathy in hypercoagulable states after infection, inflammation, transplantation, disseminated intravascular coagulation, vaccine-induced immune thrombotic thrombocytopenia, vascular access site thrombosis or occlusion.

The present non-claimed disclosure further provides for the use of chymase inhibitors of general formula II for the treatment and prophylaxis of stroke, pulmonary embolism, phlebitis or microangiopathy.

The present non-claimed disclosure further provides for the use of chymase inhibitors of general formula II for the thrombus resolution in stroke, pulmonary embolism, phlebitis or microangiopathy.

For the purpose of the present invention, the "thrombotic or thromboembolic disorders and/or thrombotic or thromboembolic complications" include disorders and complications, which occur in the arterial, the venous vascular system and the lymphatic system, which can be treated with the compounds according to the invention.

The present invention further provides the compounds according to the invention for use in a method for the treatment and/or prophylaxis of disorders, especially the disorders mentioned above, using a therapeutically effective amount of a compound according to the invention.

The present invention further provides the compounds according to the invention for use in a method for the thrombus resolution in disorders, especially in the disorders mentioned above, using a therapeutically effective amount of a compound according to the invention.

The present invention further provides medicaments comprising a compound according to the invention and one or more further active compounds.

The present invention further provides medicaments comprising a compound according to the invention and one or more further active compounds, in particular for the thrombus resolution in disorders mentioned above.

The present invention further provides medicaments comprising a compound according to the invention and one or more further active compounds, in particular for the treatment and/or prophylaxis of the disorders mentioned above.

Preferred examples of active compounds suitable for combinations include:
- plasminogen activators (thrombolytics/fibrinolytics), such as but not limited to tissue plasminogen activator (t-PA, for example Actilyse^{®}), streptokinase, tenecteplase, reteplase and urokinase, compounds which promote fibrinolysis such as inhibitors of the plasminogen activator inhibitor (PAI inhibitors), inhibitors of the thrombin-activated fibrinolysis inhibitor (TAFI inhibitors) or inhibitors of α2-antiplasmin, or plasminogen-modulating substances causing increased formation of plasmin, such as SMTP-7;
- lipid-lowering substances, especially HMG-CoA (3-hydroxy-3-methylglutaryl-coenzyme A) reductase inhibitors, for example lovastatin (Mevacor), simvastatin (Zocor), pravastatin (Pravachol), fluvastatin (Lescol) and atorvastatin (Lipitor); PCSK9 inhibitors (e.g., evolocumab, alirocumab)
- coronary therapeutics/vasodilators, especially ACE (angiotensin converting enzyme) inhibitors, for example captopril, lisinopril, enalapril, ramipril, cilazapril, benazepril, fosinopril, quinapril and perindopril, or AII (angiotensin II) receptor antagonists, for example embusartan, losartan, valsartan, irbesartan, candesartan, eprosartan and telmisartan, or β-adrenoceptor antagonists, for example carvedilol, alprenolol, bisoprolol, acebutolol, atenolol, betaxolol, carteolol, metoprolol, nadolol, penbutolol, pindolol, propanolol and timolol, or alpha-1-adrenoceptor antagonists, for example prazosine, bunazosine, doxazosine and terazosine, or diuretics, for example hydrochlorothiazide, furosemide, bumetanide, piretanide, torasemide, amiloride and dihydralazine, or calcium channel blockers, for example verapamil and diltiazem, or dihydropyridine derivatives, for example nifedipin (Adalat) and nitrendipine (Bayotensin), or nitro preparations, for example isosorbide 5-mononitrate, isosorbide dinitrate and glycerol trinitrate, or substances causing an increase in cyclic guanosine monophosphate (cGMP), for example stimulators of soluble guanylate cyclase, for example riociguat;
- anticoagulants such as:
   - indirect anticoagulants, such as proteoglycans, for example heparin (UFH), low-molecular-weight heparins (LMW), for example tinzaparin, certoparin, parnaparin, nadroparin, ardeparin, enoxaparin, reviparin, dalteparin, danaparoid, semuloparin (AVE 5026), adomiparin (M118);
   - direct thrombin inhibitors (DTI), such as dabigatran, atecegatran, argatroban, bivalirudin, tanogitran and hirudin
   - direct factor Xa inhibitors, such as rivaroxaban, apixaban, edoxaban, betrixaban, darexaban, otamixaban and fondaparinux
   - direct inhibitors of more than one coagulation factor, such as dual FXa/IIa inhibitors, such as SATI (Lopez M. et al., Thromb Res. 193(2020)15-21)
   - inhibitors of FXI or FXIa, FXII or FXIIa or of the synthesis of FXI or FXII, such as asundexian, milvexian, osocimab, abelacicumab, fesomersen
- antiplatelet drug such as:
   - substances which inhibit the aggregation of platelets (platelet aggregation inhibitors, thrombocyte aggregation inhibitors), such as acetylsalicylic acid, P2Y12 antagonists such as, for example, ticlopidine (Ticlid), clopidogrel (Plavix), prasugrel, ticagrelor, cangrelor, elinogrel, PAR-1 antagonists such as, for example, vorapaxar, PAR-4 antagonists, EP3 antagonists such as, for example, DG041;
   - platelet adhesion inhibitors such as GPVI and/or GPIb antagonists such as, for example, revacept, glenzocimab or caplacizumab;
   - fibrinogen receptor antagonists (glycoprotein-IIb/IIIa antagonists), for example abciximab, eptifibatide, tirofiban, lamifiban, lefradafiban, fradafiban, zalunfiban;
- recombinant human activated protein C such as, for example, Xigris, recombinant thrombomodulin, such as ART-123, or recombinant antithrombin-III;
- neuroprotective approaches, such as NMDA receptor antagonists, PSD-95 inhibitors, tissue kallikrein preparations, endothelin-B agonists, PARP inhibitors,
-
- anti-inflammatory compounds, such as glucocorticoids (e.g., prednison, prednisolon, methylprednisolon, triamcinolon, dexamethason, beclometason, betametason, flunisolid, budesonid or fluticasone), non-steroidal anti-inflammatory agents (NSAIDs) (e.g., acetyl salicylic acid (aspirin), ibuprofen or naproxen), 5-amino salicylic acid-derivates, leukotriene receptor antagonists (e.g., montelukast), TNF-alpha-inhibitors (e.g., etanercept, infliximab, adalimumab, golimumab, certolizumab) and/or chemokin-receptor antagonists (e.g., CCR1, 2 and/or 5 antagonists), IRAK4 inhibitors, cannabinoid receptor agonists, interleukin-1β antibodies (e.g., canakinumab)
- anti-infective approaches, for example and preferably from the group of antibacterial (e.g. penicillins, vancomycin, ciprofloxacin, mupirocin, azithromycin and metronidazole), antimycotic (e.g. naftifin, nystatin) and/or antiviral and/or antiparasitic substances • additional intensive care therapy, such as fluid therapy (e.g., crystalloid or colloidal fluids); vasopressors (e.g., norepinephrine, dopamine or vasopressin); inotropic therapy (e.g., dobutamine); blood products (e.g., erythrocyte concentrates, platelet concentrates, erythropoietin or fresh frozen plasma); assisted ventilation in sepsis-induced acute lung injury (ALI) or acute respiratory distress syndrome (ARDS; e.g., example permissive hypercapnia, low tidal volumes); sedation (e.g., diazepam, lorazepam, midazolam or propofol); glucose control, for example insulin, glucose; renal replacement therapies (e.g., continuous veno-venous haemofiltration or intermittent haemodialysis)
- mast cell stabilizing compounds (nedocromil, cromolyn sodium), tryptase inhibitors, cathepsin G inhibitors or elastase inhibitors
- antihistaminic for example cetiricine, loratadine, astemizole

"Combinations" for the purpose of the invention mean not only dosage forms which contain all the components (so-called fixed combinations) and combination packs which contain the components separate from one another, but also components which are administered simultaneously or sequentially, provided that they are used for prophylaxis and/or treatment of the same disease. It is likewise possible to combine two or more active ingredients with one another, meaning that they are thus each in two-component or multicomponent combinations.

The inventive compounds can act systemically and/or locally. For this purpose, they can be administered in a suitable manner, for example by the oral, parenteral, pulmonal, nasal, sublingual, lingual, buccal, rectal, dermal, transdermal, conjunctival or otic route, or as an implant or stent.

The inventive compounds can be administered in administration forms suitable for these administration routes.

Suitable administration forms for oral administration are those which function according to the prior art and deliver the inventive compounds rapidly and/or in modified fashion, and which contain the inventive compounds in crystalline and/or amorphized and/or dissolved form, for example tablets (uncoated or coated tablets, for example having enteric coatings or coatings which are insoluble or dissolve with a delay, which control the release of the compound according to the invention), tablets which disintegrate rapidly in the mouth, or films/wafers, films/lyophilizates, capsules (for example hard or soft gelatin capsules), sugar-coated tablets, granules, pellets, powders, emulsions, suspensions, aerosols or solutions.

Parenteral administration can be accomplished with avoidance of a resorption step (for example by an intravenous, intraarterial, intracardiac, intraspinal or intralumbar route) or with inclusion of a resorption (for example by an intramuscular, subcutaneous, intracutaneous, percutaneous or intraperitoneal route). Administration forms suitable for parenteral administration include preparations for injection and infusion in the form of solutions, suspensions, emulsions, lyophilizates or sterile powders. For local parenteral administration catheter-based approaches for intraclot-administration can be used.

Preference is given to oral administration.

Suitable administration forms for the other administration routes are, for example, pharmaceutical forms for inhalation (including powder inhalers, nebulizers), nasal drops, solutions or sprays; tablets for lingual, sublingual or buccal administration, films/wafers or capsules, suppositories, preparations for the ears or eyes, vaginal capsules, aqueous suspensions (lotions, shaking mixtures), lipophilic suspensions, ointments, creams, transdermal therapeutic systems (for example patches), milk, pastes, foams, dusting powders, implants or stents.

The inventive compounds can be converted to the administration forms mentioned. This can be accomplished in a manner known per se by mixing with inert, nontoxic, pharmaceutically suitable excipients. These excipients include carriers (for example microcrystalline cellulose, lactose, mannitol), solvents (e.g. liquid polyethylene glycols), emulsifiers and dispersing or wetting agents (for example sodium dodecylsulfate, polyoxysorbitan oleate), binders (for example polyvinylpyrrolidone), synthetic and natural polymers (for example albumin), stabilizers (e.g. antioxidants, for example ascorbic acid), colourants (e.g. inorganic pigments, for example iron oxides) and flavour and/or odour correctants.

The present invention further provides medicaments comprising at least one inventive compound, preferably together with one or more inert nontoxic pharmaceutically suitable excipients, and the use thereof for the purposes mentioned above.

In general, it has been found to be advantageous in the case of parenteral administration to administer amounts of about 0.001 to 1 mg/kg, preferably about 0.01 to 0.5 mg/kg, of body weight to achieve effective results. In the case of oral administration, the dosage is about 0.01 to 100 mg/kg body weight. Preferably the oral daily dosage is about 1 mg to 100 mg and most preferably 2 mg to 50 mg. In spite of this, it may be necessary, if appropriate, to deviate from the amounts specified, specifically depending on body weight, administration route, individual behavior towards the active ingredient, type of formulation, and time or interval of administration.

Unless stated otherwise, the percentages in the tests and examples which follow are percentages by weight; parts are parts by weight. Solvent ratios, dilution ratios and concentration data for the liquid/liquid solutions are based in each case on volume. "w/v" means "weight/volume". For example, "10% w/v" means: 100 ml of solution or suspension comprise 10 g of substance.

### Experimental Methods

### 1. Abbreviations

AA - abdominal aorta
ACN - acetonitrile
ACS - acute coronary syndrome
AMC - aminomethylcoumarine
ANOVA - analysis of variance
ARDS - acute respiratory distress syndrome
BSA - bovine serum albumin
C57BL/6 - mouse strain C57 black 6
cGMP - cyclic guanosine monophosphate
CMA1 - chymase 1
CNS - central nervous system
Ctr - control
DAB -3,3'-diaminobenzidine
DIC - disseminated intravascular coagulation
DMSO - dimethylsulfoxide
DTI - direct thrombin inhibitors
DTT - dithiothreitol
DVT - deep vein thrombosis
EDTA - ethylenediaminetetraacetic acid
EtOH - ethanol
FA - formic acid
FDR - false discovery rate
HEPES - (4-(2-hydroxyethyl)-1-piperazineethanesulfonic acid)
HPLC - high performance liquid chromatography
i.m. - intramuscular
i.v. - intravenous
IVC - inferior vena cava
KO - knock-out
LC - liquid chromatography
LFQ - label-free-quantification
LMW - low-molecular-weight heparins
MCP-4 - mast cell protease 4
MES - 2-(N-morpholino)ethanesulfonic acid
MS - mass spectrometry
NEM - N-ethylmaleimide
non-STEMI - myocardial infarction without ST segment elevation
PAI - plasminogen activator inhibitor
PBS - Phosphate-buffered saline
PEG - polyethylene glycol
PSM - peptide-spectra-matches
PVP - polyvinylpyrrolidone
rCMA-1 - recombinant human chymase 1
rmMCP-4 - recombinant mouse mast cell protease 4
rtPA - recombinant human tissue plasminogen activator
S.E.M. - standard error of mean
SLPI - secretory leukocyte protease inhibitor
STEMI - myocardial infarction with ST segment elevation
TAFI - thrombin-activated fibrinolysis inhibitor
TCA - trichloroacetic acid
TFA - trifluoroacetic acid
TIA - transitory ischemic attack
tPA - tissue plasminogen activator
UFH - unfractionated heparin
uPA - urokinase

### 2. In vitro potency and selectivity assessment of the chymase inhibitors

The suitability of the compounds according to the invention for treating thromboembolic disorders by inhibition of chymase can be demonstrated in the following assay systems:

### 2.1 Enzymatic chymase activity assay

Recombinant human chymase protein and recombinant hamster chymase (Protein Technologies, Bayer Healthcare) were used. Cleavage of substrate Abz-HPFHL-Lys(Dnp)-NH2 (10 µM) in assay buffer Tris 50 mM (pH 7.5), NaCl 150 mM, BSA 0,10%, Chaps 0,10%, glutathione 1 mM, EDTA 1 mM with either 3 nM of recombinant human chymase or 1.2 nM of recombinant hamster chymase was monitored in a fluorescent reader (excitation 340 nm, emission 465 nm). The data were normalised (enzyme reaction without inhibitor = 0 % inhibition, all other assay components but no enzyme = 100 % inhibition). Normally test compounds were tested on the same microtiter plate at 9 different concentrations in the range of 10 µM to 1 nM (10 µM, 3.1 µM, 1.0 µM, 0.3 µM, 0.1 µM, 0.03 µM, 0.01 µM, 0.003 µM, 0.001 µM). IC₅₀ values were calculated using an in-house software.

### Results

The compounds in the context of the invention inhibited chymase activity potently with IC₅₀ values depicted in Table 1.

**Table 1 Potency of the chymase inhibitors (TY-51469 is not a compound according to the present invention)**

| **Species** | **Example** | **IC₅₀ [nM]** | | **Example** | **IC₅₀ [nM]** |
|---|---|---|---|---|---|
| Human | fulacimstat | 4 | | TY-51469 | 20 |
| Hamster | fulacimstat | 3 | | TY-51469 | 66 |

### 2.2 Determination of selectivity

Selectivity of the substances was demonstrated with respect to cathepsin G inhibition and other proteases (e.g. of the coagulation and fibrinolysis field).

Recombinant human cathepsin G (CathG) protein (Protein Technologies, Bayer AG) was used. Cleavage of substrate DABCYL-Ser-Thr_Leu-Ser-Glu-Lys_Ala_Lys_Pro_Ala-Glu (EDANS) (3 µM) in assay buffer Hepes 100 mM (pH 7.0), NaCl 50 mM, BSA 0,01%, Tween 20 0,001%, glutathione 0.3 mM with recombinant CathG (2.5 nM) was monitored in a fluorescent reader (excitation 334 nm, emission 536 nm). The data were normalized (enzyme reaction without inhibitor = 0 % inhibition, all other assay components but no enzyme = 100 % inhibition). Normally test compounds were tested on the same microtiter plate at 9 different concentrations in the range of 10 µM to 1 nM (10 µM, 3.1 µM, 1.0 µM, 0.3 µM, 0.1 µM, 0.03 µM, 0.01 µM, 0.003 µM, 0.001 µM). IC50 values were calculated using an in-house software.

The potency of test compounds against several other human serine proteases, including coagulation proteases, in buffer was analyzed in biochemical assays based on the fluorimetric detection of aminomethylcoumarine (AMC) cleaved from specific synthetic peptidic substrates by the respective proteases.

Serine protease assays were comprised of the following enzymes and substrates (final assay concentrations are given). The active proteases, typically purified from human plasma, and corresponding substrates are commercially available.
- Thrombin (Kordia; 0.02 nM), Boc-Asp(OBz1)-Pro-Arg-AMC (Bachem I-1560; 5 µM).
- Factor Xa (Kordia; 1.3 nM), Boc-Ile-Glu-Gly-Arg-AMC (Bachem I-1100; 5 µM).
- Factor XIa (Kordia; 0.15 nM), Boc-Glu(OBz1)-Ala-Arg-AMC (Bachem I-1575; 5 µM)
- Plasmin (Kordia; 0.1 µg/ml, 1.2 nM), MeOSuc-Ala-Phe-Lys-AMC (Bachem I-1275; 50 µM).
- Tissue plasminogen activator (tPA; Loxo; 1 nM), CH3SO2-D-Phe-Gly-Arg-AMC (Pentapharm 091-06; 5 µM).
- Plasma kallikrein (Kordia; 0.2 nM), H-Pro-Phe-Arg-AMC (Bachem I-1295; 5 µM).
- Trypsin (Sigma; 0.042 U/ml), Boc-Ile-Glu-Gly-Arg-AMC (Bachem I-1100; 5 µM).

Test compounds were diluted in dimethylsulfoxide (DMSO) to final assay concentrations of 0-50 µM. The respective human serine protease and the respective substrate were diluted in buffer solutions (pH 7.4) consisting of 50 mM Tris/HCl, 100 mM NaCl, 5 mM CaCl₂, and 0.1% bovine serum albumin to the final concentrations given below. Diluted test compound or DMSO (1 µL) in assay buffer (20 µL), diluted serine protease solution (20 µL), and substrate solution (20 µL) were added to 384-well microtiter plates (Greiner Bio-One, Frickenhausen, Germany). Fluorescence (excitation 360 nm, emission 460 nm) was measured using a microtiter plate fluorescence reader (Safire II, Tecan, Maennedorf, Switzerland) for 30 minutes at room temperature. The concentration of test compound producing 50% inhibition of serine protease activity (IC₅₀) was determined via a nonlinear logistic regression model.

### Results

Compounds in the context of the invention were selected versus several other enzymes. The IC₅₀ values are depicted in Table 2. Importantly, none of the tested proteases of the coagulation or fibrinolytic system were inhibited by 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) or TY-51469.

**Table 2 Selectivity of the chymase inhibitors**

| **Enzyme** | **Example** | **IC₅₀ [µM]** | | **Example** | **IC₅₀ [µM]** |
|---|---|---|---|---|---|
| Cathepsin G | fulacimstat | 0.14 | | TY-51469 | 1.3 |
| Plasmin | fulacimstat | >15 | | TY-51469 | >2 |
| Plasma kallikrein | fulacimstat | >15 | | TY-51469 | >2 |
| Thrombin | fulacimstat | >15 | | TY-51469 | >2 |
| Trypsin | fulacimstat | >15 | | TY-51469 | >2 |
| Tissue plasminogen activator | fulacimstat | >15 | | TY-51469 | >2 |
| Factor Xa | fulacimstat | >15 | | TY-51469 | >2 |
| Factor Xia | fulacimstat | >15 | | TY-51469 | >2 |

### 3. Plasmin is a substrate of chymase in in vitro assays and its degradation by chymase can be inhibited by chymase inhibitors

In order to assess the impact of chymase on plasmin, plasmin activities in the presence of chymase was determined over time in a mouse and a human *in vitro* system. In addition, the occurrences of potential plasmin degradation products of murine and human plasmin in the presence of chymase in buffer systems were investigated.

### 3.1. Plasmin activity in the presence of murine and human chymase and the impact of chymase inhibitors

Recombinant mouse mMCP-4 (rmMCP-4) or recombinant human (rCMA-1) were produced as pro-forms and activated with recombinant mouse cathepsin C (R&D Systems, Minneapolis, MN, USA) to become enzymatically active as previously described (Semaan W et al., Biochem.Pharmacol. 94(2015):91-100). rmMCP-4 or rCMA-1 were thawed and diluted to a concentration of 20 µg/mL in maturation buffer (50 mM MES, 0.1 % (w/v) BSA, pH 5.5). Active murine cathepsin C was diluted to 20 µg/mL in cathepsin C buffer (50 mM MES, 50 mM NaCl, 5 mM DTT, pH 5.5). Activation was performed by adding equal volumes of recombinant chymase and cathepsin C, adding 50 µg/mL heparin and incubating 1 h at room temperature. Chymase activation was stopped with 3 mM N-ethylmaleimide (NEM), followed by a dilution with assay buffer (20 mM Tris, 2 M KCl, 0.02 % (v/v) Triton X-100, pH 9.0) to bring the recombinant chymase concentration to 2 µg/mL; 5 min was sufficient to completely stop the cathepsin C-dependent reaction.

37 nM of rmMCP-4 or rCMA-1 were pre-incubated with either vehicle or TY-51469 (for a final concentration of 0.1 or 1 µM) or 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) (at concentration ranging from 0.1 to 2.5 µM) at 37°C for 20 min, then incubated with 0.12 nM of mouse plasmin or 0.24 nM human plasmin (Innovative Research, Inc., Novi, MI, USA) in 100 µL for 24 h at 37°C. Plasmin activity was determined by the hydrolysis rate of 50 µM of substrate, D-Ala-Leu-Lys-7-amido-4-methylcoumarin (Sigma Aldrich, Saint-Louis, MO, USA) at 37°C for 1 h, combined in a white 96-well microtitre plate. The fluorescence released was measured with an Infinite M1000 spectrophotometer (Tecan Group Ltd., Männerdorf, Switzerland) with λₑₓ = 370 nm and λₑₘ = 460 nm. A standard curve of 7-amino-4-methyl-coumarin (AMC) was also obtained in order to evaluate the concentration of the fluorogenic substrate cleavage (nM).
Fig 1- Kinetics of Chymase-dependent inactivation of human (A) and mouse (B) plasmins and its prevention by 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) or TY-51469
Fig 2-Chymase-dependent inactivation of human (A) and mouse (B) plasmins is prevented by 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) or TY-51469

### Results

Fig. 1 depicts the kinetics of recombinant human chymase (rCMA-1)-induced inactivation of human purified plasmin (Fig. 1A) and of recombinant mouse mMCP-4-induced inactivation of mouse purified plasmin (Fig. 1B) in absence or presence of increasing concentrations of 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) (graph i) or TY-51469 (graph ii). Each point represents the mean ± S.E.M (n = 6-15). One-way ANOVA with Dunnett's multiple comparison test was used to determine statistical significance with *p<0.05, **p<0.01, ***p<0.001. While plasmin resulted in a strong, continuous increase in fluorescence over time as a measure of enzyme activity when exposed to the fluorogenic substrate alone, the presence of chymase led to a strong reduction of fluorescence generation, which was in turn increased again concentration-dependently by both chymase inhibitors, fulacimstat and TY-51469, respectively. Fig. 2 depicts the maximum fluorescence obtained in each group at the end of the experiment (60 min).

These results surprisingly reveal that plasmin is a substrate of chymase in mouse and human and its degradation and inactivation can be prevented by the chymase inhibitors in animals and human rendering chymase inhibitors suitable for thrombus resolution, especially in the cause of acute thrombotic and thromboembolic events.

### 3.2 Plasmin is degraded by chymase, which can be inhibited by chymase inhibitors

Mouse chymase (rmMCP-4), or human chymase (rCMA-1) were activated as described above. 74 nM of rmMCP-4 or rCMA-1 were pre-incubated with either vehicle or the chymase inhibitor TY-51469 (10 or 300 µM) at 37°C for 20 min, then incubated for 30 min at 37°C with 235 nM of mouse plasmin (Innovative Research Inc., Novi, MI, USA) for rmMCP-4 or 235 nM of human plasmin (Innovative Research Inc., Novi, MI, USA) for rCMA-1. The reaction was stopped with an equal volume of formic acid (FA) (for a final concentration of 4 % (v/v) in water) and samples were maintained on ice until protein precipitation.

### In-solution trypsin digestion, purification and desalting of the peptides on C18 columns

Proteins from *in vitro* cleavage assays were first precipitated by the addition of trichloroacetic acid (TCA) to a final concentration of 10 % (v/v) and incubated for 30 min at -20°C. Proteins were centrifuged at 10,000 xg for 15 min. Protein pellet was washed with cold 100 % acetone, air dried, and re-suspended in 50 µL of a solution containing 8 M urea, 10 mM HEPES-KOH (pH 7.4). The protein reduction step was carried out by adding DTT (Thermo Fisher Scientific, Waltham, MA, USA) to a final concentration of 5 mM and boil at 95°C for 2 min, then incubated at room temperature for 30 min. The alkylation of the proteins was carried out by adding iodoacetamide (Sigma-Aldrich, Saint-Louis, MO, USA) to a final concentration of 7.5 mM final before incubating for 20 min at room temperature away from light. Urea concentration was decreased to 2 M by the addition of 150 µL of 50 mM ammonium bicarbonate (NH₄HCO₃). The proteins were digested by adding 1 µg Pierce MS-grade trypsin (Thermo Fisher Scientific, Waltham, MA, USA) and incubated overnight at 30°C. Digestion was stopped by adding trifluoroacetic acid (TFA) to a final concentration of 0.2 % (v/v). The peptides were purified with Pierce C18 100-µL tips column (Thermo Fisher Scientific, Waltham, MA, USA). Briefly, the C18 tip column was first moistened by suctioning 100 µL of 100 % acetonitrile (ACN) three times, then equilibrated by suctioning 100 µL of 0.1 % (v/v) TFA buffer 3 times. Each peptide sample was passed on the balanced C18 tip column by 10 succeeding up-and-downs of 100 µL of the sample. This step was performed twice in order to administer the entire sample on the column. The C18 tip column was then washed with 100 µL of 0.1 % (v/v) TFA buffer 3 times. The elution of the peptides was performed in a new low-binding microtube by 10 succeeding up-and-downs with a volume of 100 µL of 50 % (v/v) ACN and 1 % (v/v) FA buffer. This step was carried out 3 times to obtain a final volume of 300 µL. The peptides were then concentrated by centrifugal evaporator at 65°C until complete drying (approximately 60 min) and then resuspended in 25 µL of 1 % (v/v) FA buffer. Peptides were assayed using a NanoDrop spectrophotometer (Thermo Fisher Scientific, Waltham, MA, USA) and read at an absorbance of 205 nm. The peptides were then transferred to a glass vial (Thermo Fisher Scientific, Waltham, MA, USA) and stored at - 20°C until analysis by mass spectrometry.

### LC-MSIMS analyses

Trypsin-digested peptides were separated using a Dionex Ultimate 3000 nanoHPLC system. 10 µL of sample (a total of 2 µg) in 1 % (v/v) FA were loaded with a constant flow of 4 µL/min onto an Acclaim PepMap100 C18 column (0.3 mm id x 5 mm, Dionex Corporation). After trap enrichment, peptides were eluted onto an EasySpray PepMap C18 nano column (75 µm x 50 cm, Dionex Corporation) with a linear gradient of 5-35 % solvent B (90 % ACN with 0.1 % FA) over 240 min with a constant flow of 200 nL/min. The HPLC system was coupled to an Orbitrap QExactive mass spectrometer (Thermo Fisher Scientific, Waltham, MA, USA) via an EasySpray source. The spray voltage was set to 2.0 kV and the temperature of the column set to 40°C. Full scan MS survey spectra (m/z 350-1600) in profile mode were acquired in the Orbitrap with a resolution of 70,000 after accumulation of 1,000,000 ions. The 10 most intense peptide ions from the preview scan in the Orbitrap were fragmented by collision-induced dissociation (normalized collision energy 35 % and resolution of 17,500) after the accumulation of 50,000 ions. Maximal filling times were 250 ms for the full scans and 60 ms for the MS/MS scans. Precursor ion charge state screening was enabled and all unassigned charge states as well as singly, 7 and 8 charged species were rejected. The dynamic exclusion list was restricted to a maximum of 500 entries with a maximum retention period of 40 s and a relative mass window of 10 ppm. The lock mass option was enabled for survey scans to improve mass accuracy. Data were acquired using the Xcalibur software (Thermo Fisher Scientific, Waltham, MA, USA).

### Protein Identification by MaxQuant Analysis

The raw files were analyzed using the MaxQuant software (version 1.6.17.0) and the Uniprot mouse proteome database (07/03/2021, 55 366 entries) or the Uniprot human proteome database (21/03/2020, 75 776 entries), which contain the mouse plasminogen database (UniProtKB - P20918 (PLMN_MOUSE)) and the human plasminogen database (UniProtKB - P00747 (PLMN_HUMAN)). The settings used for the MaxQuant analysis were: 4 miscleavages were allowed; minimum peptide length of 6, fixed modification was carbamidomethylation on cysteine; enzymes were Trypsin (K/R) and CMA1 (F/Y); variable modifications included in the analysis were methionine oxidation, protein N-terminal acetylation and protein carbamylation (K, N-terminal). A mass tolerance of 10 ppm was used for precursor ions and a tolerance of 20 ppm was used for fragment ions. Identification values "PSM FDR", "Protein FDR" and "Site decoy fraction" were set to 0.05. Minimum peptide count was set to 1. Label-Free-Quantification (LFQ) was also selected with a LFQ minimal ratio count of 1. The "Second peptides" option was also allowed. Following the analysis, the results were sorted according to several parameters. Proteins positive for at least either one of the "Reverse", "Only.identified.by.site" or "Potential.contaminant" categories were eliminated. Numbering of plasmin/plasminogen amino acids was according to methionine in position 1.

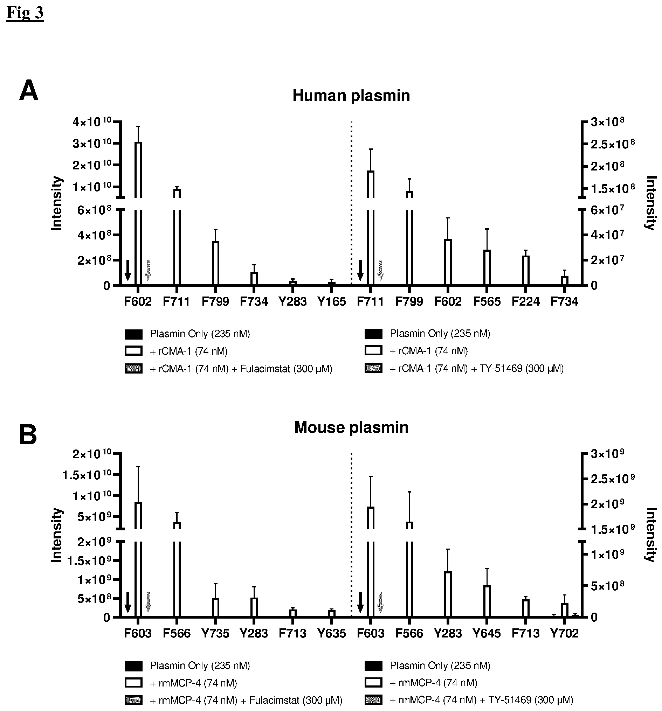

Fig 3-Human (A) and mouse (B) plasmins are substrates for chymase and their degradation by the latter protease is prevented by 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) or TY-51469

### Results:

The cleavage product identification by LC-MS/MS revealed several cleavage sites in the plasmin sequence caused by chymase, which are depicted in Fig. 3A for human plasmin and Fig. 3B for mouse plasmin with their intensity (arbitrary units, each bar represents the mean ± S.E.M; n = 4).

The homologous cleavage sites between human and mouse plasmin are at Y283/283, F602/603; F711/713, and F734/735 (human/mouse; Y=tyrosine, F=phenylalanine). Black arrows indicate the absence of these cleavages by plasmin alone, while the grey arrows show their absence, when chymase inhibitors were added to plasmin and chymase, thereby demonstrating that the identified cleavage site are caused by chymase.

### 4. Repressing chymase activity via gene knock-out or-pharmacological chymase inhibition in vivo reduces the thrombus weight in stenosis models efficaciously and dose-dependently and improves the blood flow

Because chymase is in general rapidly inactivated in plasma, it was not clear, whether the plasmin degrading properties of chymase might be relevant pathophysiologically and result in a reduced fibrin degradation *in vivo.* Therefore, we addressed the question *in vivo,* whether chymase inhibition has an impact on clot lysis and thereby vessel patency by keeping plasmin activity intact., Venous stenosis experiments were conducted either in mMCP4 knock-out mice or in mice and hamsters treated with the chymase inhibitors 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) or TY-51469 and compared to untreated or vehicle-treated animals.

By varying the timepoint of chymase inhibitor administration in relation to the start of stenosis we investigated the impact in a thrombus prophylaxis and a thrombus treatment setting.

### 4.1 Thrombus measurement in a venous stenosis model in mouse

As previously described by Payne & Brill and Canobbio et al. (Payne H., Brill A., J.Visc.Exp. (2017) doi: 10.3791/56697; Canobbio I et al., Blood130(2017)527-536), stenosis of the inferior vena cava (IVC) was performed in anesthetized mice (mixture of 2 % isoflurane and 2.5 % oxygen). A laparotomy was performed, and the guts were gently displaced without injury from the abdominal cavity and conserved in 0.9 % NaCl solution (37°C). The IVC was exposed by separation of the vessel between the renal and iliolumbar veins. For the partial ligation, a 30 G needle was used as a spacer. None of the side and back branches of the IVC were ligated. After the ligation with a polypropylene 7.0 sutures (FST, Foster City, CA) around both the IVC and the spacer, the latter was removed gently. By doing this, we ensure that the endothelium is not denuded and that a residual flow of 10 % in the IVC still remains. The guts were subsequently moved back in their original location in the abdomen and the abdominal cavity was closed in layers with a braided 5.0 absorbable suture. The analgesia protocol was maintained for 24 h after surgery with buprenorphine (0.1 mg/kg subcutaneous every 6-9 h). The IVCs were dissected post-euthanasia, and thrombi isolated, measured, weighed, and finally stored at -80°C.

Under these conditions, in wild-type male animals, thrombi with a weight of ~10 mg and a length of ~4-5 mm were observed.

The results in these 'control animals' were compared to animals with altered chymase activity, genetically- (i.e. mMCP-4 KO -/-) or pharmacologically-repressed:

### i) Knock-out mice

The mMCP-4 KO mice have been backcrossed for over 10 generations with C57BL/6 congeners and are highly congenial with the later strain (Tchougounova E. et al., J. Experiment.Med. 198(2003) 423-431). The genotype of mMCP-4 KO mice used in the present study was confirmed by ]polymerase chain reaction.

### ii) 1-(3-methyl-2-oxo-2,3-dihydro-1.3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) administration regimen

Mice were treated 1- or 24-hours post-ligation with a single oral dose of vehicle or 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) (0.1, 1 or 3 mg/kg) as previously described. The stenosis was evaluated 24- or 48-hours post-ligation. The IVCs were dissected, and thrombi were isolated, measured, and weighted.

### iii) TY-51469 administration regimen

TY-51469 (0.1, 1 or 10 mg/kg) was administered intraperitoneally 1 or 24 h post-ligation of the inferior vena cava (IVC). The stenosis was evaluated 24- or 48-hours post-ligation. The IVCs were dissected, and thrombi were isolated, measured, and weighted.

Fig 4- Fulacimstat or TY-51469 prevent (i) or resolve (ii) DVT in the IVC-ligated mouse (A) and hamster model (B)

### Results

The results of the thrombus weight and length measurements are depicted in Fig. 4A and summarized in Table 3 (results with 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat)) and Table 4 (results with TY-51469) showing that chymase activity repression in knock-out animals and in animals treated with a chymase inhibitors led to a dose-dependent thrombus reduction vs. control animals (n=6-13; each dot represents the data of one animal; columns represent the mean ± standard error of the mean; one-way ANOVA followed by Bonferroni's multiple comparisons test or Mann-Whitney T-test, * P<0.05, ** P<0.01, *** P<0.001).

Graph 4Ai) shows the thrombus weight measured 24 h after initiation of stenosis of mMCP4 knock-out mice and animal groups treated with different doses of either 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) (oral administration) or TY-51469 (intraperitoneal administration) 1 h after start of inferior vena cava (IVC) stenosis. With both compounds in a dose-dependent fashion, statistically significant effects on thrombus weight in the early post-stenosis setting (administration 1 h after ligation) were observed. Compound administration 24 h after start of stenosis with thrombus harvest after 48 h (Graph 4Aii) revealed a strong effect as well, resulting in near-to-complete thrombus reduction.

Surprisingly, under conditions of stenosis in the inferior vena cava a single administration of a chymase inhibitor results in a reduction of thrombus weight and length - not only in a prophylactic setting, i.e. administration of the compound before initiation of clot induced blood flow reduction-but also in a clear intervention setting, i.e. 24 h after the start of thrombus formation. These results were obtained by using two structurally different chymase inhibitors, 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) and TY-51469 in the well-established mouse DVT model as well as only with 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) in a newly developed hamster DVT model (both animal models with inferior vena cava stenosis) in order to confirm the general character of these findings. Achieving these effects with two unrelated chymase inhibitors from different structural classes demonstrates that this is not a compound-specific finding, but is relevant for chymase inhibition in general.

**Table 3 Impact of fulacimstat on thrombus weight and -length in a venous stenosis model in mouse**

| | Thrombus harvested 24 h after initiation of stenosis | | | | Thrombus harvested 48 h initiation of stenosis | |
|---|---|---|---|---|---|---|
| | vehicle | Administration of fulacimstat (mg/kg) 1 hr after start of stenosis | | | vehicle | Administration of fulacimstat (mg/kg) 24 h after start of stenosis |
| | | 0.1 | 1 | 3 | | 3 |
| N | 10 | 9 | 6 | 7 | 8 | 9 |
| Thrombus weight (mg) | 9.58 ± 1.84 | 5.26 ± 2.43 | 0.83 ± 0.74** | 0.34 ± 0.34*** | 14.34 ± 2.70 | 1.84 ± 0.51*** |
| Thrombus length (mm) | 4.40 ± 0.61 | 2.17 ± 0.87* | 0.42 ± 0.33*** | 0.29 ± 0.29*** | 5.31 ± 0.74 | 1.06 ± 0.28*** |

| | | | | | | |
|---|---|---|---|---|---|---|
| One-way ANOVA followed by Bonferroni's multiple comparisons test or Mann-Whitney T-test. (*) symbol represents statistical significance between vehicle and Fula-treatment. * P<0.05, ** P<0.01, *** P<0.001. Error type: Standard error of the mean (± SEM). | | | | | | |

**Table 4 Impact of mMCP-4 knock-out and of TY-51469 on thrombus weight and length in a venous stenosis model in mouse**

| | Thrombus observation 24 h after initiation of stenosis | | | | | Thrombus observation 48 h after initiation of stenosis | |
|---|---|---|---|---|---|---|---|
| | Control | Administration of TY-51469 (mg/kg) 1 h after initiation of stenosis | | | mMC P-4 KO | Control | Administration of TY-51469 (mg/kg) 24 h after initiation of stenosis |
| | | 0.1 | 1 | 10 | | | 10 |
| N | 13 | 7 | 7 | 9 | 8 | 8 | 8 |
| Thrombus weight (mg) | 10.77 ± 1.26 | 8.19 ± 2.29 | 4.04 ± 1.44** | 0.47 ± 0.4 7*** | 1.50 ± 0.98*** | 9.91 ± 1.20 | 1.29 ± 0.51*** |
| Thrombus length (mm) | 5.15 ± 0.45 | 3.79 ± 0.77 | 1.79 ± 0.65*** | 0.22 ± 0.22*** | 0.75 ± 0.49*** | 4.18 ± 0.38 | 1.0 ± 0.42*** |

| | | | | | | | |
|---|---|---|---|---|---|---|---|
| One-way ANOVA followed by Bonferroni's multiple comparisons test or Mann-Whitney T-test. (*) symbol represents statistical significance between control and TY-treatment. * P<0.05, ** P<0.01, *** P<0.001. Error type: Standard error of the mean (± SEM). | | | | | | | |

### 4.2 Thrombus measurement in a hamster venous stenosis model

In order to assure that the surprising effect in the mouse venous stenosis model was not species-specific, additional experiments were conducted in a novel hamster venous stenosis model described below.

Stenosis of the inferior vena cava (IVC) was achieved by anesthetizing hamsters of 108-120 g with a mix of isoflurane and oxygen (2 %; 2.5 % respectively). A laparotomy was performed and the guts were gently displaced without injury from the abdominal cavity and conserved in 0.9 % NaCl solution (37°C). The IVC was exposed by separation of the vessel between the renal and iliolumbar veins. A 27 G (outer circumference 0.4 mm) needle was used as a spacer. None of the side and back branches of the IVC were ligated. After the ligation with a polypropylene 7.0 suture (FST, Foster City, CA) around both, the IVC and the spacer, the latter was removed gently. The guts were subsequently moved back in their original location in the abdomen and the abdominal cavity was closed in layers with a braided 5.0 absorbable suture. The analgesia protocol was maintained for 24-h after surgery with buprenorphine (0.1 mg/kg subcutaneous every 6-9 h). The thrombi were evaluated 24 or 48 h post-stenosis: The IVCs were dissected, and thrombi were isolated, measured, and weighted.

Hamsters were treated 1 or 24 h after start of stenosis with a single oral dose of vehicle (10 % EtOH, 40 % solutol, 50 % sterile water) or fulacimstat (0.01, 0.1 and 1 mg/kg administered 1 h after start of stenosis, 1 mg/kg administered 24 h after start of stenosis) as previously described.

### Results:

The results of the thrombus weight and length measurements are depicted in Fig. 4B and summarized in Table 5 showing that chymase activity repression in hamsters treated with the chymase inhibitor 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) led to a dose-dependent thrombus reduction vs. control animals with statistically significant thrombus reductions at higher doses in the early post-stenosis setting (administration 1 h after start of stenosis, harvest after 24 h; Graph 4Bi)). Administration of 1 mg/kg fulacimstat 24 hours after start of IVC stenosis with thrombi harvested after 48 h (Graph 4Bii)) revealed a strong effect as well, resulting in total elimination of thrombi.

The venous stenosis model in the hamster yielded very similar results compared to the mouse venous stenosis model, thus demonstrating that the thrombus reduction by chymase inhibition is not a species-specific phenomenon.

Remarkable is that in this case, thrombi size in the vehicle group harvested at 24 h was in weight and length very similar to the vehicle group with thrombi harvested at 48 h, thus demonstrating that the thrombus had already reached a plateau in size after 24h, when fulacimstat was administered. (n=6-8; mean ± standard error of the mean; one-way ANOVA followed by Bonferroni's multiple comparisons test or Mann-Whitney T-test, * P<0.05, ** P<0.01, *** P<0.001 ).

**Table 5 Impact of fulacimstat on thrombus weight and length in the hamster venous stenosis model**

| | Thrombus observation 24 h after initiation of stenosis | | | | Thrombus observation 48 h after initiation of stenosis | |
|---|---|---|---|---|---|---|
| | Vehicle | Administration of fulacimstat (mg/kg) 1 h after initiation of stenosis | | | Vehicle | Administration of fulacimstat (mg/kg) 24 h after initiation of stenosis |
| | | 0.01 | 0.1 | 1 | | 1 |
| N | 8 | 7 | 6 | 6 | 8 | 6 |
| Thrombus weight (mg) | 23.76 ± 2.39 | 18.36 ± 5.15 | 9.27 ± 5.90* | 0.0*** | 24.30 ± 4.33 | 0.0*** |
| Thrombus length (mm) | 8.13 ± 0.95 | 6.07 ± 1.58 | 3.17 ± 2.00* | 0.0*** | 9.06 ± 0.98 | 0.0*** |

| | | | | | | |
|---|---|---|---|---|---|---|
| One-way ANOVA followed by Bonferroni's multiple comparisons test or Mann-Whitney T-test. * P<0.05, ** P<0.01, *** P<0.001. Error type: Standard error of the mean (± SEM). | | | | | | |

### 5. Direct chymase inhibition improves the blood flow in a venous stenosis model in mice

The main goal in thrombosis is to ensure sufficient blood flow for an appropriate maintenance of the tissue surrounding the vessel. To evaluate whether the thrombus reduction by chymase inhibition described in 4.1 and 4.2 is associated with an increase in patency in the inferior vena cava (IVC) versus control, a high-frequency ultrasound imaging system (Vevo 3100; Fujifilm VisualSonics Inc.) equipped with MX400 linear array transducer (40 MHz) was used to visualize by Doppler color blood flow mapping the blood flow in the inferior vena cava (IVC) and abdominal aorta (AA) at different time points in the venous stenosis model as described in 4.1. Mice were anesthetized during the procedure (2 % isoflurane; 4 % oxygen) and their abdomens were shaved and covered with ultrasound gel. Measurements of the blood flow in the vessels were performed 12 h pre-ligation and 6 and 24 h post-ligation in control mice and mice with intraperitoneal administration of 10 mg/kg TY-51469 1 h after initiation of stenosis.

### Results

The results of the blood flow velocity analyses in the inferior vena cava (IVC) and the abdominal aorta (AA) measured before, 6 and 24 h after initiation of the stenosis in control animals and animals treated with TY-51469 1 h after initiation of the stenosis are summarized in Table 6.

These results indicate that while there was a complete loss of blood flow observed in the control IVC-ligated animals, in the animals treated with a chymase inhibitor the blood flow in the vena cava was restored already 6h after initiation of stenosis. This outcome demonstrates that chymase inhibition does not only result in thrombus reduction, but has a clear impact on the blood flow in the injured vessel as well.

**Table 6 Blood flow velocities in murine IVC and AA +/- TY-51469 1h after start of stenosis**

| Time post-ligation | Abdominal aorta velocity (mm/s) | | Inferior vena cava velocity (mm/s) | |
|---|---|---|---|---|
| | Control | + 10 mg/kg TY-51469 | Control | + 10 mg/kg TY-51469 |
| Non-ligated | 463.8 ± 18.5 | 508.2 ± 29.7 | 103.3 ± 17.0 | 124.6 ± 28.7 |
| 6 h | 669.3 ± 38.2*** | 487.6 ± 33.1 | 0*** | 171.6 ± 27.1 |
| 24 h | 595.9 ± 22.2* | 505.8 ± 40.4 | 0*** | 158.5 ± 18.3 |

| | | | | |
|---|---|---|---|---|
| One-way ANOVA followed by Bonferroni's multiple comparisons test. * P<0.05, *** P<0.001. Error type: Standard error of the mean (± SEM). | | | | |

### 6. Reducing chymase activity in vivo via direct chymase inhibitor eliminates pulmonary embolization in mouse and hamster stenosis models

A very important issue of any thrombus resolution therapy is the question whether the disintegration of the thrombus may lead to the generation of emboli, which may be transported to the lungs and cause pulmonary embolism. To address the question whether chymase inhibition may have an impact on embolization, the lungs of the mice and hamsters of the venous stenosis experiments with and without administration of fulacimstat, were isolated. The right pulmonary lobes were used to visually quantify the number of embolized lungs.
Fig 5 - Reduced number of pulmonary emboli in lungs of mice treated with fulacimstat
Fig 6 - Reduced number of pulmonary emboli in lungs of hamsters treated with fulacimstat

### Results:

The results of the macroscopical lung analyses are depicted in Fig. 5 (mouse) and Fig. 6 (hamster) and are summarized in Table 7.

In Fig. 5A and Fig. 6A, randomly selected macroscopic images of right pulmonary lobes extracted from 5 vehicle or 5 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) treated mice or hamsters (average of 6-10 animals/group) are presented. Pulmonary thromboemboli are indicated by black arrows and were observed in vehicle-treated mice or hamsters in the upper panels in Fig. 5A and Fig. 6A, respectively. The right pulmonary lobes of mice and hamsters treated perorally with 3 mg/kg (mouse) and 1 mg/kg (hamster) of fulacimstat 24 h after venous stenosis, are depicted in the lower panels in Fig. 5A and Fig. 6A. They showed normal lung anatomy and no signs for pulmonary embolism 24 h after induction of stenosis.

Part B in both figures summarizes the lung embolism incidence in vehicle-treated mice (Fig. 5) and hamsters (Fig. 6) in the stenosis model at 24 h or 48 h after start of stenosis in pie charts. Emboli were observed in 30-50% of the vehicle animals.

Mice treated with 3 mg/kg fulacimstat (Fig. 5) and hamsters treated with 1 mg/kg fulacimstat (Fig. 6) did not show signs of embolism at any time point, i.e. administration 1h after start of stenosis and read out at 24 h or administration 24h after start of stenosis and read out at 48 h.

Therefore, the profibrinolytic approach by chymase inhibition did not only show no increase in emboli vs. control animals, but it surprisingly resulted in a strong reduction of pulmonary emboli.

The vehicle data after 24 h may imply that emboli might already be present in the animals before treatment with fulacimstat at 24 h after start of stenosis. The finding in both species that no emboli were observed in the treated mice and hamsters after 48 h suggests to our surprise that chymase inhibition may not only reduce thrombi in the local environment of their generation, as shown for the thrombi in the vena cava, but it may reduce emboli, as well.

Surprisingly, when examining the lungs in the venous (IVC) stenosis experiments in mice and hamsters, a reduced number of pulmonary emboli in the lungs of the animals under chymase inhibition compared to control animals was observed. This very important and relevant case of thromboembolism implies that chymase not only has a role at the local site of thrombus formation, but has antifibrinolytic effects in emboli, as well. Therefore, chymase inhibitors are effective agents during embolic diseases, including for example the resolution of emboli from deep veins causing pulmonary embolism or from the left atrial appendix in atrial fibrillation patients causing stroke or systemic embolism.

**Table 7 Impact of fulacimstat on the incidence of pulmonary embolism in the venous stenosis model**

| Species | fulacimstat (mg/kg) | Compound administration 1h after stenosis, PE occurrence (absolute and relative (%)) after 24h | Compound administration 24 h after stenosis, PE occurrence (absolute and relative (%)) after 48h |
|---|---|---|---|
| Mouse | 0 (vehicle) | 3/10 (30) | 5/10 (50) |
| | 0.1 | 1/9 (11) | N/A |
| | 1 | 0/6 (0)* | N/A |
| | 3 | 0/7 (0)* | 0/9 (0)* |
| Hamster | 0 (vehicle) | 4/9 (44) | 5/10 (50) |
| | 0.01 | 3/7(43) | N/A |
| | 0.1 | 3/11(27) | N/A |
| | 1 | 0/6(0)* | 0/6 (0)* |

| | | | |
|---|---|---|---|
| One-way ANOVA followed by Bonferroni's multiple comparisons test. * P<0.05,. Error type: Standard error of the mean (± SEM). | | | |

### 7. Chymase inhibitors reduce efficaciously and dose-dependently the thrombus weight in thrombosis models of vessel wall injury (FeCl₃-induced injury model)

To exclude that the effect of chymase inhibition is limited to stenosis models, we addressed the question whether thrombus generation triggered by vessel wall injury can be prevented by chymase inhibition in short-term ferric chloride-induced injury models, which were conducted in mouse and hamster.

### 7.1 Ferric chloride-induced injury at the inferior vena cava in mouse

The FeCl₃-induced injury model was performed as previously described (Aghourian MN et al, J.Thromb.Hemost.10(2012)447-452; Li W et al., J.Vis.Exp.54479 (2016) doi:10.3791/54479; Wang X et al., J.Thromb.Hemost.4(2006)403-416). Wild type male mice were treated with either vehicle, 10 mg/kg fulacimstat or 10 mg/kg TY-51469 1 h prior to the anesthesia of the animals, whereas mMCP-4 knock-out animals were not treated before anesthesia. The endothelial injury model of the inferior vena cava (IVC) was achieved by anesthetizing the mouse with ketamine/xylazine (87/13 mg/kg IM). A laparotomy was performed, and the guts were gently displaced without injury from the abdominal cavity and conserved in 0.9 % NaCl solution (37°C). The IVC was exposed by separation of the vessel between the renal and iliolumbar veins. A 2x2 mm double layered absorbent gauze saturated in 0.37 M (10 % w/v in sterile water) ferric chloride hydrate (FeCl₃) (Sigma-Aldrich, St-Louis, MO, USA) was applied directly on the IVC for 3 min and then removed. The guts were subsequently moved back in their original location in the abdomen. Mice were sacrificed 30 min later to access the thrombotic area in the IVC. IVCs were dissected and thrombi were isolated and weighted with an analytical balance (0.1 mg accuracy, Entris II, Sartorius).

Fig 7- Chymase inhibition reduces thrombus weight in the FeCl₃-induced injury model in the inferior vena cava (IVC) in mouse (A) or hamster (B) and in the vena femoralis (hamster, C)

### Results

The results of the thrombus measurements are depicted in Fig. 7A and summarized in Table 8. They show that oral administration of 10 mg/kg 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) and intraperitoneal administration of 10 mg/kg of TY-51469 before the injury - and therefore in a prevention setting - or mMCP-4 KO resulted in a statistically significant thrombus reduction vs. control animals.

**Table 8 Impact of chymase inhibition on thrombus weight in a FeCl₃-induced injury thrombosis model at the inferior vena cava in mouse**

| | Vehicle | 10 mg/kg fulacimstat | Control | 10 mg/kg TY-51469 | mMCP-4 KO |
|---|---|---|---|---|---|
| N | 7 | 6 | | 10 | 10 |
| Thrombus weight (mg) | 3.19 ± 0.37 | 0.77 ± 0.25*** | 3.70 ± 0.21 | 1.31 ± 0.23^{&&&} | 0.82 ± 0.308^{&&&} |

| | | | | | |
|---|---|---|---|---|---|
| One-way ANOVA followed by Bonferroni's multiple comparisons test or Mann-Whitney T-test. (*) symbol represents statistical significance between vehicle and fulacimstat treatment. (^{&}) symbol represents statistical significance between control, TY-51469 treatment and mMCP-4 KO mice. * P<0.05, ** P<0.01, *** P<0.001. Error type: Standard error of the mean (± SEM). | | | | | |

7.2 Ferric chloride-induced injury at the vena cava or the vena femoralis in hamster The FeCl₃-induced injury model in hamster is highly inspired by the methodology used in mice (section 7.1). Golden Syrian hamsters were treated orally with either vehicle, 3 or 10 mg/kg 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) 1 h prior to the anesthesia of the animals.

### Ferric chloride-induced injury at the vena cava

The endothelial injury of the inferior vena cava (IVC) was achieved by anesthetizing the hamster, weighting between 115-130 g, with ketamine/xylazine (87-200/13 mg/kg IM). Animals were kept at body temperature of 37°C with a heating pad throughout the experimentation. A laparotomy was performed, and the guts gently displaced without injury from the abdominal cavity and conserved in 0.9 % NaCl solution (37°C). The IVC was subsequently exposed by separation of that vessel between the renal and iliolumbar veins. A 2x2 mm double layered absorbent gauze saturated in 0.37 M (10 % w/v in sterile water) hydrate ferric chloride (FeCl₃) (Sigma-Aldrich, St-Louis, MO, USA) was applied directly on the IVC for 3 minutes and then removed. The guts were subsequently moved back in their original location in the abdomen. Hamsters were sacrificed 30 minutes later to localize the thrombotic area within the injured IVCs.

### Ferric chloride-induced injury at the vena femoralis

The endothelial injury model of the femoral vein (FV) was achieved by anesthetizing the hamsters of 115-130 g, with ketamine/xylazine (87-200/13 mg/kg IM). Animals were kept at body temperature of 37°C with a heating pad throughout the experimentation. The left vena femoralis was exposed after a skin incision and separation from arteria femoralis. A 2x8 mm double layered absorbent gauze saturated with 3 µl of 5 % hydrate ferric chloride (Sigma-Aldrich, St-Louis, MO, USA) solved in water (5% w/v in sterile water) was applied directly on the vena femoralis for 5 minutes and then removed. Hamsters were sacrificed 30 minutes later, and the femoral vein was dissected.

### Harvest and characterization of the thrombi

Relevant pieces of the inferior vena cava and the vena femoralis were dissected and thrombi were isolated and weighted with an analytical balance (Sartorius).

### Results

The results of the thrombus measurements are depicted in Fig. 7B and Fig 7C, respectively, and summarized in Table 9, showing that chymase activity reduction with 3 and 10 mg/kg 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) resulted in a statistically significant reduction of thrombus weight vs. control animals. This was demonstrated not only in the inferior vena cava, but also in the much smaller vena femoralis, thereby showing that this effect of chymase inhibition is not limited to large vessels.

**Table 9 Impact of chymase inhibition by fulacimstat on the thrombus weights in FeCl₃-induced injury thrombosis model at the inferior vena cava and the vena femoralis**

| | Injury at the vena cava | | | Injury at the vena femoralis | | |
|---|---|---|---|---|---|---|
| | Vehicle | fulacimstat (mg/kg) | | Vehicle | fulacimstat (mg/kg) | |
| | | 3 | 10 | | 3 | 10 |
| N | 6 | 6 | 6 | 16 | 7 | 8 |
| Thrombus weight (mg) | 5.02 ± 0.78 | 1.83 ± 0.55** | 0.78 ± 0.31*** | 0.80 ± 0.05 | 0.60 ± 0.05* | 0.36 ± 0.06*** |

| | | | | | | |
|---|---|---|---|---|---|---|
| One-way ANOVA followed by Bonferroni's multiple comparisons test or Mann-Whitney T-test. * P<0.05, ** P<0.01, *** P<0.001. Error type: Standard error of the mean (± SEM). | | | | | | |

### 7.3 Ferric chloride-induced injury of the carotid artery in the mouse and the hamster

To exclude that the effect of chymase inhibition is limited to venous thrombosis models, we addressed the question whether thrombus generation triggered by arterial vessel wall injury can be prevented by chymase inhibition in short-term ferric chloride-induced carotid artery injury models, which were conducted in mouse and hamster.

Fig 13 - Fulacimstat reduces thrombus induced occlusion in the FeCl3-induced injury model in the common carotid (CCA) in mouse or hamster. Each series of images are representative of 6 independent experiments.

### Ferric chloride-induced injury of the carotid artery in the mouse

The FeCl₃-induced injury model was performed as previously described (Bonnard T et al., J.Vis.Exp.2015; doi:10.3791/52838). Wild type male mice were treated with either vehicle, 10 or 20 mg/kg fulacimstat 1 h prior to the anesthesia.

The endothelial injury model of the common carotid artery (CCA) was conducted by anesthetizing the mouse with ketamine/xylazine (87/13 mg/kg IM). A 1 cm vertical skin incision of the neck was performed and the right CCA and vagus nerve were exposed and separated. A 2x2 mm double layered absorbent gauze saturated in 0.28 M (7.5 % w/v in sterile water) ferric chloride hydrate (FeCl₃) (Sigma-Aldrich, St-Louis, MO, USA) was applied directly on the exposed CCA for 1 min and then removed. The CCAs were subsequently moved back in their original location near the trachea. Mice were sacrificed 30 min later. Chemically injured as well as intact contralateral CCAs were dissected and conserved in PFA (4% w/v) over-night, then EtOH (70%) at 4°C for subsequent histology analysis.

### Results

Histology of CCAs post-FeCl₃ administration and quantification of thrombus area (Image J), with identification of the reference section for thrombus area measurement (first section showing FeCl₃-induced vascular injury) are depicted in Fig. 13 A and B. Each series of images are representative of 6 independent experiments. They show that oral administration of 10 or 20 mg/kg fulacimstat before the injury resulted in a statistically significant thrombus reduction vs. control animals, in dose-dependent fashion in the mouse (Fig. 13 C; One-way ANOVA followed by Bonferroni's multiple comparisons test or Mann-Whitney T-test. * P<0.05, ** P<0.01, *** P<0.001. Error type: Standard error of the mean (± SEM)).

### Ferric chloride-induced injury of the carotid artery in the hamster

The FeCl₃-induced injury model in hamster is highly inspired by the methodology used in mice (section 7.3). Golden Syrian hamsters were treated orally with either vehicle or 10 mg/kg 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) 1 h prior to the anesthesia of the animals.

The endothelial injury of the CCAs was achieved by anesthetizing the hamster, weighting between 115-130 g, with ketamine/xylazine (87-200/13 mg/kg IM). Animals were kept at body temperature of 37°C with a heating pad throughout the experimentation. A 2 cm vertical skin incision of the neck was performed and the right CCA and vagus nerve were exposed by separation. A 2x2 mm double layered absorbent gauze saturated in 1.11 M (30 % w/v in sterile water ferric chloride hydrate (FeCl3) (Sigma-Aldrich, St-Louis, MO, USA) was applied directly on the exposed CCA for 3 min and then removed (Kuo et al., J.Biomed.Sci, 2009). The CCAs were subsequently moved back in their original location near the trachea. Hamsters were sacrificed 30 min later to access the thrombotic area in the CCA. Chemically injured as well as intact contralateral CCAs were dissected, and thrombi were isolated and weighted with an analytical balance (0.1 mg accuracy, Entris II, Sartorius).

### Results

The results of the thrombus measurements are quantified in Fig. 13 D. They show that oral administration of 10 mg/kg fulacimstat before the injury resulted in a statistically significant thrombus reduction vs. control animals in the hamster. (One-way ANOVA followed by Bonferroni's multiple comparisons test T-test. ** P<0.01. Error type: Standard error of the mean (± SEM))

### 7.4 Overall interpretation of the results of the FeCl₃-induced injury models

Mice and hamsters with reduced chymase activity either by chymase inhibitors or mMCP-4 knockout had reduced thrombus weights compared to vehicle control animals in these models of venous and arterial vascular wall injury. This is surprising, because in contrast to the stenosis model, the thrombi in this model are generated rapidly after the injury of the vascular wall and are isolated already after 30 min. Therefore, it can be viewed as a thrombus generation rather than as a thrombus resolution model. However, even under these conditions, the profibrinolytic effect of the compounds administered prophylactically appear strong enough to balance the procoagulant effects of a growing thrombus.

Surprisingly, in contrast to the ferric chloride-induced injury experiments in mesenteric arterioles of Ponomaryov et al., ferric chloride-induced injury experiments at different venous and arterial vessels described in this invention demonstrate significant differences in the groups treated with chymase inhibitors vs. the vehicle groups. In these short-term thrombosis experiments, the compounds were given before the initiation of the thrombus formation by ferric chloride, implying that even in the early phase of ongoing clot formation chymase inhibitors have an impact on the thrombus weight and thereby on the risk for vessel occlusion.

### 8. Chymase is present in thrombi generated in the in vivo studies in mouse and in human thrombus specimens

To address the question whether chymase is present in thrombi in the animal studies and in human thrombi, and thereby evaluate translatability aspects towards the clinical situation, immuno-histochemical experiments were conducted.

Due to the lack of antibodies binding hamster chymase, the experiments were focused on thrombi from in vivo studies in mice.

### 8.1 Immunohistochemical analysis of mouse chymase and perivascular mast cells in mouse thrombi

Immunochemistry was performed on healthy and ligated parts of the inferior vena cava of wild type and mMCP-4 KO mice. The specimens were fixed with 10 % buffered formalin solution, processed, embedded in paraffin and finally sliced (4 µm slice) at the service of the histopathology platform at RI-MUHC (Montreal, Québec, Canada). Toluidine blue (TB) was used to stain mast cells, and mMCP-4 was detected using a goat Polyclonal Anti-MCPT4 antibody (US Biological life science, cat. # M2414-20A, 1x400 dilution) and biotinylated horse anti-goat IgG (Vector Laboratories, cat. # BA9500, 1x200 dilution Chymase was detected using goat polyclonal IgG to human MCPT4 (Abcam, cat. # ab111239, 1x200 dilution).

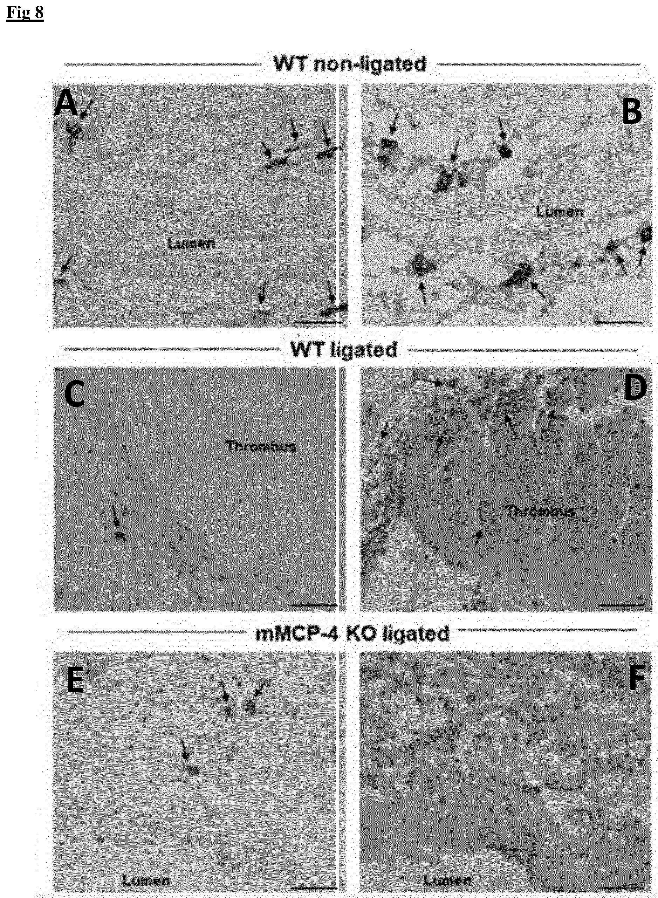

Fig 8 - Deep vein thromboses show immunoreactive chymase-containing mast cells in IVC-ligated wild type mice. Each image is representative of 3 different tissue samples

### Results

Representative images of the immunochemical detection of chymase in the thrombi derived from the experiments are depicted in Fig. 8 (scale bar: 50 µm; black arrows in left panels: mast cells; black arrows in right panel: mMCP-4 immunoreactivity). The images are representative of 3 independent experiments.

24h after start of stenosis, mMCP-4-specific immunoreactivity was localized in the periphery as well as within the thrombi in the ligated vena cava of wild type animals (Fig. 8D), but not in thrombus-free, ligated veins isolated from mMCP-4 KO mice (Fig. 8F) nor from non-ligated thrombus-free vessels from wild type animals (Fig. 8B). In the later specimens the vast majority of mMCP-4-specific immunoreactivity was detected in the perivascular mast cells. Toluidine blue-stained mast cells, on the other hand, were identified in the adventitia of the vena cava specimens derived from both WT and mMCP-4 KO mice (Fig. 8A, E).

### 8.2 Immunohistochemical analysis of human chymase and mast cells in human specimens from deep vein thrombosis, pulmonary embolism and popliteal artery occlusion

To demonstrate that chymase is present not only in thrombi in animal experiments but plays a role in the degradation of plasmin in human thrombi as well, specimens from human thrombotic events were evaluated regarding their chymase immunoreactivity.

Human venous thrombi pre-made slides were obtained from Tissue for Research Ltd (Ellingham, Suffolk, UK), human arterial thrombi from Discovery Life Science (Powell, OH, USA) whereas pulmonary embolism samples were obtained from the Dept of Pathology, McGill University.

For the identification and quantification of mast cells toluidine blue staining of basophilic granules was used. It is an established method, which stain metachromatically red-purple with blue background. Staining solutions were obtained from Newcomers Supply.

For the identification of chymase in human samples a immunochemical analysis was performed. Deparaffinized and rehydrated tissue slides (4 microns) were placed on the Leica Bond Max automated staining system using the Bond Polymer Refine Detection kit (DS9800). HIER (Heat Induced Antigen Retrieval) was performed using ER2 solution (AR9640), at 95°C for 20 minutes. Peroxidase block was then performed for 5 minutes. Primary antibody (mouse anti-human Mast Cell Chymase antibody [CC1] (Abcam: ab2377)) was then added and incubated for 15 minutes. A rabbit anti-mouse secondary antibody solution was added for 8 minutes. Anti-rabbit poly-HRP reagent from Leica kit was added for 15 minutes. DAB (3,3'-diaminobenzidine) solution was added for 5 minutes. Counterstain: Hematoxylin was added for 2 minutes. Slides were rehydrated with graded ethanol (70%, 95%, and 100%) and cleaned through three changes of xylene, and then air dried and mounted. Human Tonsil tissue was used as quality control.

Fig 9 - Human venous thrombus, pulmonary embolism and arterial thrombosis show immunoreactive chymase-containing mast cells. Each image is representative of 3 different tissue samples.

### Results

Representative images of chymase-specific immunostaining in human specimens are depicted in Fig 9 (dilution of the human chymase antibody: 1/200; black arrow indicated mMCP-4 immunoreactivity). All images are representative of 3 independent experiments.

Chymase is located inside human deep vein thrombi (from left iliac, right femoral, and left groin varicose vein) as depicted in the example in Fig. 9A, inside pulmonary emboli as shown in Fig. 9B, and in a popliteal artery thrombus (Fig. 9C). All samples reveal chymase immunoreactivity and also chymase-positive mast cell inside thrombi (black arrows).

These results show for the first time, that chymase is present in human thrombi and therefore plays a role in the degradation of plasmin and thus has antifibrinolytic effects in that human pathology. A chymase inhibitor displays profibrinolytic effects by keeping plasmin intact and result in a rapid thrombus resolution - as has been shown in the animal models.

### 9. Chymase activity is present in thrombi and can be inhibited by chymase inhibitors, which leads to an increase in plasmin activity in the thrombi

While the presence of chymase in the thrombi could be verified, the impact of it in the thrombus was not clear. Therefore, experiments were conducted to measure the activity of chymase and plasmin in thrombus homogenates of control animals and animals treated with chymase inhibitors.

### 9.1 Ex vivo determined intra-thrombus chymase activity is decreased and plasmin activity is increased in thrombi treated with a chymase inhibitor

To demonstrate that the thrombus reduction in animals treated with chymase inhibitors was associated with a decrease in chymase activity and an increase in plasmin activity in the thrombi, chymase and plasmin were extracted from mouse and hamster thrombi of experiments described thereafter and enzyme activities were measured in mouse and hamster.

### Chymase extraction ex vivo from mouse and hamster thrombi

Thrombi were homogenized in 10 volumes (w/v) of 20 mM PBS (pH 7.4) supplemented with 0.5 mg/mL of BSA using a glass-Teflon homogenizer (Kakizoe E. et al., J. Invest. Dermatol. 116(2001)118-123). The homogenates were centrifuged at 18,000 xg for 30 min at 4°C and the supernatants were discarded. We repeated this last step two more time, except for the last centrifugation where we kept the supernatants. Supernatants were kept at 4°C until tested for chymase enzymatic activity the same day.

### Plasmin extraction ex vivo from mouse and hamster thrombi

Thrombi were homogenized in 10 volumes (w/v) of 20 mM PBS (pH 7.4) supplemented with 1.5 mg/mL of BSA using a glass-Teflon homogenizer. The homogenates were transferred to 1 mL ultracentrifuge tubes and centrifuged at 100,000 xg for 20 min at 4°C⁴⁸. Supernatants were kept at 4°C until tested for plasmin enzymatic activity the same day.

### Measurement of intra-thrombus plasmin or chymase enzymatic activity

30 µL of supernatants from thrombus extracts were used and placed directly in a white 96-well plates. No variation in protein concentration per volume unit of thrombi were found with Bradford protein assay (result not showed). Some homogenates were treated with either 10 µM of TY-51469 or fulacimstat (volume of 5 µL) prior to fluorescence readings. 65-70 µL of 20 mM PBS (pH 7.4) supplemented with 1.5 mg/mL of BSA (for plasmin) or 0.5 mg/mL (for chymase) were added to each well to achieve a final volume of 100 µL. Plasmin activity was determined by the hydrolysis rate of 50 µM of substrate, D-Ala-Leu-Lys-7-amido-4-methylcoumarin (Sigma Aldrich, Saint-Louis, MO, USA) at 37°C for 1 h. Chymase activity was determined by the hydrolysis rate of 10 µM of substrate, Suc-Leu-Leu-Val-Tyr-7-amido-4-methylcoumarin (Peptide Institute Inc., Osaka, Japan) at 37°C for 1 h. The fluorescence released was measured with an Infinite M1000 spectrophotometer (Tecan Group Ltd., Männerdorf, Switzerland) with λₑₓ = 370 nm and λₑₘ = 460 nm. A standard curve of AMC was also obtained in order to evaluate the concentration of the fluorogenic substrate cleavage (nM).

Fig 10 - Chymase inhibitors increase plasmin activity in thrombi from mice (A) or hamsters (B)

### Results

The results of intra-thrombus enzymatic activities for chymase or plasmin are depicted in Fig. 10 and summarized in Table 10. Plasmin and chymase activities were measured for 60 min as AMC specific cleavage (nM) of the fluorogenic substrate D-Ala-Leu-Lys-AMC (for the former) or Suc-Leu-Leu-Val-Tyr-AMC (for the latter enzyme), respectively. Each bar represents the mean ± S.E.M of a minimum of 6 experiments. ** P<0.01, *** P<0.001.

Chymase activity was observed in mouse and hamster thrombi (Fig. 10Ai and Fig. 10Bi, respectively). To our knowledge this is the first time that chymase activity is shown ex vivo in thrombotic material. These experiments demonstrate that - while chymase is known to be inactivated in whole blood or plasma very rapidly - in the local microenvironment between vascular wall and thrombus, the chymase activity surprisingly remains intact. The chymase activity is reduced almost completely in thrombi treated with the chymase inhibitors 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) or TY-51469 (the right bar in Fig. 10Ai and Fig. 10Bi, respectively). Measurements of plasmin activity in the same thrombi demonstrate that after addition of chymase inhibitors the plasmin activity is strongly increased in both mouse and hamster thrombi (Fig.10Aii, Fig.10Bii), which confirms the proposed mechanism of action.

While rapidly inactivated in plasma - chymase surprisingly remains active in the local microenvironment of a thrombus, and addition of chymase inhibitors results in a decrease of chymase activity and an increase of its substrate plasmin in this environment.

**Table 10 Intra-thrombus enzymatic activities of chymase and plasmin in thrombi derived from mouse and hamster venous stenosis experiments**

| | Thrombus from venous stenosis in mouse | | Thrombus from venous stenosis in hamster | |
|---|---|---|---|---|
| | Control | + 10 µM TY-51469 | Control | + 10 µM fulacimstat |
| N | 6-8 | 7 | 6 | 6 |
| Chymase activity (nM AMC) | 21.05 ± 2.61 | 0.33 ± 0.25*** | 13.56 ± 0.86 | 0.0 ± 0.83*** |
| Plasmin activity (nM AMC) | 3.74 ± 0.47 | 13.16 ± 1.26*** | 4.29 ± 1.10 | 9.44 ± 0.98** |

| | | | | |
|---|---|---|---|---|
| One-way ANOVA followed by Bonferroni's multiple comparisons test or Mann-Whitney T-test. * P<0.05, ** P<0.01, *** P<0.001. Error type: Standard error of the mean (± SEM). | | | | |

Surprisingly, while it was common belief that the principal mechanism behind the murine venous stenosis experiment of Ponomaryov et al. (Ponomaryov et al., CircRes.121(2017)941-950) was related to coagulation, activity measurements of chymase and plasmin activity in the removed thrombi from animals in our studies reveal a strong association between chymase and the fibrinolytic system. The present invention demonstrated that while chymase activity was reduced, plasmin activity was increased in the thrombi of animals treated with chymase inhibitor.

### 9.2 Impact of chymase inhibitors on human clot in vitro (mod. "Halo assay" method)

To demonstrate that plasmin-dependent fibrinolysis of human thrombi is reduced by human chymase, the impact of this mast cell-derived serine protease was tested in halo-type human blood concentric clot formations.

### Formation of halo-shaped blood clot containing human chymase

Clot formation based on the methods of Bonnard et al., (Sci Rep. 2017 May 24;7(1):2346) is induced by the addition of 31.2 mM of calcium chloride (CaCl₂) to blood samples collected from healthy donors. 1 µl of human chymase, for a final concentration of 0.5 µM or 1 µM, in presence or absence of 10 µM of fulacimstat (only for the latter concentration of rCMA-1), is deposited onto the bottom edge of the wells of a flat-bottomed, transparent 96-well plate. 20 µl of CaCl₂-supplemented blood is subsequently added in circular motion around the edge of each well, allowing the formation of a halo-shaped blood clot (fluidic cohesion effect), leaving the center of the wells empty. The plate is subsequently sealed and incubated at 37°C for 30 min to allow solidification of the blood halo.

### Thrombolysis study with rtPA

Recombinant human tissue plasminogen activator (rtPA, Cathflo^{®}, Genentech Inc., SF, USA) induces blood clot fibrinolysis by activating endogenous plasmin. rtPA was diluted to a concentration of 1.5 nM in 80 ul of PBS at a pH 7.4 and was added into each well. The 96-wells plate was subsequently incubated at 37°C for 30 min. Negative controls (Aₜₒₜₐₗ) were obtained from addition of 80 ul of PBS without fibrinolytic agents, and positive controls (A_{zero}) correspond to wells containing 20 µl of fresh blood, not supplemented with CaCl₂, and 80 µl of PBS. The fibrinolysis rate was measured with a plate reader, (Infinite M1000 spectrophotometer (Tecan Group Ltd., Männerdorf, Switzerland)). The degradation of the halo was measured in real time as coverage of each experimental well by the thrombolysed blood increases absorbance at 510 nm. Changes in absorbance were measured every minute at 37°C for 2h, after a 5 second orbital shaking at 195.3 rpm at an amplitude of 3.5 mm. Each absorbance measurement is converted to a percentage of degradation according to the following formula: Dx(t) = 100(Aₓ(t) - A_{zero}(t))/(Aₜₒₜₐₗ(t) - A_{zero}(t)).

### Analysis of the fibrinolysis kinetics

In addition to real time monitoring and plotting of blood degradation kinetics, other parameters were derived from the same curves:
i) The activation time (Aₜ) required for the induction of fibrinolysis which is defined as the first value of t for which Dₓ(t)/dt > 1.
ii) The time required to obtain 50% lysis (T0.5) is defined as the first value of t which Dₓ(t) = 50%.
iii) The maximum clot lysis rate (CLRₘₐₓ) corresponds to the maximum value of Dₓ(t)/dt.

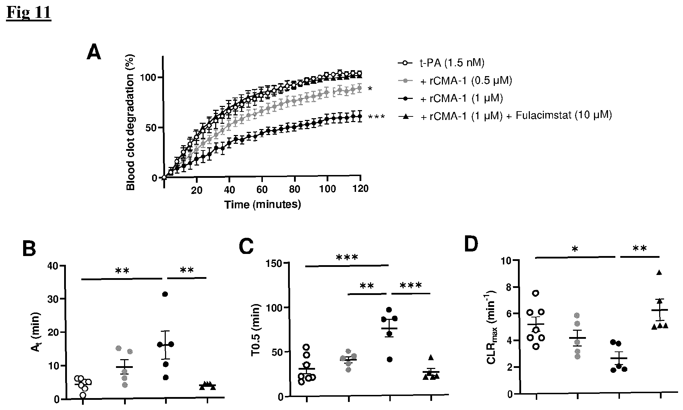

Fig 11- Plasmin-dependent fibrinolysis of human clots is reduced by human chymase (CMA-1) in a fulacimstat-sensitive fashion

### Results

As shown in Fig. 11, human chymase incorporated prior to CaCl₂-induced clotting, significantly reduces the tPA-induced fibrinolytic activity in human blood halo clots in a fulacimstat-sensitive fashion. Blood halos preincubated for 30 minutes with human chymase (0.5 µM and 1 µM) show rt-PA-dependent reduction in fibrinolysis in a concentration-dependent fashion and which is reduced by fulacimstat (Fig.11A). B) At: Fibrinolysis activation time; C) T0.5: time required to obtain 50% lysis; D) CLRmax: maximum clot lysis rate. Each point and bar correspond to the mean ± S.E.M (n = 5-7). (* P<0.05; ** P<0.01; *** P<0.001)

Normally, human chymase is inactivated rapidly in whole blood. However, when added to whole blood directly before initiation of the coagulation by recalcification, human chymase active in the thrombus and led to a concentration-dependent reduction in thrombus degradation (Fig.11A). The effect of chymase was reverted by the addition of fulacimstat, resulting in an accelerated clot resolution. These observations are complementary to the fulacimstat-dependent potentiation of plasmin activity found in mouse and hamster thrombi homogenates, as previously described in section 9.1.

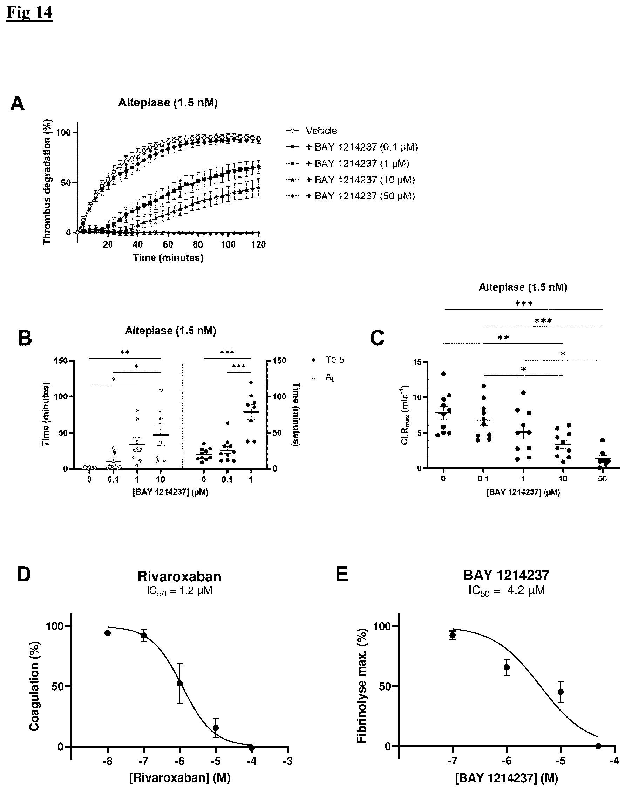

Fig. 14 A to C show that human blood halo clots require the activation of endogenous plasmin to be degraded. In additional control experiments, the roles of endogenous Factor Xa and plasmin in the induction or resolution of halo-shaped human blood clots in absence of rCMA-1 were investigated with rivaroxaban (Fig. 14 D) or the plasminogen/plasmin inhibitor, 10-Chloro-4-(piperidin-4-yl)pyrimido[1,2-b]indazol-2(1*H*)-one hydrochloride (Example 3 in WO2015067549; Fig 14 B, C and E) in concentration-response experiments. Each point illustrated the mean + SEM of at least 6 experiments. (* P<0.05, ** P<0.01, *** P<0.001.)

### Formation of halo-shaped blood clot with subsequently added human chymase

Clot formation based on the methods of Bonnard et al., (Sci Rep. 2017 May 24;7(1):2346) is induced by the addition of 31.2 mM of calcium chloride (CaCl2) to blood samples collected from healthy donors. 20 µl of CaCl2-supplemented blood is deposited in wells of a flat-bottomed, transparent 96-well plate, in circular motion around the edge of each well, allowing the formation of a halo-shaped blood clot (fluidic cohesion effect), leaving the center of the wells empty. The plate is subsequently sealed and incubated at 37°C for 30 min to allow solidification of the blood halo. Alteplase (1.5 nM) and recombinant human chymase (for a final concentration of 0.5 µM or 1 µM) are added simultaneously in presence or absence of 10 µM of fulacimstat (only for the latter concentration of rCMA-1) (Fig. 15 A). In another series of experiments, 10-Chloro-4-(piperidin-4-yl)pyrimido[1,2-b]indazol-2(1H)-one hydrochloride (Example 3 in WO2015067549) (0.1 µM or 1 µM) is co-administered with tPA in presence of the pre-formed halo. The plate is incubated for 2 hours at 37°C with lateral mixing (2 rpm), before measurement of the absorbance at 510 nm.

### Results

Normally, human chymase is inactivated rapidly in whole blood. As shown in Fig. 15 B, however, human chymase incorporated post-CaCl2-induced clotting, significantly reduces the tPA-induced fibrinolytic activity in human blood halo clots in a fulacimstat-sensitive fashion. Each point and bar correspond to the mean ± SEM of 6 to 13 experiments (*** P<0.001). The effect of chymase was reverted by the addition of fulacimstat, resulting in an accelerated clot resolution.

In a last series of control experiments, the plasminogen/plasmin inhibitor 10-Chloro-4-(piperidin-4-yl)pyrimido[1,2-b]indazol-2(1H)-one hydrochloride (Example 3 in WO2015067549)very significantly reduced the tPA-induced fibrinolysis of human blood halos (Fig 15 B).

### 10. Reducing chymase activity in vivo via gene knock-out or direct chymase inhibition has no impact on bleeding times in animal models

A very important liability of current strategies to resolve thrombi is the enhanced bleeding risk, which limits the doses of e.g. rtPA, which can be used, but even more limits the number of patients, who would be treated with such a fibrinolytic approach. Therefore, there is a clear need for a novel thrombus resolution strategy with no impact on hemostasis. To address the question whether chymase inhibition has an impact on hemostasis, bleeding time experiments with and without chymase inhibitors were conducted in mouse and hamster.

### 10.1 Impact of chymase inhibitors on the tail bleeding time in mouse

Mice were anesthetized with a mix of intramuscular (IM) ketamine/xylazine (87/13 mg/kg). 300 U/kg (via the jugular vein) of unfractionated heparin (as specified by the manufacturer) or 10 mg/kg of TY-51469 (IP) or 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) (PO) was injected 1 h prior to the test. DVT mice were evaluated 24 h after ligation, with or without a single dose of TY-51469 (10 mg/kg) 1 h post-ligation. They were then immobilized in a vertical position with their tail in a 2 mL tube filled with 1.5 mL Drabkin's reagent (Sigma Aldrich Canada Co., Oakville, ON, Canada) (Saito MS et al., Int. J. Exp. Pathol. 97(2016)285-292). The tail was cut 3 mm starting from the extremity. The time at which the bleeding stopped was noted. After 30 min, all animals were euthanized.

### Results

The results of the bleeding time experiments are depicted in Fig. 12A and summarized in Table 11, showing that chymase inhibition by 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) or TY-51469 had no impact on the bleeding time at doses, which were very efficacious in thrombus resolution. Even in mMCP-4 KO animals with a total lack of chymase no prolongation of bleeding times was observed. In contrast, heparin as a typical control compound in bleeding models led to a strong increase in bleeding time. Each bar represents the mean ± S.E.M of 6 mice. * P<0.05, ** P<0.01, *** P<0.001 compared to control-treated animals.

**Table 11 Impact of chymase inhibition on bleeding times in the tail bleeding model in mouse**

| | Ctrl | Fulacimstat 10 mg/kg | Fulacimstat 20 mg/kg | TY-51469 10 mg/kg | Heparin (UFH) 300 U/kg | mMCP-4 KO |
|---|---|---|---|---|---|---|
| N | 6 | 6 | 5 | 6 | 6 | 6 |
| Bleeding time (min) | 3.92 ± 0.62 | 3.95 ± 0.70 | 4.17 ± 0.43 | 4.08 ± 0.89 | 26.83 ± 0.75*** | 3.97 ± 0.49 |
| Bleeding time prolongation (xfold of Ctr) | 1.0 | 1.0 | 1.0 | 1.0 | 6.8 | 1.0 |

| | | | | | | |
|---|---|---|---|---|---|---|
| One-way ANOVA followed by Bonferroni's multiple comparisons test. *** P<0.001. Error type: Standard error of the mean (± SEM). | | | | | | |

Fig 12 - Fulacimstat or TY-51469 do not increase bleeding time in the mouse (A) or hamster (B)

### 10.2 Impact of the chymase inhibitor on bleeding time in a femoral vein puncture model in hamster

300 U/kg of unfractionated heparin (as specified by the manufacturer) or 10 mg/kg of fulacimstat was injected 1-hour prior to the test through the jugular vein or *per os,* respectively. The hamsters were anesthetized with a mix of intramuscular ketamine/xylazine (87-200/13 mg/kg). The femoral vein was exposed in the right hind limb. A small cut with a micro scissor to the mid femoral vein was made and excess blood was absorbed with filter paper without touching the incision site. Time before complete cessation of the hemorrhage was recorded as the bleeding time⁴³

### Results

The results of bleeding time measurements in hamsters are depicted in Fig. 12B and summarized in Table 12, showing that while heparin prolonged the bleeding time statistically significant, in hamsters treated with 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine -5-carboxylic acid (fulacimstat) no impact on bleeding time compared to control animals could be observed.

**Table 12 Impact of the chymase inhibitor fulacimstat on the bleeding time in the femoral vein puncture model in hamster**

| | Control | Fulacimstat 10 mg/kg | Heparin (UFH) 300 U/kg |
|---|---|---|---|
| N | 6 | 6 | 6 |
| Bleeding time (min) | 2.37 ± 0.46 | 2.52 ± 0.27 | 6.20 ± 0.20*** |
| Bleeding time prolongation (xfold of Ctr) | 1 | 1.06 | 2.62 |

| | | | |
|---|---|---|---|
| One-way ANOVA followed by Bonferroni's multiple comparisons test. *** P<0.001. Error type: Standard error of the mean (± SEM). | | | |

### Overall analysis of the results of the bleeding time experiments in mouse and hamster in context with the thrombus resolution results

In both bleeding time models in mouse and hamster no prolongation of the bleeding time was observed at doses of chymase inhibitors, which led to a strong thrombus resolution effect.

So, surprisingly, with this chymase inhibition approach rapid thrombus resolution is achieved without any signs of bleeding - making the chymase inhibition approach not only efficacious, but unexpectedly safe, as well.

The proposed mechanism of action is illustrated in Fig. 16.

Fig. 16 - A novel approach for safe thrombus resolution and vessel re-opening by targeting intra-thrombus chymase
In summary, in various parts of this invention it has been shown for the first time that chymase inhibitors inhibit the plasmin degradation by chymase, which has been identified for the first time as pathophysiological relevant process in animal and human thrombi with an impact on thrombus size in in vivo experiments. Inhibiting the degradation of plasmin via chymase inhibitors accelerates the lysis of fibrin clots in vessels without impacting bleeding time or hemostasis and thereby represents a novel approach for extraordinary safe revascularization of vessels occluded by thrombi or emboli. (experimental part, Fig. 16).

Chymase Inibitors of the present invention are therefore effective agents for the treatment and prophylaxis of stroke, pulmonary embolism, deep or superficial vein thrombosis, thrombotic microangiopathy, thrombotic microangiopathy in hypercoagulable states after infection, inflammation, transplantation, disseminated intravascular coagulation, vaccine-induced immune thrombotic thrombocytopenia, vascular access site thrombosis or occlusion.

### 11. Working examples of pharmaceutical compositions

The substances according to the invention can be converted to pharmaceutical preparations as follows:

### Tablet:

### Composition:

100 mg of the compound, 50 mg of lactose (monohydrate), 50 mg of maize starch, 10 mg of polyvinylpyrrolidone (PVP 25) (from BASF, Germany) and 2 mg of magnesium stearate.

Tablet weight 212 mg. Diameter 8 mm, radius of curvature 12 mm.

### Production:

The mixture of the compound of Example 1, lactose and starch is granulated with a 5% strength solution (m/m) of the PVP in water. After drying, the granules are mixed with the magnesium stearate for 5 min. This mixture is compressed in a conventional tabletting press (see above for format of the tablet).

### Oral suspension:

### Composition:

1000 mg of the compound of Example 1, 1000 mg of ethanol (96%), 400 mg of Rhodigel (xanthan gum) (from FMC, USA) and 99 g of water.

10 ml of oral suspension correspond to a single dose of 100 mg of the compound of the invention.

### Production:

The Rhodigel is suspended in ethanol, and the compound of Example 1 is added to the suspension. The water is added while stirring. The mixture is stirred for about 6 h until swelling of the Rhodigel is complete.

### Solution for oral administration:

### Composition:

500 mg of the inventive compound, 2.5 g of polysorbate and 97 g of polyethylene glycol 400. A single dose of 100 mg of the inventive compound corresponds to 20 g of oral solution.

### Production:

The inventive compound is suspended in the mixture of polyethylene glycol and polysorbate while stirring. The 15 stirring operation is continued until dissolution of the inventive compound is complete.

### i.v. Solution:

The inventive compound is dissolved in a concentration below the saturation solubility in a physiologically acceptable solvent (e.g. isotonic sodium chloride solution, glucose solution 5% and/or PEG 400 solution 30%). The solution is subjected to sterile filtration and dispensed into sterile and pyrogen-free injection vessels.

## Claims

1. Chymase Inhibitor for the use in the treatment and prophylaxis of acute ischemic stroke, transitory ischaemic attacks (TIA), ischemic strokes including cardioembolic strokes, strokes due to atrial fibrillation, non-cardioembolic strokes, lacunar stroke, strokes due to large or small artery diseases, strokes due to undetermined cause, cryptogenic strokes, embolic strokes, embolic strokes of undetermined source, events of thrombotic and/or thromboembolic origin leading to stroke or transitory ischaemic attacks, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion, wherein the chymase inhibitor is a compound of formula (I) 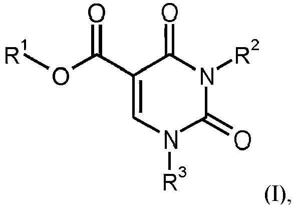 wherein
R¹ is hydrogen, methyl or ethyl,
R² is a group of the formula 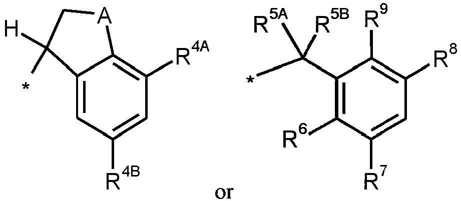 where
* is the attachment site to the uracil nitrogen atom,
A is -CH₂-, -CH₂-CH₂-, -O-CH₂-## or oxygen,
in which
## is the attachment site to the phenyl ring,
R^{4A} is hydrogen, fluorine, chlorine, trifluoromethyl or methyl,
R^{4B} is hydrogen, fluorine, chlorine, trifluoromethyl or methyl,
with the proviso that at least one of the R^{4A} and R^{4B} radicals is not hydrogen,
R^{5A} is hydrogen,
R^{5B} is hydrogen,
R⁶ is hydrogen,
R⁷ is hydrogen,
R⁸ is fluorine, chlorine, difluoromethyl, trifluoromethyl or methyl,
R⁹ is fluorine, chlorine, difluoromethyl, trifluoromethyl or methyl,
R³ is a group of the formula 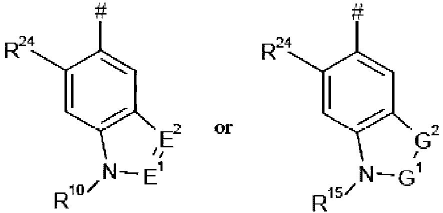 where
# is the attachment site to the uracil nitrogen atom,
E¹ is CR¹¹ or N,
in which
R¹¹ is hydrogen, (C₁-C₄)-alkyl, (C₃-C₇)-cycloalkyl or aminocarbonyl,
E² is CR¹² or N,
in which
R¹² is hydrogen, (C₁-C₄)-alkyl or (C₃-C₇)-cycloalkyl,
G¹ is C=O or SO₂,
G² is CR^{16A}R^{16B} NR¹⁷, O or S,
in which
R^{16A} is hydrogen, fluorine, (C₁-C₄)-alkyl or hydroxyl,
R^{16B} is hydrogen, fluorine, chlorine, (C₁-C₄)-alkyl or trifluoromethyl, or
R^{16A} and R^{16B} together with the carbon atom to which they are bonded form a 3- to 6-membered carbocycle,
R¹⁷ is hydrogen, (C₁-C₆)-alkyl, (C₃-C₇)-cycloalkyl or (C₁-C₄)-alkoxycarbonyl, in which (C₁-C₆)-alkyl may be substituted by 1 to 3 substituents independently selected from the group of fluorine, trifluoromethyl, cyano, (C₃-C₇)-cycloalkyl, hydroxyl, trifluoromethoxy, (C₁-C₄)-alkoxy, azetidinyl, oxetanyl, tetrahydrofuranyl and pyrrolidinyl,
R²⁴ is fluorine or methyl,
n is a number 0 or 1,
R¹⁰ is (C₁-C₄)-alkyl or (C₃-C₇)-cycloalkyl,
in which (C₁-C₄)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
R¹⁵ is hydrogen, (C₁-C₆)-alkyl or (C₃-C₇)-cycloalkyl,
in which (C₁-C₆)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl, and the salts, solvates and solvates of the salts thereof.

2. Chymase Inhibitor according to claim 1 for the use in the treatment and prophylaxis of acute ischemic stroke, transitory ischaemic attacks (TIA), ischemic strokes including cardioembolic strokes, strokes due to atrial fibrillation, non-cardioembolic strokes, lacunar stroke, strokes due to large or small artery diseases, strokes due to undetermined cause, cryptogenic strokes, embolic strokes, embolic strokes of undetermined source, events of thrombotic and/or thromboembolic origin leading to stroke or transitory ischaemic attacks, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion, wherein the chymase inhibitor is a compound of formula (I)
wherein
R¹ is hydrogen, methyl or ethyl,
R² is a group of the formula 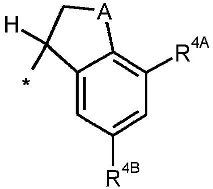 where
* is the attachment site to the uracil nitrogen atom,
A is -CH₂-,
R^{4A} is chlorine or trifluoromethyl,
R^{4B} is hydrogen,
R³ is a group of the formula 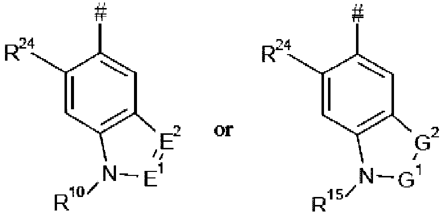 where
# is the attachment site to the uracil nitrogen atom,
E¹ is CR¹¹
in which
R¹¹ is hydrogen,
E² is N,
G¹ is C=O,
G² is CR^{16A}R^{16B} NR¹⁷, O or S,
in which
R^{16A} is hydrogen, fluorine, methyl or hydroxyl,
R^{16B} is hydrogen, fluorine, methyl or trifluoromethyl, or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof.

3. Chymase Inhibitor according to claim 2 for the use in the treatment and prophylaxis of acute ischemic stroke, transitory ischaemic attacks (TIA), ischemic strokes including cardioembolic strokes, strokes due to atrial fibrillation, non-cardioembolic strokes, lacunar stroke, strokes due to large or small artery diseases, strokes due to undetermined cause, cryptogenic strokes, embolic strokes, embolic strokes of undetermined source, events of thrombotic and/or thromboembolic origin leading to stroke or transitory ischaemic attacks, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion, wherein the chymase inhibitor is a compound of formula (I)
wherein
R¹ is hydrogen,
R² is a group of the formula 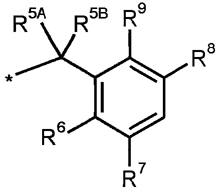 where
* is the attachment site to the uracil nitrogen atom,
R^{5A} is hydrogen,
R^{5B} is hydrogen,
R⁶ is hydrogen,
R⁷ is hydrogen,
R⁸ is fluorine, chlorine or trifluoromethyl,
R⁹ is fluorine, chlorine, trifluoromethyl or methyl,
R³ is a group of the formula 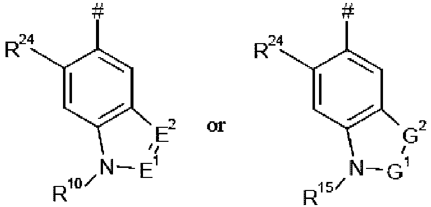 where
# is the attachment site to the uracil nitrogen atom,
E¹ is CR¹¹
in which
R¹¹ is hydrogen,
E² is N,
G¹ is C=O,
G² is CR^{16A}R^{16B} NR¹⁷, O or S,
in which
R^{16A} is hydrogen, fluorine, methyl or hydroxyl,
R^{16B} is hydrogen, fluorine, methyl or trifluoromethyl, or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof

4. Chymase Inhibitor according to claim 1 or 2 for the use in the treatment and prophylaxis of acute ischemic stroke, transitory ischaemic attacks (TIA), ischemic strokes including cardioembolic strokes, strokes due to atrial fibrillation, non-cardioembolic strokes, lacunar stroke, strokes due to large or small artery diseases, strokes due to undetermined cause, cryptogenic strokes, embolic strokes, embolic strokes of undetermined source, events of thrombotic and/or thromboembolic origin leading to stroke or transitory ischaemic attacks, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion, wherein the chymase inhibitor is a compound of formula (I), selected from the group of 1-(1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(6-fluoro-1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 2,4-dioxo-3-[(1R)-4-(trifluoromethyl)-2,3-dihydro-1H-inden-1-yl]-1-(1,3,3-trimethyl-2-oxo-2,3-dihydro-1H-indol-5-yl)-1,2,3,4-tetrahydropyrimidine-5-carboxylic acid (R enantiomer), 1-(1'-methyl-2'-oxo-1',2'-dihydrospiro-[cyclopropane-1,3'-indole]-5'-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer), 1-(3-methyl-2-oxo-2,3-dihydro-1,3-benzothiazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and ethyl 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylate (R enantiomer).

5. Chymase Inhibitor according to claim 1, 2 or 4 for the use in the treatment and prophylaxis of acute ischemic stroke, transitory ischaemic attacks (TIA), ischemic strokes including cardioembolic strokes, strokes due to atrial fibrillation, non-cardioembolic strokes, lacunar stroke, strokes due to large or small artery diseases, strokes due to undetermined cause, cryptogenic strokes, embolic strokes, embolic strokes of undetermined source, events of thrombotic and/or thromboembolic origin leading to stroke or transitory ischaemic attacks, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion, wherein the chymase inhibitor of formula (I) is 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) of the formula

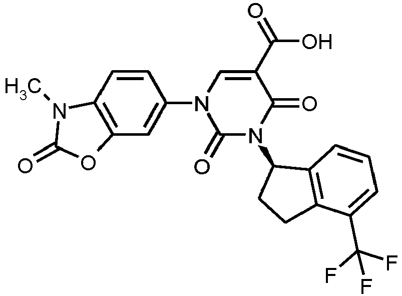

6. Combination of one or more compounds of the formula (I) with one or more other active compounds for use according to any of claims 1 to 5.

7. Pharmaceutical composition comprising at least one compound of the formula (I) according to any of claims 1 to 6 in combination with one or more inert non-toxic pharmaceutically suitable excipients for use according to any of claims 1 to 5.

8. Chymase Inhibitor according to claim 1 to 5 for use in a method for the treatment and/or prophylaxis of acute ischemic stroke, transitory ischaemic attacks (TIA), ischemic strokes including cardioembolic strokes, such as strokes due to atrial fibrillation, non-cardioembolic strokes, such as lacunar stroke, strokes due to large or small artery diseases, or strokes due to undetermined cause, cryptogenic strokes, embolic strokes, embolic strokes of undetermined source, or events of thrombotic and/or thromboembolic origin leading to stroke or transitory ischaemic attacks TIA, pulmonary embolism, thrombotic microangiopathy, thrombotic microangiopathy in hypercoagulable states after infection, disseminated intravascular coagulation, vaccine-induced immune thrombotic thrombocytopenia, vascular access site thrombosis or occlusion, by administering systemically and/or locally a therapeutically effective amount of at least one compound according to any of Claims 1 to 5 or a medicament comprising at least one compound according to any of Claims 1 to 5 in combination with an inert, non-toxic, pharmaceutically acceptable additive.

9. Chymase Inhibitor according to claim 1 to 5 for use in a method according to Claim 8 wherein the medicament further comprises at least one further active compound selected from the group consisting of anticoagulant and/or antiplatelet drugs.

10. A medicament, comprising a compound of the formula (I) for use according to any of Claims 1 to 5 in combination with one or more further active ingredients selected from the group consisting of anticoagulant and/or antiplatelet drugs.

## Patentansprüche

1. Chymase-Inhibitor zur Verwendung bei der Behandlung und Prophylaxe des akuten ischämischen Schlaganfalls, transitorischer ischämischer Attacken (TIA), ischämischer Schlaganfälle einschließlich kardioembolischer Schlaganfälle, Schlaganfälle aufgrund von Vorhofflimmern, nicht-kardioembolischer Schlaganfälle, lakunärer Schlaganfälle, Schlaganfälle aufgrund von Erkrankungen großer oder kleiner Arterien, Schlaganfälle unklarer Ursache, kryptogener Schlaganfälle, embolischer Schlaganfälle, embolischer Schlaganfälle unklarer Ursache, Ereignissen thrombotischen und/oder thromboembolischen Ursprungs, die zu Schlaganfall oder transitorischen ischämischen Attacken führen, Lungenembolie, thrombotischer Mikroangiopathie, thrombotischer Mikroangiopathie in hyperkoagulierbaren Zuständen nach Infektion, disseminierter intravasaler Gerinnung, impfstoffinduzierter immunthrombotischer Thrombozytopenie, Thrombose oder Okklusion an Gefäßzugangsstellen, wobei der Chymase-Inhibitor eine Verbindung der Formel (I) ist 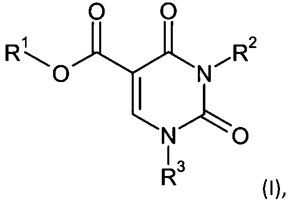 wobei
R¹ Wasserstoff, Methyl oder Ethyl ist,
R² eine Gruppe der Formel 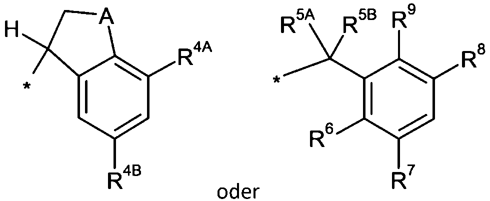 ist
wobei
* die Bindungsstelle zum Uracil-Stickstoffatom ist,
A -CH¹⁹-, -CH¹⁹-CH¹⁹-, -O-CH¹⁹-## oder Sauerstoff ist,
wobei
## die Bindungsstelle zum Phenylring ist,
R^{4A} Wasserstoff, Fluor, Chlor, Trifluormethyl oder Methyl ist,
R^{4B} Wasserstoff, Fluor, Chlor, Trifluormethyl oder Methyl ist,
mit der Maßgabe, dass mindestens einer der R^{4A}- und R^{4B}-Reste nicht Wasserstoff ist,
R^{5A} Wasserstoff ist,
R^{5B} Wasserstoff ist,
R⁶ Wasserstoff ist,
R⁷ Wasserstoff ist,
R⁸ Fluor, Chlor, Difluormethyl, Trifluormethyl oder Methyl ist,
R⁹ Fluor, Chlor, Difluormethyl, Trifluormethyl oder Methyl ist,
R³ eine Gruppe der Formel 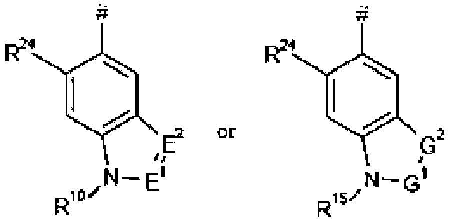
wobei
# die Bindungsstelle zum Uracil-Stickstoffatom ist,
E¹ CR¹⁰ oder N ist,
wobei
R¹¹ Wasserstoff, (C⁵-C⁶)-Alkyl, (C⁷-C⁸)-Cycloalkyl oder Aminocarbonyl ist,
E² CR¹¹ oder N ist,
wobei
R¹² Wasserstoff, (C⁵-C⁶)-Alkyl oder (C⁷-C⁸)-Cycloalkyl ist,
G¹ C=O oder SO¹⁹ ist,
G² CR^{16A}R^{16B}, NR¹², O oder S ist,
wobei
R^{16A} Wasserstoff, Fluor, (C⁵-C⁶)-Alkyl oder Hydroxyl ist,
R^{16B} Wasserstoff, Fluor, Chlor, (C⁵-C⁶)-Alkyl oder Trifluormethyl ist, oder
R ^{16A} und R^{16B} zusammen mit dem Kohlenstoffatom, an das sie gebunden sind, einen 3- bis 6-gliedrigen Carbocyclus bilden,
R¹⁷ Wasserstoff, (C⁵-C⁹)-Alkyl, (C⁷-C³)-Cycloalkyl oder (C⁵-C⁶)-Alkoxycarbonyl ist,
wobei (C⁵-C⁹)-Alkyl durch 1 bis 3 Substituenten substituiert sein kann, die unabhängig voneinander aus der Gruppe Fluor, Trifluormethyl, Cyano, (C⁷-C³)-Cycloalkyl, Hydroxyl, Trifluormethoxy, (C⁵-C⁶)-Alkoxy, Azetidinyl, Oxetanyl, Tetrahydrofuranyl und Pyrrolidinyl ausgewählt sind,
R²⁴ Fluor oder Methyl ist,
n eine Zahl 0 oder 1 ist,
R¹⁰ (C⁵-C⁶)-Alkyl oder (C⁷-C³)-Cycloalkyl ist,
wobei (C⁵-C⁶)-Alkyl durch 1 oder 2 Substituenten substituiert sein kann, die unabhängig voneinander aus der Gruppe Fluor, Trifluormethyl, Cyclopropyl, Cyclobutyl, Hydroxyl, Methoxy, Ethoxy, Azetidinyl, Oxetanyl, Tetrahydrofuranyl und Pyrrolidinyl ausgewählt sind,
R¹⁵ Wasserstoff, (C⁵-C⁹)-Alkyl oder (C⁷-C⁸)-Cycloalkyl ist,
wobei (C⁵-C⁹)-Alkyl durch 1 oder 2 Substituenten substituiert sein kann, die unabhängig voneinander aus der Gruppe Fluor, Trifluormethyl, Cyclopropyl, Cyclobutyl, Hydroxyl, Methoxy, Ethoxy, Azetidinyl, Oxetanyl, Tetrahydrofuranyl und Pyrrolidinyl ausgewählt sind,
und deren Salze, Solvate und Solvate der Salze.

2. Chymase-Inhibitor nach Anspruch 1 zur Verwendung bei der Behandlung und Prophylaxe des akuten ischämischen Schlaganfalls, transitorischer ischämischer Attacken (TIA), ischämischer Schlaganfälle einschließlich kardioembolischer Schlaganfälle, Schlaganfälle aufgrund von Vorhofflimmern, nicht-kardioembolischer Schlaganfälle, lakunärer Schlaganfälle, Schlaganfälle aufgrund von Erkrankungen großer oder kleiner Arterien, Schlaganfälle unklarer Ursache, kryptogener Schlaganfälle, embolischer Schlaganfälle, embolischer Schlaganfälle unklarer Ursache, Ereignissen thrombotischen und/oder thromboembolischen Ursprungs, die zu Schlaganfall oder transitorischen ischämischen Attacken führen, Lungenembolie, thrombotischer Mikroangiopathie, thrombotischer Mikroangiopathie in hyperkoagulierbaren Zuständen nach Infektion, disseminierter intravasaler Gerinnung, impfstoffinduzierter immunthrombotischer Thrombozytopenie, Thrombose oder Okklusion an Gefäßzugangsstellen, wobei der Chymase-Inhibitor eine Verbindung der Formel (I) ist wobei
R¹ Wasserstoff, Methyl oder Ethyl ist,
R² eine Gruppe der Formel 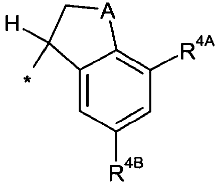
wobei
* die Bindungsstelle zum Uracil-Stickstoffatom ist,
A -CH¹⁹- ist,
R^{4A} Chlor oder Trifluormethyl ist,
R^{4B} Wasserstoff ist,
R³ eine Gruppe der Formel 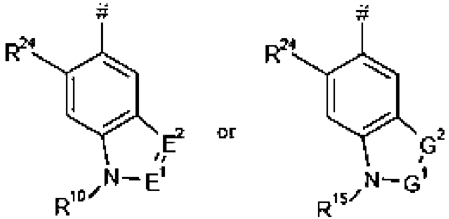
wobei
# die Bindungsstelle zum Uracil-Stickstoffatom ist,
E¹ CR¹⁰ ist
wobei
R¹¹ Wasserstoff ist,
E² N ist,
G¹ C=O ist,
G² CR^{16A}R^{16B}, NR¹², O oder S ist,
wobei
R^{16A} Wasserstoff, Fluor, Methyl oder Hydroxyl ist,
R^{16B} Wasserstoff, Fluor, Methyl oder Trifluormethyl ist,
oder
R ^{16A} und R^{16B} zusammen mit dem Kohlenstoffatom, an das sie gebunden sind, einen Cyclopropylring bilden,
R¹⁷ Wasserstoff, (C⁵-C⁶)-Alkyl oder (C⁷-C³)-Cycloalkyl ist,
wobei (C⁵-C⁶)-Alkyl durch 1 bis 3 Substituenten substituiert sein kann, die unabhängig voneinander aus der Gruppe Fluor, Trifluormethyl, Cyano, Cyclopropyl, Cyclobutyl, Hydroxyl, Trifluormethoxy, Methoxy, Ethoxy, Azetidinyl, Oxetanyl, Tetrahydrofuranyl und Pyrrolidinyl ausgewählt sind,
R²⁴ Wasserstoff oder Fluor ist,
R¹⁰ (C⁵-C⁶)-Alkyl ist,
R¹⁵ Wasserstoff, Methyl oder Ethyl ist,
wobei Methyl und Ethyl durch 1 Substituenten substituiert sein können, der aus der Gruppe Fluor, Trifluormethyl und Cyclopropyl ausgewählt ist,
und deren Salze, Solvate und Solvate der Salze.

3. Chymase-Inhibitor nach Anspruch 2 zur Verwendung bei der Behandlung und Prophylaxe des akuten ischämischen Schlaganfalls, transitorischer ischämischer Attacken (TIA), ischämischer Schlaganfälle einschließlich kardioembolischer Schlaganfälle, Schlaganfälle aufgrund von Vorhofflimmern, nicht-kardioembolischer Schlaganfälle, lakunärer Schlaganfälle, Schlaganfälle aufgrund von Erkrankungen großer oder kleiner Arterien, Schlaganfälle unklarer Ursache, kryptogener Schlaganfälle, embolischer Schlaganfälle, embolischer Schlaganfälle unklarer Ursache, Ereignissen thrombotischen und/oder thromboembolischen Ursprungs, die zu Schlaganfall oder transitorischen ischämischen Attacken führen, Lungenembolie, thrombotischer Mikroangiopathie, thrombotischer Mikroangiopathie in hyperkoagulierbaren Zuständen nach Infektion, disseminierter intravasaler Gerinnung, impfstoffinduzierter immunthrombotischer Thrombozytopenie, Thrombose oder Okklusion an Gefäßzugangsstellen, wobei der Chymase-Inhibitor eine Verbindung der Formel (I) ist wobei
R¹ Wasserstoff ist,
R² eine Gruppe der Formel 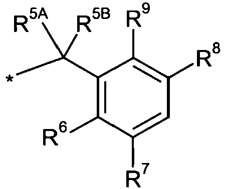 wobei
* die Bindungsstelle zum Uracil-Stickstoffatom ist,
R^{5A} Wasserstoff ist,
R^{5B} Wasserstoff ist,
R⁶ Wasserstoff ist,
R⁷ Wasserstoff ist,
R⁸ Fluor, Chlor oder Trifluormethyl ist,
R⁹ Fluor, Chlor, Trifluormethyl oder Methyl ist,
R³ eine Gruppe der Formel 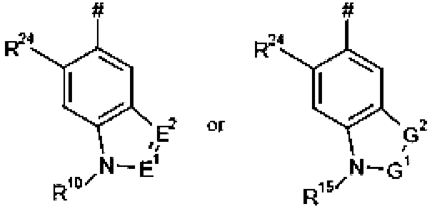 wobei
# die Bindungsstelle zum Uracil-Stickstoffatom ist,
E¹ CR¹⁰ ist
wobei
R¹¹ Wasserstoff ist,
E² N ist,
G¹ C=O ist,
G² CR^{16A}R^{16B}, NR¹², O oder S ist,
wobei
R^{16A} Wasserstoff, Fluor, Methyl oder Hydroxyl ist,
R^{16B} Wasserstoff, Fluor, Methyl oder Trifluormethyl ist,
oder
R ^{16A} und R^{16B} zusammen mit dem Kohlenstoffatom, an das sie gebunden sind, einen Cyclopropylring bilden,
R¹⁷ Wasserstoff, (C⁵-C⁶)-Alkyl oder (C⁷-C³)-Cycloalkyl ist,
wobei (C⁵-C⁶)-Alkyl durch 1 bis 3 Substituenten substituiert sein kann, die unabhängig voneinander aus der Gruppe Fluor, Trifluormethyl, Cyano, Cyclopropyl, Cyclobutyl, Hydroxyl, Trifluormethoxy, Methoxy, Ethoxy, Azetidinyl, Oxetanyl, Tetrahydrofuranyl und Pyrrolidinyl ausgewählt sind,
R²⁴ Wasserstoff oder Fluor ist,
R¹⁰ (C⁵-C⁶)-Alkyl ist,
R¹⁵ Wasserstoff, Methyl oder Ethyl ist,
wobei Methyl und Ethyl durch 1 Substituenten substituiert sein können, der aus der Gruppe Fluor, Trifluormethyl und Cyclopropyl ausgewählt ist,
und deren Salze, Solvate und Solvate der Salze

4. Chymase-Inhibitor nach Anspruch 1 oder 2 zur Verwendung bei der Behandlung und Prophylaxe des akuten ischämischen Schlaganfalls, transitorischer ischämischer Attacken (TIA), ischämischer Schlaganfälle einschließlich kardioembolischer Schlaganfälle, Schlaganfälle aufgrund von Vorhofflimmern, nicht-kardioembolischer Schlaganfälle, lakunärer Schlaganfall, Schlaganfälle aufgrund von Erkrankungen großer oder kleiner Arterien, Schlaganfälle unklarer Ursache, kryptogener Schlaganfälle, embolischer Schlaganfälle, embolischer Schlaganfälle unklarer Ursache, Ereignissen thrombotischen und/oder thromboembolischen Ursprungs, die zu Schlaganfall oder transitorischen ischämischen Attacken führen, Lungenembolie, thrombotischer Mikroangiopathie, thrombotischer Mikroangiopathie in hyperkoagulierbaren Zuständen nach Infektion, disseminierter intravasaler Gerinnung, impfstoffinduzierter immunthrombotischer Thrombozytopenie, Thrombose oder Okklusion an Gefäßzugangsstellen, wobei der Chymase-Inhibitor eine Verbindung der Formel (I) ist, ausgewählt aus der Gruppe bestehend aus 1-(1,3-Dimethyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carbonsäure (R Enantiomer), 1-(6-Fluor-1,3-dimethyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carbonsäure (R Enantiomer), 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure (R Enantiomer), 2,4-Dioxo-3-[(1R)-4-(trifluormethyl)-2,3-dihydro-1H-inden-1-yl]-1-(1,3,3-trimethyl-2-oxo-2,3-dihydro-1H-indol-5-yl)-1,2,3,4-tetrahydropyrimidin-5-carbonsäure (R Enantiomer), 1-(1'-Methyl-2'-oxo-1',2'-dihydrospiro[cyclopropan-1,3'-indol]-5'-yl)-2,4-dioxo-3-[(1R)-4-(trifluormethyl)-2,3-dihydro-1H-inden-1-yl]-1,2,3,4-tetrahydropyrimidin-5-carbonsäure (R Enantiomer), 1-(3-Methyl-2-oxo-2,3-dihydro-1,3-benzothiazol-6-yl)-2,4-dioxo-3-[(1R)-4-(trifluormethyl)-2,3-dihydro-1H-inden-1-yl]-1,2,3,4-tetrahydropyrimidin-5-carbonsäure (R Enantiomer) und Ethyl 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carboxylat (R Enantiomer).

5. Chymase-Inhibitor nach Anspruch 1, 2 oder 4 zur Verwendung bei der Behandlung und Prophylaxe des akuten ischämischen Schlaganfalls, transitorischer ischämischer Attacken (TIA), ischämischer Schlaganfälle einschließlich kardioembolischer Schlaganfälle, Schlaganfälle aufgrund von Vorhofflimmern, nicht-kardioembolischer Schlaganfälle, lakunärer Schlaganfall, Schlaganfälle aufgrund von Erkrankungen großer oder kleiner Arterien, Schlaganfälle unklarer Ursache, kryptogener Schlaganfälle, embolischer Schlaganfälle, embolischer Schlaganfälle unklarer Ursache, Ereignissen thrombotischen und/oder thromboembolischen Ursprungs, die zu Schlaganfall oder transitorischen ischämischen Attacken führen, Lungenembolie, thrombotischer Mikroangiopathie, thrombotischer Mikroangiopathie in hyperkoagulierbaren Zuständen nach Infektion, disseminierter intravasaler Gerinnung, impfstoffinduzierter immunthrombotischer Thrombozytopenie, Thrombose oder Okklusion an Gefäßzugangsstellen, wobei der Chymase-Inhibitor der Formel (I) 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure (R Enantiomer) der Formel ist

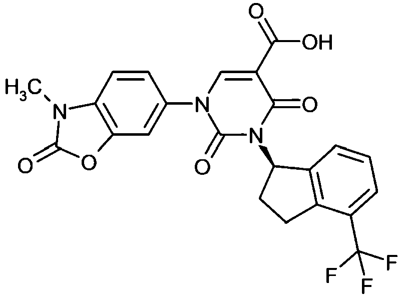

6. Kombination einer oder mehrerer Verbindungen der Formel (I) mit einer oder mehreren anderen Wirkverbindungen zur Verwendung gemäß einem der Ansprüche 1 bis 5.

7. Pharmazeutische Zusammensetzung umfassend mindestens eine Verbindung der Formel (I) gemäß einem der Ansprüche 1 bis 6 in Kombination mit einem oder mehreren inerten, nicht toxischen, pharmazeutisch geeigneten Hilfsstoffen zur Verwendung gemäß einem der Ansprüche 1 bis 5.

8. Chymase-Inhibitor nach Anspruch 1 bis 5 zur Verwendung in einem Verfahren zur Behandlung und/oder Prophylaxe des akuten ischämischen Schlaganfalls, transitorischer ischämischer Attacken (TIA), ischämischer Schlaganfälle einschließlich kardioembolischer Schlaganfälle, wie Schlaganfälle aufgrund von Vorhofflimmern, nicht-kardioembolischer Schlaganfälle, wie lakunärer Schlaganfall, Schlaganfälle aufgrund von Erkrankungen großer oder kleiner Arterien, oder Schlaganfälle unklarer Ursache, kryptogener Schlaganfälle, embolischer Schlaganfälle, embolischer Schlaganfälle unklarer Ursache, oder Ereignissen thrombotischen und/oder thromboembolischen Ursprungs, die zu Schlaganfall oder transitorischen ischämischen Attacken TIA führen, Lungenembolie, thrombotischer Mikroangiopathie, thrombotischer Mikroangiopathie in hyperkoagulierbaren Zuständen nach Infektion, disseminierter intravasaler Gerinnung, impfstoffinduzierter immunthrombotischer Thrombozytopenie, Thrombose oder Okklusion an Gefäßzugangsstellen, durch systemisches und/oder lokales Verabreichen einer therapeutisch wirksamen Menge von mindestens einer Verbindung gemäß einem der Ansprüche 1 bis 5 oder eines Arzneimittels umfassend mindestens eine Verbindung gemäß einem der Ansprüche 1 bis 5 in Kombination mit einem inerten, nicht toxischen, pharmazeutisch akzeptablen Zusatzstoff.

9. Chymase-Inhibitor nach Anspruch 1 bis 5 zur Verwendung in einem Verfahren nach Anspruch 8, wobei das Arzneimittel weiterhin mindestens eine weitere Wirkverbindung ausgewählt aus der Gruppe bestehend aus Antikoagulanzien und/oder Thrombozytenaggregationshemmern umfasst.

10. Arzneimittel, umfassend eine Verbindung der Formel (I) zur Verwendung gemäß einem der Ansprüche 1 bis 5 in Kombination mit einem oder mehreren weiteren Wirkstoffen ausgewählt aus der Gruppe bestehend aus Antikoagulanzien und/oder Thrombozytenaggregationshemmern.

## Revendications

1. Inhibiteur de chymase pour l'utilisation dans le traitement et la prophylaxie de l'accident vasculaire cérébral ischémique aigu, des attaques ischémiques transitoires (AIT), des accidents vasculaires cérébraux ischémiques, y compris les accidents vasculaires cérébraux cardioemboliques, les accidents vasculaires cérébraux dus à la fibrillation auriculaire, les accidents vasculaires cérébraux non cardioemboliques, les accidents vasculaires cérébraux lacunaires, les accidents vasculaires cérébraux dus à des maladies des grandes ou petites artères, les accidents vasculaires cérébraux de cause indéterminée, les accidents vasculaires cérébraux cryptogéniques, les accidents vasculaires cérébraux emboliques, les accidents vasculaires cérébraux emboliques de source indéterminée, les événements d'origine thrombotique et/ou thromboembolique conduisant à un accident vasculaire cérébral ou à des attaques ischémiques transitoires, l'embolie pulmonaire, la microangiopathie thrombotique, la microangiopathie thrombotique dans des états hypercoagulables après infection, la coagulation intravasculaire disséminée, la thrombocytopénie thrombotique immunitaire induite par le vaccin, la thrombose ou l'occlusion du site d'accès vasculaire, dans lequel l'inhibiteur de chymase est un composé de formule (I) 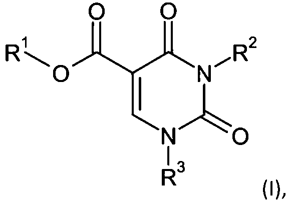 dans lequel
R¹ est hydrogène, méthyle ou éthyle,
R² est un groupe de la formule 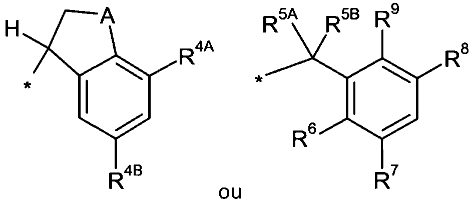 où
* est le site de fixation à l'atome d'azote de l'uracile,
A est -CH₂-, -CH₂-CH₂-, -O-CH₂-## ou oxygène,
dans lequel
## est le site de fixation à l'anneau phényle,
R^{4A} est hydrogène, fluor, chlore, trifluorométhyle ou méthyle,
R^{4B} est hydrogène, fluor, chlore, trifluorométhyle ou méthyle,
à condition qu'au moins l'un des radicaux R^{4A} et R^{4B} ne soit pas hydrogène,
R^{5A} est hydrogène,
R^{5B} est hydrogène,
R⁶ est hydrogène,
R⁷ est hydrogène,
R⁸ est fluor, chlore, difluorométhyle, trifluorométhyle ou méthyle,
R⁹ est fluor, chlore, difluorométhyle, trifluorométhyle ou méthyle,
R³ est un groupe de la formule 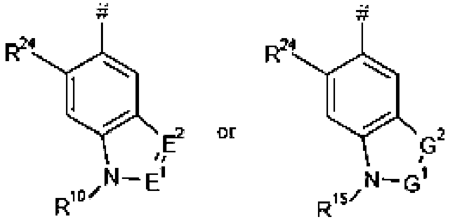 où
# est le site de fixation à l'atome d'azote de l'uracile,
E¹ est CR¹¹ ou N,
dans lequel
R¹¹ est hydrogène, (C₁-C₄)-alkyle, (C₃-C₇)-cycloalkyle ou aminocarbonyle,
E² est CR¹² ou N,
dans lequel
R¹² est hydrogène, (C₁-C₄)-alkyle ou (C₃-C₇)-cycloalkyle,
G¹ est C=O ou SO₂,
G² est CR^{16A}R^{16B}, NR¹⁷, O ou S,
dans lequel
R^{16A} est hydrogène, fluor, (C₁-C₄)-alkyle ou hydroxyle,
R^{16B} est hydrogène, fluor, chlore, (C₁-C₄)-alkyle ou trifluorométhyle,
ou
R ^{16A} et R^{16B} ensemble avec l'atome de carbone auquel ils sont liés forment un carbocycle à 3 à 6 membres,
R¹⁷ est hydrogène, (C₁-C₆)-alkyle, (C₃-C₇)-cycloalkyle ou (C₁-C₄)-alkoxycarbonyle,
dans lequel (C₁-C₆)-alkyle peut être substitué par 1 à 3 substituants sélectionnés indépendamment du groupe de fluor, trifluorométhyle, cyano, (C₃-C₇)-cycloalkyle, hydroxyle, trifluorométhoxy, (C₁-C₄)-alkoxy, azétidinyle, oxétanyl, tétrahydrofuranyl et pyrrolidinyle,
R²⁴ est fluor ou méthyle,
n est un nombre 0 ou 1,
R¹⁰ est (C₁-C₄)-alkyle ou (C₃-C₇)-cycloalkyle, dans lequel (C₁-C₄)-alkyle peut être substitué par 1 ou 2 substituants sélectionnés indépendamment du groupe de fluor, trifluorométhyle, cyclopropyle, cyclobutyle, hydroxyle, méthoxy, éthoxy, azétidinyle, oxétanyl, tétrahydrofuranyl et pyrrolidinyle,
R¹⁵ est hydrogène, (C₁-C₆)-alkyle ou (C₃-C₇)-cycloalkyle,
dans lequel (C₁-C₆)-alkyle peut être substitué par 1 ou 2 substituants sélectionnés indépendamment du groupe de fluor, trifluorométhyle, cyclopropyle, cyclobutyle, hydroxyle, méthoxy, éthoxy, azétidinyle, oxétanyl, tétrahydrofuranyl et pyrrolidinyle,
et les sels, solvates et solvates des sels de ceux-ci.

2. Inhibiteur de chymase selon la revendication 1 pour l'utilisation dans le traitement et la prophylaxie de l'accident vasculaire cérébral ischémique aigu, des attaques ischémiques transitoires (AIT), des accidents vasculaires cérébraux ischémiques, y compris les accidents vasculaires cérébraux cardioemboliques, les accidents vasculaires cérébraux dus à la fibrillation auriculaire, les accidents vasculaires cérébraux non cardioemboliques, les accidents vasculaires cérébraux lacunaires, les accidents vasculaires cérébraux dus à des maladies des grandes ou petites artères, les accidents vasculaires cérébraux de cause indéterminée, les accidents vasculaires cérébraux cryptogéniques, les accidents vasculaires cérébraux emboliques, les accidents vasculaires cérébraux emboliques de source indéterminée, les événements d'origine thrombotique et/ou thromboembolique conduisant à un accident vasculaire cérébral ou à des attaques ischémiques transitoires, l'embolie pulmonaire, la microangiopathie thrombotique, la microangiopathie thrombotique dans des états hypercoagulables après infection, la coagulation intravasculaire disséminée, la thrombocytopénie thrombotique immunitaire induite par le vaccin, la thrombose ou l'occlusion du site d'accès vasculaire, dans lequel l'inhibiteur de chymase est un composé de formule (I) dans lequel
R¹ est hydrogène, méthyle ou éthyle,
R² est un groupe de la formule 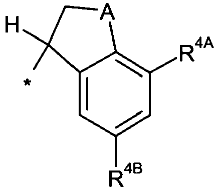 où
* est le site de fixation à l'atome d'azote de l'uracile,
A est -CH₂-,
R^{4A} est chlore ou trifluorométhyle,
R^{4B} est hydrogène,
R³ est un groupe de la formule 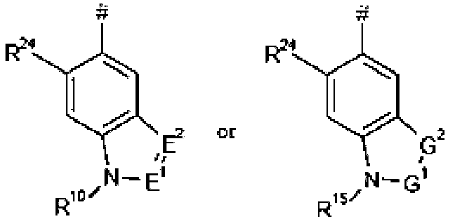 où
# est le site de fixation à l'atome d'azote de l'uracile,
E¹ est CR¹¹
dans lequel
R¹¹ est hydrogène,
E² est N,
G¹ est C=O,
G² est CR^{16A}R^{16B}, NR¹⁷, O ou S,
dans lequel
R^{16A} est hydrogène, fluor, méthyle ou hydroxyle,
R^{16B} est hydrogène, fluor, méthyle ou trifluorométhyle,
ou
R ^{16A} et R^{16B} ensemble avec l'atome de carbone auquel ils sont liés forment un anneau cyclopropyle,
R¹⁷ est hydrogène, (C₁-C₄)-alkyle ou (C₃-C₅)-cycloalkyle,
dans lequel (C₁-C₄)-alkyle peut être substitué par 1 à 3 substituants sélectionnés indépendamment du groupe de fluor, trifluorométhyle, cyano, cyclopropyle, cyclobutyle, hydroxyle, trifluorométhoxy, méthoxy, éthoxy, azétidinyle, oxétanyl, tétrahydrofuranyl et pyrrolidinyle,
R²⁴ est hydrogène ou fluor,
R¹⁰ est (C₁-C₄)-alkyle,
R¹⁵ est hydrogène, méthyle ou éthyle,
dans lequel méthyle et éthyle peuvent être substitués par 1 substituant sélectionné du groupe de fluor, trifluorométhyle et cyclopropyle,
et les sels, solvates et solvates des sels de ceux-ci.

3. Inhibiteur de chymase selon la revendication 2 pour l'utilisation dans le traitement et la prophylaxie de l'accident vasculaire cérébral ischémique aigu, des attaques ischémiques transitoires (AIT), des accidents vasculaires cérébraux ischémiques, y compris les accidents vasculaires cérébraux cardioemboliques, les accidents vasculaires cérébraux dus à la fibrillation auriculaire, les accidents vasculaires cérébraux non cardioemboliques, les accidents vasculaires cérébraux lacunaires, les accidents vasculaires cérébraux dus à des maladies des grandes ou petites artères, les accidents vasculaires cérébraux de cause indéterminée, les accidents vasculaires cérébraux cryptogéniques, les accidents vasculaires cérébraux emboliques, les accidents vasculaires cérébraux emboliques de source indéterminée, les événements d'origine thrombotique et/ou thromboembolique conduisant à un accident vasculaire cérébral ou à des attaques ischémiques transitoires, l'embolie pulmonaire, la microangiopathie thrombotique, la microangiopathie thrombotique dans des états hypercoagulables après infection, la coagulation intravasculaire disséminée, la thrombocytopénie thrombotique immunitaire induite par le vaccin, la thrombose ou l'occlusion du site d'accès vasculaire, dans lequel l'inhibiteur de chymase est un composé de formule (I) dans lequel
R¹ est hydrogène,
R² est un groupe de la formule 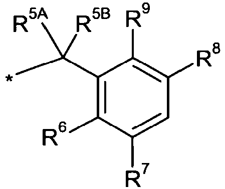 où
* est le site de fixation à l'atome d'azote de l'uracile,
R^{5A} est hydrogène,
R^{5B} est hydrogène,
R⁶ est hydrogène,
R⁷ est hydrogène,
R⁸ est fluor, chlore ou trifluorométhyle,
R⁹ est fluor, chlore, trifluorométhyle ou méthyle,
R³ est un groupe de la formule 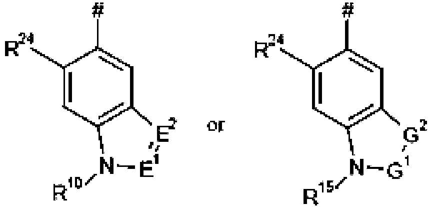 où
# est le site de fixation à l'atome d'azote de l'uracile,
E¹ est CR¹¹
dans lequel
R¹¹ est hydrogène,
E² est N,
G¹ est C=O,
G² est CR^{16A}R^{16B}, NR¹⁷, O ou S,
dans lequel
R^{16A} est hydrogène, fluor, méthyle ou hydroxyle,
R^{16B} est hydrogène, fluor, méthyle ou trifluorométhyle,
ou
R ^{16A} et R^{16B} ensemble avec l'atome de carbone auquel ils sont liés forment un anneau cyclopropyle,
R¹⁷ est hydrogène, (C₁-C₄)-alkyle ou (C₃-C₅)-cycloalkyle,
dans lequel (C₁-C₄)-alkyle peut être substitué par 1 à 3 substituants sélectionnés indépendamment du groupe de fluor, trifluorométhyle, cyano, cyclopropyle, cyclobutyle, hydroxyle, trifluorométhoxy, méthoxy, éthoxy, azétidinyle, oxétanyl, tétrahydrofuranyl et pyrrolidinyle,
R²⁴ est hydrogène ou fluor,
R¹⁰ est (C₁-C₄)-alkyle,
R¹⁵ est hydrogène, méthyle ou éthyle,
dans lequel méthyle et éthyle peuvent être substitués par 1 substituant sélectionné du groupe de fluor, trifluorométhyle et cyclopropyle,
et les sels, solvates et solvates des sels de ceux-ci.

4. Inhibiteur de la chymase selon la revendication 1 ou 2 pour une utilisation dans le traitement et la prophylaxie de l'accident vasculaire cérébral ischémique aigu, des accidents ischémiques transitoires (AIT), des accidents vasculaires cérébraux ischémiques, y compris les accidents vasculaires cérébraux cardioemboliques, les accidents vasculaires cérébraux dus à une fibrillation auriculaire, les accidents vasculaires cérébraux non cardioemboliques, l'infarctus lacunaire, les accidents vasculaires cérébraux dus à des maladies des grosses ou des petites artères, les accidents vasculaires cérébraux de cause indéterminée, les accidents vasculaires cérébraux cryptogéniques, les accidents vasculaires cérébraux emboliques, les accidents vasculaires cérébraux emboliques de source indéterminée, les événements d'origine thrombotique et/ou thromboembolique conduisant à un accident vasculaire cérébral ou à des accidents ischémiques transitoires, l'embolie pulmonaire, la microangiopathie thrombotique, la microangiopathie thrombotique dans des états d'hypercoagulabilité après infection, la coagulation intravasculaire disséminée, la thrombocytopénie thrombotique immunitaire induite par un vaccin, la thrombose ou l'occlusion au niveau du site d'accès vasculaire, dans lequel l'inhibiteur de la chymase est un composé de formule (I), choisi dans le groupe constitué de l'acide 1-(1,3-diméthyl-2-oxo-2,3-dihydro-1H-benzimidazol-5-yl)-2,4-dioxo-3-[(1R)-4-(trifluorométhyl)-2,3-dihydro-1H-indén-1-yl]-1,2,3,4-tétrahydropyrimidine-5-carboxylique (R énantiomère), l'acide 1-(6-fluoro-1,3-diméthyl-2-oxo-2,3-dihydro-1H-benzimidazol-5-yl)-2,4-dioxo-3-[(1R)-4-(trifluorométhyl)-2,3-dihydro-1H-indén-1-yl]-1,2,3,4-tétrahydropyrimidine-5-carboxylique (R énantiomère), l'acide 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylique (R énantiomère), l'acide 2,4-dioxo-3-[(1R)-4-(trifluorométhyl)-2,3-dihydro-1H-indén-1-yl]-1-(1,3,3-triméthyl-2-oxo-2,3-dihydro-1H-indol-5-yl)-1,2,3,4-tétrahydropyrimidine-5-carboxylique (R énantiomère), l'acide 1-(1'-méthyl-2'-oxo-1',2'-dihydrospiro[cyclopropane-1,3'-indole]-5'-yl)-2,4-dioxo-3-[(1R)-4-(trifluorométhyl)-2,3-dihydro-1H-indén-1-yl]-1,2,3,4-tétrahydropyrimidine-5-carboxylique (R énantiomère), l'acide 1-(3-méthyl-2-oxo-2,3-dihydro-1,3-benzothiazol-6-yl)-2,4-dioxo-3-[{(1R)-4-(trifluorométhyl)-2,3-dihydro-1H-indén-1-yl]-1,2,3,4-tétrahydropyrimidine-5-carboxylique (R énantiomère) et l'éthyl 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylate (R énantiomère).

5. Inhibiteur de la chymase selon la revendication 1, 2 ou 4 pour une utilisation dans le traitement et la prophylaxie de l'accident vasculaire cérébral ischémique aigu, des accidents ischémiques transitoires (AIT), des accidents vasculaires cérébraux ischémiques, y compris les accidents vasculaires cérébraux cardioemboliques, les accidents vasculaires cérébraux dus à une fibrillation auriculaire, les accidents vasculaires cérébraux non cardioemboliques, l'infarctus lacunaire, les accidents vasculaires cérébraux dus à des maladies des grosses ou des petites artères, les accidents vasculaires cérébraux de cause indéterminée, les accidents vasculaires cérébraux cryptogéniques, les accidents vasculaires cérébraux emboliques, les accidents vasculaires cérébraux emboliques de source indéterminée, les événements d'origine thrombotique et/ou thromboembolique conduisant à un accident vasculaire cérébral ou à des accidents ischémiques transitoires, l'embolie pulmonaire, la microangiopathie thrombotique, la microangiopathie thrombotique dans des états d'hypercoagulabilité après infection, la coagulation intravasculaire disséminée, la thrombocytopénie thrombotique immunitaire induite par un vaccin, la thrombose ou l'occlusion au niveau du site d'accès vasculaire, dans lequel l'inhibiteur de la chymase de formule (I) est l'acide 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylique (R énantiomère) de formule

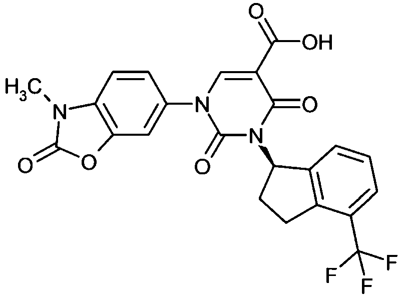

6. Association d'un ou de plusieurs composés de la formule (I) avec un ou plusieurs autres composés actifs pour une utilisation selon l'une quelconque des revendications 1 à 5.

7. Composition pharmaceutique comprenant au moins un composé de la formule (I) selon l'une quelconque des revendications 1 à 6 en association avec un ou plusieurs excipients inertes, non toxiques et pharmaceutiquement appropriés pour une utilisation selon l'une quelconque des revendications 1 à 5.

8. Inhibiteur de la chymase selon l'une quelconque des revendications 1 à 5 pour une utilisation dans un procédé de traitement et/ou de prophylaxie de l'accident vasculaire cérébral ischémique aigu, des accidents ischémiques transitoires (AIT), des accidents vasculaires cérébraux ischémiques, y compris les accidents vasculaires cérébraux cardioemboliques, tels que les accidents vasculaires cérébraux dus à une fibrillation auriculaire, les accidents vasculaires cérébraux non cardioemboliques, tels que l'infarctus lacunaire, les accidents vasculaires cérébraux dus à des maladies des grosses ou des petites artères, ou les accidents vasculaires cérébraux de cause indéterminée, les accidents vasculaires cérébraux cryptogéniques, les accidents vasculaires cérébraux emboliques, les accidents vasculaires cérébraux emboliques de source indéterminée, ou les événements d'origine thrombotique et/ou thromboembolique conduisant à un accident vasculaire cérébral ou à des accidents ischémiques transitoires AIT, l'embolie pulmonaire, la microangiopathie thrombotique, la microangiopathie thrombotique dans des états d'hypercoagulabilité après infection, la coagulation intravasculaire disséminée, la thrombocytopénie thrombotique immunitaire induite par un vaccin, la thrombose ou l'occlusion au niveau du site d'accès vasculaire, par administration systémique et/ou locale d'une quantité thérapeutiquement efficace d'au moins un composé selon l'une quelconque des revendications 1 à 5 ou d'un médicament comprenant au moins un composé selon l'une quelconque des revendications 1 à 5 en association avec un additif inerte, non toxique et pharmaceutiquement acceptable.

9. Inhibiteur de la chymase selon l'une quelconque des revendications 1 à 5 pour une utilisation dans un procédé selon la revendication 8, dans lequel le médicament comprend en outre au moins un autre composé actif choisi dans le groupe constitué de médicaments anticoagulants et/ou antiplaquettaires.

10. Médicament comprenant un composé de la formule (I) pour une utilisation selon l'une quelconque des revendications 1 à 5 en association avec un ou plusieurs autres principes actifs choisis dans le groupe constitué de médicaments anticoagulants et/ou antiplaquettaires.
